(19) Europäisches Patentamt
European Patent Office
Office européen des brevets

(11) EP 3 390 672 B1

(12) **EUROPEAN PATENT SPECIFICATION**

(45) Date of publication and mention of the grant of the patent:
**26.05.2021 Bulletin 2021/21**

(21) Application number: **16876172.4**

(22) Date of filing: **19.12.2016**

(51) Int Cl.:
***C12Q 1/70*** *(2006.01)* ***C12Q 1/68*** *(2018.01)*

(86) International application number:
**PCT/SG2016/050611**

(87) International publication number:
**WO 2017/105353 (22.06.2017 Gazette 2017/25)**

(54) **DETECTION AND QUANTIFICATION OF TARGET NUCLEIC ACID SEQUENCE OF A MICROORGANISM**

DETEKTION UND QUANTIFIZIERUNG EINER ZIELNUKLEINSÄURESEQUENZ EINES MIKROORGANISMUS

DÉTECTION ET QUANTIFICATION D'UNE SÉQUENCE D'ACIDE NUCLÉIQUE D'UN MICRO-ORGANISME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2015 SG 10201510448Q**

(43) Date of publication of application:
**24.10.2018 Bulletin 2018/43**

(73) Proprietor: **Lucence Life Sciences Pte. Ltd.**
**Singapore 159552 (SG)**

(72) Inventors:
- **TAN, Min-Han**
  **Singapore 138669 (SG)**
- **VO, Thu Hong Anh**
  **Singapore 138669 (SG)**

(74) Representative: **Mewburn Ellis LLP**
**Aurora Building**
**Counterslip**
**Bristol BS1 6BX (GB)**

(56) References cited:
**US-A1- 2003 228 575 US-A1- 2015 361 505**

- **HIDEYUKI ISHII ET AL: "Clinical usefulness of serum EBV DNA levels of BamHI W and LMP1 for nasal NK/T-cell lymphoma", JOURNAL OF MEDICAL VIROLOGY, JOHN WILEY & SONS, INC, US, vol. 79, no. 5, 1 May 2007 (2007-05-01), pages 562-572, XP002628523, ISSN: 0146-6615, DOI: 10.1002/JMV.20853 [retrieved on 2007-03-26]**
- **BERGER CHRISTOPH ET AL: "Dynamics of Epstein-Barr virus DNA levels in serum during EBV-associated disease", August 2001 (2001-08), JOURNAL OF MEDICAL VIROLOGY, VOL. 64, NR. 4, PAGE(S) 505-512, XP002790292, ISSN: 0146-6615 * page 506, column 2, paragraphs 1,2 ***
- **WAGNER, H.J. ET AL.: 'Detection and quantification of latently infected B lymphocytes in Epstein-Barr virus-seropositive, healthy individuals by polymerase chain reaction.' J CLIN MICROBIOL vol. 30, no. 11, November 1992, pages 2826 - 2829, XP055391684**
- **ARRIBAS, J.R. ET AL.: 'Detection of Epstein-Barr virus DNA in cerebrospinal fluid for diagnosis of AIDS-related central nervous system lymphoma.' J CLIN MICROBIOL vol. 33, no. 6, June 1995, pages 1580 - 1583, XP055391686**
- **MIYASHITA, E.M. ET AL.: 'A Novel Form of Epstein-Barr Virus Latency in Normal B Cells In Vivo.' CELL vol. 80, no. 4, 24 February 1995, pages 593 - 601, XP055391691**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- **LIU, P. ET AL.: 'Direct Sequencing and Characterization of a Clinical Isolate of Epstein-Barr Virus from Nasopharyngeal Carcinoma Tissue by Using Next- Generation Sequencing Technology.' J VIROL vol. 85, no. 21, 31 August 2011, pages 11291 - 11299, XP055391759**
- **LO, Y.M.D. ET AL.: 'Quantitative Analysis of Cell -free Epstein-Barr Virus DNA in Plasma of Patients with Nasopharyngeal Carcinoma.' CANCER RES. vol. 59, no. 6, 15 March 1999, pages 1188 - 1191, XP000979273**
- **CHEUNG, A. ET AL.: 'Long Internal Direct Repeat in Epstein-Barr virus DNA.' J VIROL vol. 44, no. 1, October 1982, pages 286 - 294, XP002045655**
- **JENSON, H.B. ET AL.: 'Sequences of the Epstein-Barr Virus (EBV) large internal repeat form the center of a 16-kilobase-pair palindrome of EBV (P3 HR - 1) heterogeneous DNA.' J VIROL vol. 61, no. 5, May 1987, pages 1495 - 1506, XP055391768**
- **GARDINER-GARDEN, M. ET AL.: 'CpG island s in Vertebrate Genomes.' J MOL BIOL vol. 196, no. 2, 20 July 1987, pages 261 - 282, XP024021238**
- **VO, J.H. ET AL.: 'Comparison of Circulating Tumour Cells and Circulating Cell - Free Epstein-Barr Virus DNA in Patients with Nasopharyngeal Carcinoma Undergoing Radiotherapy.' SCI REP vol. 6, no. 1, 19 December 2016, pages 1 - 9, XP055391772**


Remarks:

The file contains technical information submitted after the application was filed and not included in this specification

## Description

## FIELD OF THE INVENTION

The present disclosure relates to molecular biology in particular biomarkers. In particular, the present disclosure relates to the detection and quantification of biomarkers associated with microorganisms such as bacteria, viruses, fungi and parasites, and methods of determining the likelihood that a patient suffers or is likely to suffer from diseases associated with microorganisms, and to predict the treatment outcome of a patient having diseases associated with microorganisms, by detecting and quantifying the biomarkers associated with the microorganisms.

## BACKGROUND OF THE INVENTION

Infections with microorganisms, including viruses, bacteria, fungi and parasites, have been recognized as risk factors for a wide range of diseases in humans, such as cancers, autoimmune diseases and cardiovascular diseases, which are associated with high mortality rates. Early detection of the causative microorganisms is crucial for the prevention, detection, diagnosis and treatment of these diseases associated with infections with microorganisms.

Examples of conventional methods used for the detection of microorganisms include culture-based procedures, serologic tests and microscopy. Each of these methods is associated with its own limitations. For example, culture-based procedures, which are dependent on the growth of the microorganisms, often takes days for results to become available, and such procedures often reveal false negative results due to the administration of an empiric antibiotic therapy. Serologic tests often face high false negative rate due to the absence or weakness of antibody production in the patient's body, or high false positive rate due to the presence of cross-reacting antibodies. Microscopy techniques may be limited by sample staining and fixing and the use of strong illumination, which may destroy or distort cellular features of the microorganisms to be detected.

Molecular diagnostic procedures such as PCR-based techniques have been introduced for the detection of microorganism infections in recent years. Wagner et al. (Detection and quantification of latently infected B lymphocytes in Epstein-Barr virus-seropositive, healthy individuals by polymerase chain reaction., J. Clin. Microbiol., vol. 30, no. 11, November 1992 (1992-11), pages 2826-2829) describes the detection by PCR of Epstein-Barr virus in blood sample obtained from EBV-seropositive blood donors. Arribas et al. (Detection of Epstein-Barr virus DNA in cerebrospinal fluid for diagnosis of AIDS-related central nervous system lymphoma., J. Clin. Microbiol., vol. 33, no. 6, June 1995 (1995-06), pages 1580-1583) describes a method for detecting by PCR EBV nucleic acid in the cerebrospinal fluid of patients for diagnosing the central nervous system lymphoma. Miyashita et al. (A Novel Form of Epstein-Barr Virus Latency in Normal B Cells In Vivo., CELL, vol. 80, no. 4, 24 February 1995 (1995-02-24), pages 593-601) describes a method for detecting EBV DNA in infected cells in peripheral blood of seropositive patients comprising amplifying a fragment of the BamHI-W region of EBV. However, the currently available PCR-based techniques have some limitations in the quantitative measurement of the bacterial/viral load in patients' samples, as well as problems of false positive results. Thus, what is needed is an improved method of detecting and/or quantifying of microorganisms with better sensitivity and specificity.

## SUMMARY OF THE INVENTION

In one aspect, there is provided a method for detecting and/or quantifying the presence of a target nucleic acid sequence of a microorganism in a sample obtained from a subject, comprising amplifying the target sequence in a CpG island of the nucleic acid of the microorganism, irrespective of the methylation status of the CpG island.

In another aspect, there is provided a method for detecting and/or quantifying the presence of a target nucleic acid sequence of Epstein-Barr virus (EBV) in a sample obtained from a subject, comprising amplifying a target sequence in the *BamHI-W* region of EBV, wherein the target sequence comprises the sequence of AGATCTAAGGCCGGGAGAGGCAGCCCCAAAGCGGGTGCAGTAACAGGTAATCTC TGGTAGTGATTTGGACCCGAAATCTGACACTTTAGAGCTCTGGAGGACTTTAAAA CTCTAAAAATCAAAACTTTAGAGGCGAATGGGCG (SEQ ID NO: 1), wherein amplifying the target sequence comprises the use of a pair of oligonucleotide primers and a probe, wherein the first oligonucleotide primer comprises the sequence of 5'-AGATCTAAGGCCGGGAGAGG-3' (SEQ ID NO:2), and the second oligonucleotide primer comprises the sequence of 5'-CGCCCATTCGCCTCTAAAGT-3' (SEQ ID NO: 3), and wherein the probe comprises the sequence of 5'-(6-FAM)CTCTGGTAGTGATTTGGACCCGAAATCTG(TAMRA)-3'(SEQ ID NO: 4), and wherein the method is a quantitative polymerase chain reaction (qPCR).

In yet another aspect, there is provided a method of detecting a disease associated with microorganism infection, or risk of developing a disease associated with microorganism infection in a subject, comprising detecting and/or quantifying the presence of a nucleic acid sequence of the microorganism using the method of the present invention in a sample obtained from the subject, wherein the presence of the nucleic acid sequence of the microorganism in the sample indicates that the subject has a disease associated with microorganism infection or is at risk of developing a disease associated with microorganism infection.

In another aspect, there is provided a method of detecting and treating a disease associated with microorganism infection, comprising: (i) detecting and/or quantifying the presence of a nucleic acid sequence of the microorganism using the method of the present invention in a sample obtained from the subject, wherein the presence of the nucleic acid sequence of the microorganism in the sample indicates that the subject has a disease associated with microorganism; and (ii) administering to the subject a medicament suitable for the treatment of the disease associated with the microorganism.

In yet another aspect, there is provided a method of predicting the treatment outcome of a disease associated with microorganism infection in a patient, comprising:(i) quantifying the nucleic acid sequence of the microorganism in a sample collected from the patient before treatment or before a treatment step, and quantifying the nucleic acid sequence of the microorganism in a sample collected from the same patient after treatment or after a treatment step; (ii) comparing the amount of the nucleic acid sequence of the microorganism in the sample before and after treatment or a treatment step, wherein a decrease in the amount of the nucleic acid sequence of the microorganism in the sample after treatment or a treatment step indicates that treatment outcome of the disease associated with microorganism infection in the patient is positive, wherein the quantifying of the nucleic acid sequence of the microorganism in the sample is performed according to the method of the present invention.

In a further aspect, there is provide a kit for detecting and/or quantifying the nucleic acid sequence of a microorganism in a sample obtained from a subject, comprising a pair of oligonucleotide primers specific for the amplification of a target sequence in a CpG island of the nucleic acid of the microorganism.

## BRIEF DESCRIPTION OF THE DRAWINGS

The invention will be better understood with reference to the detailed description when considered in conjunction with the non-limiting examples and the accompanying drawings, in which:

**Fig. 1** depicts the overall survival curves of patients with nasopharyngeal carcinoma (NPC) with NPC levels measured using different circulating biomarkers. Results are shown using Kaplan-Meier plots for dichotomized biomarker variables. Fig. 1A shows the overall survival curves of NPC patients with EBV measured by the number of copies of *Bam*HI-W per ml using qPCR. Fig. 1B shows the overall survival curves of NPC patients with EBV measured by the number of copies of *EBNA1* per ml using qPCR. Fig. 1C shows the overall survival curves of NPC patients with EBV measured by the number of copies of *EBNA1* per ml using dPCR. Fig. ID and IE show the overall survival curves of NPC patients with NPC level measured in terms of the counts of circulating tumor cells (CTCs). The results show that the levels of *Bam*HI-W (detected using dPCR) and *EBNA1* (detected using dPCR or qPCR) serve as better indicators of the overall survival rate as compared to CTCs.

**Fig. 2** is a flow chart depicting the overall results of detecting NPC using different EBV biomarkers. A total of 46 NPC patients, all of Asian ethnicity, took part in the study. The blood sample collected from each of these patients have been subjected to all three assays - BamHI-W qPCR assay, *EBNA1* qPCR assay, and *EBNA1* dPCR assay. As shown in the flow chart, *Bam*HI-W qPCR assay detected 41 patients as NPC positive and 5 patients as NPC negative; *EBNA1* qPCR assay detected 31 patients as NPC positive and 15 patients as NPC negative; *EBNA1* dPCR assay detected 39 patients as NPC positive and 7 patients as NPC negative. The results indicate that the *Bam*HI-W qPCR assay has better sensitivity for the detection of NPC as compared to *EBNA1* qPCR and *EBNA1* dPCR assays.

**Fig. 3** is a flow chart depicting the process of designing and selecting the synthetic internal control. 50 random sequences with the length of 150 bases were generated using R program. Sequences with multiple base repeats and runs (i.e. ATATAT, CCCCCC, etc.) were eliminated. 15 sequences passed this criterion. Primers and probes were designed for these 15 sequences such that the target sequences are about 100bp and located in the central region of the 150-base sequences. Primer-dimer and primer-probe dimer formation were checked by NetPrimer program. The $\Delta$G values between all the primers and probe (in both Internal Control (IC) and *Bam*HI-W reaction) must be more positive than -9 kcal/mole (see Table 1). Only one sequence qualified under this criterion, and this sequence was then checked for similarity in NCBI Genomic Reference Sequences, NCBI Chromosome Sequences and Nucleotide Collection using BLAST program. No significant similarity was found in all the databases, indicating the selected internal control sequence is unique.

Table 1. Values of ∆**G** between the primers and probe of selected Internal Control (IC) and the primers and probe of *Bam*HI-W.

| **∆G** | | ***Bam*HI-W** | | |
|---|---|---|---|---|
| | | **Forward Primer** | **Reverse Primer** | **Probe** |
| **IC** | **Forward Primer** | -7.24 | No | -8.26 |
| | **Reverse Primer** | No | -5.67 | -5.41 |
| | **Probe** | -5.67 | -3.94 | No |

**Fig. 4** depicts the detection of EBV using *Bam*HI-W qPCR assay, in plasma samples with and without the internal control (IC) plasmid. X-axis represents the copy number of *Bam*HI-W detected, and Y-axis represents the threshold cycle (Ct) which is the intersection between an amplification curve and a threshold line. The results indicate that the internal control plasmid does not interfere with the detection of EBV using *Bam*HI-W qPCR assay.

**Fig. 5** illustrates the different experimental settings used to test the impact of the internal control (IC) plasmid on the detection of EBV using *Bam*HI-W qPCR assay. In setting 1, no IC plasmid was added into the sample, and the qPCR was performed on the DNA sample after it was extracted In Setting 2, DNA was extracted from the sample first, and the IC plasmid was added into the extracted DNA sample. Both the IC and the EBV target sequence in the extract DNA were amplified by the qPCR. In Setting 3, the IC plasmid was added before the DNA extraction step. Both the IC and the EBV target sequence were extracted using the DNA extraction method, and amplified by the qPCR.

**Fig. 6** shows examples of qPCR amplification curves with the internal control (IC) plasmid added in the sample to detect false negative qPCR amplifications. Fig. 6A and 6B indicate positive results for EBV detection using *Bam*HI-W qPCR assay. Fig. 6C indicates true negative result for EBV detection using *Bam*HI-W qPCR assay, as the IC plasmid was amplified by the qPCR assay. Fig. 6D indicates false negative result for EBV detection using *Bam*HI-W qPCR assay, as the IC plasmid was not amplified by the qPCR assay. Over all, it can be concluded that IC can serve as a control to identify a false negative detection of *Bam*HI-W.

**Fig. 7** depicts the construction of a *Bam*HI-W standard plasmid and an internal control (IC) plasmid.

**Fig. 8** shows the confirmation of the *Bam*HI-W sequence in the *Bam*HI-W standard plasmid and the internal control (IC) sequence in the IC plasmid constructed as depicted in Fig. 7. Fig. 8A shows the sequence of the BamHI-W standard forward strand. Fig. 8B shows the sequence of the IC forward strand. Fig. 8C shows the sequence of the *Bam*HI-W standard reverse strand. Fig. 8D shows the sequence of the IC reverse strand. The results indicate that the *Bam*HI-W sequence inserted in the *Bam*HI-W plasmid has 100% sequence identity as the *Bam*HI-W region of EBV, and that the IC sequence inserted in the IC plasmid has 10% sequence identity as the designed sequence of the IC.

**Fig. 9** shows the results of plasmid validation in clinical samples using *Bam*HI-W qPCR assay. Fig. 9A shows the results where the internal control (IC) plasmids were added in the plasma sample from the patient prior to DNA extraction. Fig. 9B shows the results of amplification of the constructed *Bam*HI-W standard plasmids added directly to PCR plates. The results indicate that the constructed IC and *Bam*HI-W plasmids can be amplified after being extracted together with DNA from clinical samples (Fig. 9A) and when being added directly in the PCR plates (Fig. 9B).

**Fig. 10** shows the derivation of conversion factor between *Bam*HI-W standard plasmids constructed and the International Standard (IU) of EBV defined by the National Institute for Biological Standards and Control (NIBSC). Fig. 10A shows the plot of the number of copies of *Bam*HI-W plasmids added in the sample (y-axis) against the number of IUs defined by NIBSC (x-axis), both in log scale. Fig. 10B shows the plot of the number of copies of *Bam*HI-W plasmids added in the sample (y-axis) against the number of IUs defined by NIBSC (x-axis). The conversion factor between *Bam*HI-W standard plasmid and the NIBSC standard is 1 IU of EBV = 1.38 copies of *Bam*HI-W.

**Fig. 11** shows the PCR amplification curves of the target sequences of *Mycobacterium tuberculosis.* In Fig. 11A and 11C, the target sequence is nucleotide sequence from nucleotide position 1542511 to nucleotide position 1542349 of the genomic sequence of Mycobacterium tuberculosis. In Fig. 11B and 11D, the target sequence is nucleotide sequence from nucleotide position 1542328 to nucleotide position 1542215 of the genomic sequence of *Mycobacteriaum tuberculosis.* Fig. 11A and 11B represent the amplification curve using a sample from a patient known to have tuberculosis, and 11C and 11D represent the amplification curve using a sample from a healthy subject control. The results indicate that both target sequences of *Mycobacterium tuberculosis* are present in the sample from the patient with tuberculocis, but absent in the sample from the healthy subject control.

**Fig. 12** shows a gel electrophoresis photo of using different sets of *Bam*HI-W primers directed to different target sequences for the amplification of *Bam*HI-W. An EBV-positive cell line, C666-1, was used as the positive control. EBV-negative RKO, a buffy coat of healthy donor was used as the negative control. The sequences of primer pair 2 are: forward primer 5'-GGAATAAGCCCCCAGACAGG-3' (SEQ ID NO:12), reverse primer 5'- TTACGTAAACGCGCTGGACT-3' (SEQ ID NO.: 14); the sequences of primer pair 7 are: forward primer 5'-AGATCTAAGGCCGGGAGAGG-3' (SEQ ID NO:2), reverse primer 5'-CGCCCATTCGCCTCTAAAGT-3' (SEQ ID NO: 3); the sequences of primer pair 10 are: forward primer 5'- AGGAAGCGGGTCTATGGTTG-3' (SEQ ID NO:13), reverse primer 5'-GACTGAGAAGGTGGCCTAGC-3' (SEQ ID NO:15). The results indicate that all three sets of primers can be used for the successful detection of *Bam*HI-W.

**DETAILED DESCRIPTION OF THE PRESENT INVENTION**

In light of the issues discussed in the background of the invention, the present disclosure provides a method of detecting and/or quantifying a microorganism that has better sensitivity and/or specificity compared to the currently available methods.

In one aspect, there is provided a method for detecting and/or quantifying the presence of a target nucleic acid sequence of a microorganism in a sample obtained from a subject, comprising amplifying the target sequence in a CpG island of the nucleic acid of the microorganism, irrespective of the methylation status of the CpG island.

The term "amplifying" or "amplification" as used herein refers to the production of additional copies of the target sequence.

The term "CpG island" as used herein refers to nucleic acid regions with a high frequency of CpG sites. A CpG site (or CG site) is a region of DNA where a cytosine nucleotide is followed by a guanine nucleotide in the linear sequence of nucleotides along its 5' to 3' direction. CpG is shorthand for 5'-C-phosphate-G-3'. A CpG island can have at least about 100 nucleotides, or at least about 500 nucleotides, or at least about 1000 nucleotides, or at least about 2000 nucleotides, or at least about 3000 nucleotides, or at least about 4000 nucleotides, or at least about 5000 nucleotides, or at least about 6000 nucleotides, or at least about 7000 nucleotides, or at least about 8000 nucleotides, or at least about 9000 nucleotides, or at least about 10000 nucleotides, or at least about 15000 nucleotides, or at least about 20000 nucleotides, or at least about 25000 nucleotides, or at least about 30000 nucleotides, or at least about 35000 nucleotides, or at least about 40000 nucleotides, or at least about 45000 nucleotides, or at least about 50000 nucleotides, or at least about 60000 nucleotides, or at least about 70000 nucleotides, or at least about 80000 nucleotides, or at least about 90000 nucleotides, or at least about 100000 nucleotides, or between about 100 nucleotides to about 5000 nucleotides, or between about 200 nucleotides to about 4900 nucleotides, or between about 300 nucleotides to about 4800 nucleotides, or between about 400 nucleotides to about 4700 nucleotides , or between about 500 nucleotides to about 4600 nucleotides, or between about 600 nucleotides to about 4500 nucleotides, or between about 700 nucleotides to about 4400 nucleotides, or between about 800 nucleotides to about 4300 nucleotides, or between about 900 nucleotides to about 4200 nucleotides, or between about 1000 nucleotides to about 4100 nucleotides, or between about 1100 nucleotides to about 4000 nucleotides, or between about 1200 nucleotides to about 3900 nucleotides, or between about 1300 nucleotides to about 3800 nucleotides, or between about 1400 nucleotides to about 3700 nucleotides, or between about 1500 nucleotides to about 3600 nucleotides, or between about 1600 nucleotides to about 3500 nucleotides, or between about 1700 nucleotides to about 3400 nucleotides, or between about 1800 nucleotides to about 3300 nucleotides, or between about 1900 nucleotides to about 3200 nucleotides, or between about 2000 nucleotides to about 3100 nucleotides, or between about 2100 nucleotides to about 3000 nucleotides, or between about 2200 nucleotides to about 2900 nucleotides, or between about 2300 nucleotides to about 2800 nucleotides, or between about 2400 nucleotides to about 2700 nucleotides, or between about 2500 nucleotides to about 2600 nucleotides, or about 150 nucleotides, or about 250 nucleotides, or about 350 nucleotides , or about 450 nucleotides, or about 550 nucleotides, or about 650 nucleotides, or about 750 nucleotides, or about 850 nucleotides, or about 950 nucleotides, or about 1050 nucleotides, or about 1150 nucleotides, or about 1250 nucleotides, or about 1350 nucleotides, or about 1450 nucleotides, or about 1550 nucleotides, or about 1650 nucleotides, or about 1750 nucleotides, or about 1850 nucleotides, or about 1950 nucleotides, or about 2050 nucleotides, or about 2150 nucleotides, or about 2250 nucleotides, or about 2350 nucleotides, or about 2450 nucleotides, or about 2550 nucleotides, or about 2650 nucleotides, or about 2750 nucleotides, or about 2850 nucleotides, or about 2950 nucleotides, or about 3050 nucleotides, or about 3150 nucleotides, or about 3250 nucleotides, or about 3350 nucleotides, or about 3450 nucleotides, or about 3550 nucleotides, or about 3650 nucleotides, or about 3750 nucleotides, or about 3850 nucleotides, or about 3950 nucleotides, or about 4050 nucleotides, or about 4150 nucleotides, or about 4250 nucleotides, or about 4350 nucleotides , or about 4450 nucleotides, or about 4550 nucleotides, or about 4650 nucleotides, or about 4750 nucleotides, or about 4850 nucleotides, or about 4950 nucleotides. A CpG island usually has a C, G percentage of greater than about 50%, or greater than about 51%, or greater than about 52%, or greater than about 53%, or greater than about 54%, or greater than about 55%, or greater than about 56%, or greater than about 57%, or greater than about 58%, or greater than about 59%, or greater than about 60%, or greater than about 61%, or greater than about 62%, or greater than about 63%, or greater than about 64%, or greater than about 65%, or greater than about 66%, or greater than about 67%, or greater than about 68%, or greater than about 69%, or greater than about 70%, or greater than about 71%, or greater than about 72%, or greater than about 73%, or greater than about 74%, or greater than about 75%, or greater than about 76%, or greater than about 77%, or greater than about 78%, or greater than about 79%, or greater than about 80%. The observed-to-expected CpG ratio in CpG island is usually greater than about 55%, or greater than about 56%, or greater than about 57%, or greater than about 58%, or greater than about 59%, or greater than about 60%, or greater than about 61%, or greater than about 62%, or greater than about 63%, or greater than about 64%, or greater than about 65%, or greater than about 66%, or greater than about 67%, or greater than about 68%, or greater than about 69%, or greater than about 70%, or greater than about 71%, or greater than about 72%, or greater than about 73%, or greater than about 74%, or greater than about 75%, or greater than about 76%, or greater than about 77%, or greater than about 78%, or greater than about 79%, or greater than about 80%, or greater than about 81%, or greater than about 82%, or greater than about 83%, or greater than about 84%, or greater than about 85%, or greater than about 86%, or greater than about 87%, or greater than about 88%, or greater than about 89%, or greater than about 90%, or greater than about 91%, or greater than about 92%, or greater than about 93%, or greater than about 94%, or greater than about 95%, or greater than about 96%, or greater than about 97%, or greater than about 98%, or greater than about 99%. The "observed-to-expected CpG ratio" can be derived where the "observed" is the actual number of CpGs in the sequence, and where the "expected" is calculated as:

$$(\text{actual number of C} \times \text{actual number of G})/\text{length of sequence}$$

or as:

$$((\text{actual number of C} + \text{actual number of G})/2)^2/\text{length of sequence.}$$

A number of softwares or analytical tools can be used for the prediction of CpG island in a nucleic acid sequence. Examples of analytical tools available online include but are not limited to: Sequence Manipulation Suite available at http://www.bioinformatics.org/sms2/cpg_islands.html; and Emboss Cpgplot available at http://www.ebi.ac.uk/Tools/seqstats/emboss_cpgplot/.

The term "target sequence" or "target nucleic acid sequence" or their grammatical variants as used herein refer to a region of the nucleic acid sequence of the microorganism of interest to be amplified. The presence of the target sequence in a sample obtained from a subject indicates the presence of the nucleic acid sequence associated with the microorganism of interest, and/or the presence of the microorganism in the sample or in the subject.

In some examples, it is preferred that the target sequence to be amplified for the detection and/or quantification of the microorganism is located within the 5'end of the CpG island. The 5' end location would allow for preferential preservation during exonuclease III degradation of the DNA. Thus, in some examples, the target sequence is located within the first 50%, or the first 49%, or the first 48%, or the first 47%, or the first 46%, or the first 45%, or the first 44%, or the first 43%, or the first 42%, or the first 41%, or the first 40%, or the first 39%, or the first 38%, or the first 37%, or the first 36%, or the first 35%, or the first 34%, or the first 33%, or the first 32%, or the first 31%, or the first 30%, or the first 29%, or the first 28%, or the first 27%, or the first 26%, or the first 25%, or the first 24%, or the first 23%, or the first 22%, or the first 21%, or the first 20%, or the first 19%, or the first 18%, or the first 17%, or the first 16%, or the first 15%, or the first 14%, or the first 13%, or the first 12%, or the first 11%, or the first 10%, or the first 9%, or the first 8%, or the first 7%, or the first 6%, or the first 5%, or the first 4%, or the first 3%, or the first 2%, or the first 1% nucleotides of the CpG island of the nucleic acid of the microorganism, counted from the 5' end.

The term "methylation" as used herein refers to DNA methylation which typically occurs at a CpG site. Such methylation results in the conversion of the cytosine to 5-methylcytosine, and can by catalysed by the enzyme DNA methyltransferase. A CpG cite can be either methylated or unmethylated.

The method of for detecting and/or quantifying the presence of a target nucleic acid sequence of a microorganism as provided in the present disclosure comprises amplifying the target sequence in a CpG island of the nucleic acid of the microorganism, irrespective of the methylation status of the CpG island. This means that the methylation status of the CpG island for which the target sequence lies within is not important. One of the advantages provided by such a method is that no methylation-status-specific sequencing will be required to select the target sequence to be amplified.

The term "microorganism" as used herein refers to a microscopic living organism, which may be single-celled or multicellular. The microorganism contains DNAs, thus microorganisms that do not contain DNAs (e.g. RNA viruses) are excluded. Examples of microorganisms include but are not limited to bacteria, DNA viruses, fungi and parasites. In some specific examples, the microorganisms are bacteria and DNA viruses. DNA viruses include but are not limited to DNA viruses with double stranded DNAs and DNA viruses with single stranded DNAs. In some examples, the microorganisms are pathogenic.

The term "pathogenic", "pathogen" and other grammatical variants as used herein refer to the ability of the microorganisms to cause diseases. Examples of such diseases include but are not limited to acinetobacter infections, Actinomycosis, African sleeping sickness (African trypanosomiasis), AIDS (Acquired immunodeficiency syndrome), Amebiasis, Anaplasmosis, Angiostrongyliasis, Anisakiasis, Anthrax, Arcanobacterium haemolyticum infection, Argentine hemorrhagic fever, Ascariasis, Aspergillosis, Astrovirus infection, Alzheimer's disease, Amyotrophic lateral sclerosis, Anorexia nervosa, Anxiety disorder, Asthma, Atherosclerosis, Autoimmune diseases, Babesiosis, Bacillus cereus infection, Bacterial pneumonia, Bacterial vaginosis, Bacteroides infection, Balantidiasis, Bartonellosis, Baylisascaris infection, BK virus infection, Black piedra, Blastocystosis, Blastomycosis, Bolivian hemorrhagic fever, Botulism (and infant botulism), Brazilian hemorrhagic fever, Brucellosis, Bubonic plague, *Burkholderia* infection, Buruli ulcer, Calicivirus infection (norovirus and sapovirus), Campylobacteriosis, Candidiasis (moniliasis; thrush), Capillariasis, Carrion's disease, Cellulitis, Chagas disease, Chancroid, Chickenpox, Chikungunya, Chlamydia, Chlamydophila pneumoniae infection, Cholera, Chromoblastomycosis, Chytridiomycosis, Clonorchiasis, Clostridium difficile colitis, Coccidioidomycosis, Colorado tick fever, Common cold (acute viral rhinopharyngitis; acute coryza), Creutzfeldt-Jakob disease, Crimean-Congo hemorrhagic fever, Cryptococcosis, Cryptosporidiosis, Cutaneous larva migrans, Cyclosporiasis, Cysticercosis, Cytomegalovirus infection, Cancers, Chronic obstructive pulmonary disease, Crohn's disease, Coronary heart disease, Dengue fever, Desmodesmus infection, Dientamoebiasis, Diphtheria, Diphyllobothriasis, Dracunculiasis, Dementia, Diabetes mellitus type 1, Diabetes mellitus type 2, Dilated cardiomyopathy, Ebola hemorrhagic fever, Echinococcosis, Ehrlichiosis, Enterobiasis, Enterococcus infection, Enterovirus infection, Epidemic typhus, Erythema infectiosum, Exanthem subitum, Epilepsy, Epstein-Barr virus infectious mononucleosis, Fasciolasis, Fasciolopsiasis, Fatal familial insomnia, Filariasis, Food poisoning by clostridium perfringens, Free-living amebic infection, Fusobacterium infection, Gas gangrene (Clostridial myonecrosis), Geotrichosis, Gerstmann-Sträussler-Scheinker syndrome, Giardiasis, Glanders, Gnathostomiasis, Gonorrhoea, Granuloma inguinale, Group A Streptococcal infection, Group B Streptococcal infection, Guillain-Barré syndrome, Haemophilus influenzae infection, Hand, foot and mouth disease, Hantavirus pulmonary syndrome, Heartland virus disease, Helicobacter pylori infection, Hemolytic-uremic syndrome, Hemorrhagic fever with renal syndrome, Hepatitis A, Hepatitis B, Hepatitis C, Hepatitis D, Hepatitis E, Herpes simplex, Histoplasmosis, Hookworm infection, Human bocavirus infection, Human ewingii ehrlichiosis, Human granulocytic anaplasmosis, Human metapneumovirus infection, Human monocytic ehrlichiosis, Human papillomavirus infection, Human parainfluenza virus infection, Hymenolepiasis, Influenza, Isosporiasis, Irritable bowel syndrome, Kawasaki disease, Keratitis, Kingella kingae infection, Kuru, Lassa fever, Legionellosis, Legionellosis, Leishmaniasis, Leprosy, Leptospirosis, Listeriosis, Lyme disease, Lymphatic filariasis, Lymphocytic choriomeningitis, Lupus, Malaria, Marburg hemorrhagic fever, Measles, Middle East respiratory syndrome, Melioidosis, Meningitis, Meningococcal disease, Metagonimiasis, Microsporidiosis, Molluscum contagiosum, Monkeypox, mumps, Murine typhus, Mycoplasma pneumonia, Mycetoma, Myiasis, Multiple sclerosis, Myocardial infarction, Neonatal conjunctivitis, Nocardiosis, Onchocerciasis, Opisthorchiasis, Paracoccidioidomycosis, Paragonimiasis, Pasteurellosis, Pediculosis capitis, Pediculosis corporis, Pediculosis pubis, Pelvic inflammatory disease, Pertussis, Plague, Pneumococcal infection, Pneumocystis pneumonia, Panencephalitis, Pneumonia, Poliomyelitis, Prevotella infection, Primary amoebic meningoencephalitis, Progressive multifocal leukoencephalopathy, Psittacosis, Parkinson's disease, Psoriasis, Rabies, Relapsing fever, Respiratory syncytial virus infection, Rhinosporidiosis, Rhinovirus infection, Rickettsial infection, Rickettsialpox, Rift valley fever, Rocky mountain spotted fever, Rotavirus infection, Rubella, Rheumatoid arthritis, Salmonellosis, SARS, Scabies, Schistosomiasis, Sepsis, Shigellosis, Shingles, Smallpox (variola), Sporotrichosis, Staphylococcal food poisoning, Staphylococcal infection, Strongyloidiasis, Subacute sclerosing, Sarcoidosis, Schizophrenia, Stroke, Syphilis, Taeniasis, Tetanus, Tinea barbae, Tinea capitis, Tinea corporis, Tinea cruris, Tinea manum, Tinea nigra, Tinea pedis, Tinea unguium, Tinea versicolor, Toxocariasis, Toxocariasis, Trachoma, Toxoplasmosis, Trichinosis, Trichomoniasis, Trichuriasis, Tuberculosis, Tularemia, Typhoid fever, Typhus fever, Thromboangiitis obliterans, Tourette syndrome, Tuberculosis, Ureaplasma urealyticum infection, Vasculitis, Variant Creutzfeldt-Jakob disease, Valley fever, Venezuelan equine encephalitis, Venezuelan hemorrhagic fever, Vibrio vulnificus infection, Vibrio parahaemolyticus enteritis, Viral pneumonia, West nile fever, White piedra, Yersinia pseudotuberculosis infection, Yersiniosis, Yellow fever and Zygomycosis. In one specific example, the disease is cancer. In another specific example, the disease is tuberculosis.

One specific example of a DNA virus is Epstein-Barr virus (EBV), also called human herpesvirus 4 (HHV-4). EBV is one of the most common viruses in humans, and is commonly transmitted by saliva and established latent infection in B lymphocytes where it persists for the lifetime of the host. EBV infection is associated with particular forms of cancer, such as nasopharyngeal carcinoma, gastric cancer, Hodgkin's lymphoma and Burkitt's lymphoma.

In an example of the present disclosure, the target sequence to be amplified for the detection of EBV comprises a sequence within a CpG island of the *Bam*HI-W region of EBV.

The term "*Bam*HI-W region" as used herein refers to a repeating *Bam*HI-W restriction fragment of the EBV genome. *Bam*HI-W is a 3-kb long sequence and the genome of an EBV typically contains six to twenty copies of the *Bam*HI-W sequence. The *Bam*HI-W has the following sequence:

>J02072.1 Epstein Barr Virus large internal repeat (*Bam*HI-W fragment)

GGATCCCCCCACCGGCCCTTCTCTCTGTCCCCCTGCTCCTCTCCAACCTTCGCTCC
ACCCTAGACCCCAGCTTCTGGCCTCCCCGGGTCCACCAGGCCAGCCGGAGGGAC
CCCGGCAGCCCGGGCGAGTCGCCTTCCCTCTCCCCTGGCCTCTCCTTCCCGCCTCC
CACCCGAGCCCCCTCAGCTTGCCTCCCCACCGGGTCCATCAGGCCGGCCGGAGG
GACCCCGGCGGCCCGGTGTCAGTCCCCCCTGCAGCCGCCCAGTCTCTGCCTCCAG
GCAAGGGCGCCAGCTTTTCTCCCCCCAGCCTGAGGCCCAGTCTCCTGTGCACTGT
CTGTAAAGTCCAGCCTCCCACGCCCGTCCACGGCTCCCGGGCCCAGCCTCGTCCA
CCCCTCCCCACGGTGGACAGGCCCTCTGTCCACCCGGGCCATCCCCGCCCCCCTG
TGTCCACCCCAGTCCCGTCCAGGGGGGACTTTATGTGACCCTTGGGCCTGGCTCC
CCATAGACTCCCATGTAAGCCTGCCTCGAGTAGGTGCCTCCAGAGCCCCTTTTGC
CCCCCTGGCGGCCCAGCCCGACCCCCGGGCGCCCCCAAACTTTGTCCAGATGTCC
AGGGGTCCCCGAGGGTGAGGCCCAGCCCCCTCCCGCCCCTGTCCACTGCCCCGGT
CCCCCCAGAAGCCCCCAAAAGTAGAGGCTCAGGCCATGCGCGCCCTGTCACCAG
GCCTGCCAAAGAGCCAGATCTAAGGCCGGGAGAGGCAGCCCCAAAGCGGGTGC
AGTAACAGGTAATCTCTGGTAGTGATTTGGACCCGAAATCTGACACTTTAGAGCT
CTGGAGGACTTTAAAACTCTAAAAATCAAAACTTTAGAGGCGAATGGGCGCCAT
TTTGTCCCCACGCGCGCATAATGGCGGACCTAGGCCTAAAACCCCCAGGAAGCG
GGTCTATGGTTGGCTGCGCTGCTGCTATCTTTAGAGGGGAAAAGAGGAATAAGC
CCCCAGACAGGGGAGTGGGCTTGTTTGTGACTTCACCAAAGGTCAGGGCCCAAG
GGGGTTCGCGTTGCTAGGCCACCTTCTCAGTCCAGCGCGTTTACGTAAGCCAGAC
AGCAGCCAATTGTCAGTTCTAGGGAGGGGGACCACTGCCCCTGGTATAAAGTGG

TCCTGCAGCTATTTCTGGTCGCATCAGAGCGCCAGGAGTCCACACAAATGTAAGA
GGGGGTCTTCTACCTCTCCCTAGCCCTCCGCCCCCTCCAAGGACTCGGGCCCAGT
TTCTAACTTTTCCCCTTCCCTCCCTCGTCTTGCCCTGCGCCCGGGGCCACCTTCAT
CACCGTCGCTGACTCCGCCATCCAAGCCTAGGGGAGACCGAAGTGAAGGCCCTG
GACCAACCCGGCCCGGGCCCCCCGGTATCGGGCCAGAGGTAAGTGGACTTTAAT
TTTTTCTGCTAAGCCCAACACTCCACCACACCCAGGCACACACTACACACACCCA
CCCGTCTCAGGGTCCCCTCGGACAGCTCCTAAGAAGGCACCGGTCGCCCAGTCCT
ACCAGAGGGGGCCAAGAACCCAGACGAGTCCGTAGAAGGGTCCTCGTCCAGCAA
GAAGAGGAGGTGGTAAGCGGTTCACCTTCAGGGGTAAGTAACCTGACCTCTCCA
GGGCTCACATAAAGGGAGGCTTAGTATACATGCTTCTTGCTTTTCACAGGAACCT
GGGGGCTAGTCTGGGTGGGATTAGGCTGCCTCAAGTTGCATCAGCCAGGGCTTC
ATGCCCTCCTCAGTTCCCTAGTCCCCGGGCTTCAGGCCCCCTCCGTCCCCGTCCTC
CAGAGACCCGGGCTTCAGGCCCTGCCTCTCCTGTTACCCTTTTAGAACCACAGCC
TGGACACATGTGCCAGACGCCTTGGCCTCTAAGGCCCTCGGGTCCCCCTGGACCC
CGGCCTCAGCAACCCTGCTGCTCCCCTCCTGCCACCCCAGCCTCCCCCCCTCCCC
GTCCCCCTTCGCTCCTGATCCTCCCCCGGTCCCCAGTAGGGCCGCCTGCCCCCCTG
CACCCAGTACCTGCCCCTCTTGGCCACGCACCCCGGGCCAGGCCACCTTAGACCC
GGCCAAGCCCCATCCCTGAAGACCCAGCGGCCATTCTCTCTGGTAACGAGCAGA
GAAGAAGTAGAGGCCCGCGGCCATTGGGCCCAGATTGAGAGACCAGTCCAGGG
GCCCGAGGTTGGAGCCAGCGGGCACCCGAGGTCCCAGCACCCGGTCCCTCCGGG
GGGCAGAGACAGGCAGGGCCCCCCGGCAGCTGGCCCCGAGGAGGCGCCCGGAG
TGGGGCCGGTCGGCTGGGCTGGCCGAGCCCGGGTCTGGGAGGTCTGGGGTGGCG
AGCCTGCTGTCTCAGGAGGGGCCTGGCTCCGCCGGGTGGCCCTGGGGTAAGTCT
GGGAGGCAGAGGGTCGGCCTAGGCCCGGGGAAGTGGAGGGGGATCGCCCGGGT
CTCTGTTGGCAGAGTCCGGGCGATCCTCTGAGACCCTCCGGGCCCGGACGGTCGC
CCTCAGCCCCCCAGACAGACCCCAGGGTCTCCAGGCAGGGTCCGGCATCTTCAG
GGGCAGCAGGCTCACCACCACAGGCCCCCCAGACCCGGGTCTCGGCCAGCCGAG
CCGACCGGCCCCGCGCCTGGCGCCTCCTCGGGGCCAGCCGCCGGGGTTGGTTCTG
CCCCTCTCTCTGTCCTTCAGAGGAACCAGGGACCTCGGGCACCCCAGAGCCCCTC
GGGCCCGCCTCCAGGCGCCCTCCTGGTCTCCGCTCCCCTCTGAGCCCCGTTAAAC
CCAAAGAATGTCTGAGGGGAGCCACCCTCGGGGCCCAGGCCCCAGAGTCCAGAG
GTCAGGGGCACCTCAGGGTGCCTCCCCGGGTCCCAGGCCAGCCGGAGGGACCCC
GGCAGCCCGGGCGGCCCCAGAGGCCGGTTCCTCGCCCCTTCCCCGGGCTTCAGA
GCCCAGGATGTCCCCCAGAAGGACCCTAGGCGTCCCCTCTCCTCCCCTCCAGGC

CCGAGCCTCTCCCTCGCGGAGAGGGGCCTCTTTGGGCCCTCAAGTCCAGCCCCAC
CGAGACCCGAGTGGCCC (SEQ ID NO.: 5).

There has been evidence showing EBV has evolved to utilize DNA methylation to maximize persistence and to protect itself from immune detection. Moreover, methylation of EBV *Bam*HI-W fragment is important to its expression as a W promoter that drives expression of the EB viral nuclear antigens (EBNAs) at the initiation of virus-induced B-cell transformation. The role of methylation in EBV activity has also been recognized in human tissue. Pharmacologic reversal of dense CpG methylation in tumor tissue can be achieved in patients undergoing treatment with DNA methyltransferase inhibitor. On the other hand, the CpG island of EBV *Bam*HI-W has been shown to be unmethylated in the latent cycle of EBV. Despite the variance in the methylation status of the target nucleic acid sequence within the CpG island of EBV, the method as disclosed herein can be used for the detection and/or quantification of such a target sequence.

In some examples, the CpG island of the *Bam*HI-W region of EBV is selected from the group consisting of:
CTCCTCTCCAACCTTCGCTCCACCCTAGACCCCAGCTTCTGGCCTCCCCGGGTCCACCAGGCCAGCCGGAGGG
ACCCCGGCAGCCCGGGCGAGTCGCCTTCCCTCTCCCCTGGCCTCTCCTTCCCGCCTCCCACCCGAGCCCCCTC
AGCTTGCCTCCCCACCGGGTCCATCAGGCCGGCCGGAGGGACCCCGGCGGCCCGGTGTCA (SEQ ID NO.: 6)(nucleotides at position number 36 to 241 of *Bam*HI-W region),
AGGCCATGCGCGCCCTGTCACCAGGCCTGCCAAAGAGCCAGATCTAAGGCCGGGAGAGGCAGCCCCAAAGCG
GGTGCAGTAACAGGTAATCTCTGGTAGTGATTTGGACCCGAAATCTGACACTTTAGAGCTCTGGAGGACTTTAAA
ACTCTAAAAATCAAAACTTTAGAGGCGAATGGGCGCCATTTTGTCCCCACGCGCGCATAATGGCGGACCTAGGC
CTAAAACCCCCAGGAAGCGGGTCTATGGTTGGCTGCGCTGCTGCTATCTTTAGAGGGGAAAAGAGGAATAAGCC
CCCAGACAGGGGAGTGGGCTTGTTTGTGACTTCACCAAAGGTCAGGGCCCAAGGGGGTTCGCGTTGCTAGGCC
ACCTTCTCAGTCCAGCGCGTTTACGTAAGC (SEQ ID NO.: 7) (nucleotides at position number 691 to 1088 of *Bam*HI-W region),
CTGGTATAAAGTGGTCCTGCAGCTATTTCTGGTCGCATCAGAGCGCCAGGAGTCCACACAAATGTAAGAGGGGG
TCTTCTACCTCTCCCTAGCCCTCCGCCCCCTCCAAGGACTCGGGCCCAGTTTCTAACTTTTCCCCTTCCCTCCCT
CGTCTTGCCCTGCGCCCGGGGCCACCTTCATCACCGTCGCTGACTCCGCCATCCAAGCCTAGGGGAGACCG
AAGTGAAGGCCCTGGACCAACCCGGCCCGGGCCCCCCGGTATCGGGCCAGAGGTAAGTGGACTTTAATTTTTT
CTGCTAAGCCCAACACTCCACCACACCCAGGCACACACTACACACACCCACCCGTCTCAGGGTCCCCTCGGA
(SEQ ID NO.: 8) (nucleotides at position number 1134 to 1498 of *Bam*HI-W region), and
CGAGGAGGCGCCCGGAGTGGGGCCGGTCGGCTGGGCTGGCCGAGCCCGGGTCTGGGAGGTCTGGGGTGGC
GAGCCTGCTGTCTCAGGAGGGGCCTGGCTCCGCCGGGTGGCCCTGGGGTAAGTCTGGGAGGCAGAGGGTCG
GCCTAGGCCCGGGGAAGTGGAGGGGGATCGCCCGGGTCTCTGTTGGCAGAGTCCGGGCGATCCTCTGAGACC
CTCCGGGCCCGGACGGTCGCCCTCAGCCCCCCAGACAGACCCCAGGGTCTCCAGGCAGGGTCCGGCATCTTC
AGGGGCAGCAGGCTCACCACCACAGGCCCCCCAGACCCGGGTCTCGGCCAGCCGAGCCGACCGGCCCCGCG
CCTGGCGCCTCCTCGGGGCCAGCCGCCGGGGTTGGTTCTGCCCCTCTCTCTGTCCTTCAGAGGAACCAGGGAC
CTCGGGCACCCCAGAGCCCCTCGGGCCCGCCTCCAGGCGCCCTCCTGGTCTCCGCTCCCCTCTGAGCCCCGT
TAAACCCAAAGAATGTCTGAGGGGAGCCACCCTCGG (SEQ ID NO.: 9) (nucleotides at position number 2278 to 2814 of *Bam*HI-W region).

In some examples, the target sequence within the CpG island comprises the sequence selected from the group consisting of:
AGATCTAAGGCCGGGAGAGGCAGCCCCAAAGCGGGTGCAGTAACAGGTAATCTCTGGTAGTGATTTGGACCCG
AAATCTGACACTTTAGAGCTCTGGAGGACTTTAAAACTCTAAAAATCAAAACTTTAGAGGCGAATGGGCG (SEQ ID NO.: 1) (nucleotides at position number 730 to 872 of *Bam*HI-W region),
GGAATAAGCCCCCAGACAGGGGAGTGGGCTTGTTTGTGACTTCACCAAAGGTCAGGGCCCAAGGGGGTTCGCG
TTGCTAGGCCACCTTCTCAGTCCAGCGCGTTTACGTAA (SEQ ID NO.: 10) (nucleotides at position number 976 to 1086 of *Bam*HI-W region),
AGGAAGCGGGTCTATGGTTGGCTGCGCTGCTGCTATCTTTAGAGGGGAAAAGAGGAATAAGCCCCCAGACAGG
GGAGTGGGCTTGTTTGTGACTTCACCAAAGGTCAGGGCCCAAGGGGGTTCGCGTTGCTAG-
GCCACCTTCTCAGTC (SEQ ID NO.: 11) (nucleotides at position number 923 to 1070 of *Bam*HI-W region), a complementary sequence, a fragment and a variant thereof.

The term "fragment" as used herein refers to a nucleic acid sequence that is a constituent of the reference, or a constituent of the complementary sequence of the reference sequence. In some examples, a fragment comprises about 9 to about 300 nucleotides, or about 10 to about 290 nucleotides, or about 20 to about 280 nucleotides, or about 30 to about 270 nucleotides, or about 40 to about 260 nucleotides, or about 50 to about 250 nucleotides, or about 60 to about 240 nucleotides, or about 70 to about 230 nucleotides, or about 80 to about 220 nucleotides, or about 90 to about 210 nucleotides, or about 100 to about 200 nucleotides, or about 110 to about 190 nucleotides, or about 120 to about 180 nucleotides, or about 130 to about 170 nucleotides, or about 140 to about 160 nucleotides, or 10 nucleotides, 15 nucleotides, 25 nucleotides, or 35 nucleotides, or 45 nucleotides, or 55 nucleotides, or 65 nucleotides, or 75 nucleotides, or 85 nucleotides, or 95 nucleotides, or 105 nucleotides, or 115 nucleotides, or 125 nucleotides, or 135 nucleotides, or 145 nucleotides, or 155 nucleotides, or 165 nucleotides, or 175 nucleotides, or 185 nucleotides, or 195 nucleotides, or 205 nucleotides, or 215 nucleotides, or 225 nucleotides, or 235 nucleotides, or 245 nucleotides, or 255 nucleotides, or 265 nucleotides, or 275 nucleotides, or 285 nucleotides, or 295 nucleotides of the reference sequence or the complementary sequence of the reference sequence . A fragment can also comprise about 5% to about 95%, about 10% to about 90%, about 15% to about 85%, about 20% to about 80%, about 25% to about 75%, about 30% to about 70%, about 35% to about 65%, about 40% to about 60%, about 45% to about 55%, or about 50% of the reference sequence or the complementary sequence of the reference sequence.

The term "variant" as used herein refers to sequences that are substantially similar to the reference sequence. These nucleotide sequence variants may have at least 25%, 30%, 35%, 40%, 45%, 50%, 55%, 60%, 65%, 70%, 75%, 80%, 85%, 90%, 95%, 96%, 97%, 98% or 99% sequence identity to the "non-variant" reference sequence. Variants may be a result of substitution, deletion or additional of any number of nucleotides in the refernce sequence as a result of the mutation of the wild type DNA .

In one example, the amplification of the target sequence comprises the use of at least one oligonucleotide primer capable of binding to the target sequence. The at least one oligonucleotide primer binds within a region from about 9 to about 300 nucleotides, or about 10 to about 290 nucleotides, or about 20 to about 280 nucleotides, or about 30 to about 270 nucleotides, or about 40 to about 260 nucleotides, or about 50 to about 250 nucleotides, or about 60 to about 240 nucleotides, or about 70 to about 230 nucleotides, or about 80 to about 220 nucleotides, or about 90 to about 210 nucleotides, or about 100 to about 200 nucleotides, or about 110 to about 190 nucleotides, or about 120 to about 180 nucleotides, or about 130 to about 170 nucleotides, or about 140 to about 160 nucleotides, from the first nucleotide of the target sequence. The length of the at least one primer is between 5 to 40 nucleotides, or between 10 to 35 nucleotides, or between 15 to 30 nucleotides, or between 20 to 25 nucleotides, or 8 nucleotides, or 9 nucleotides, or 10 nucleotides, or 12 nucleotides, or 14 nucleotides, or 16 nucleotides, or 18 nucleotides, or 20 nucleotides, or 22 nucleotides, or 24 nucleotides, or 26 nucleotides, or 28 nucleotides, or 30 nucleotides, or 32 nucleotides, or 34 nucleotides, or 36 nucleotides, or 38 nucleotides, or 40 nucleotides. In one specific example, the length of the at least one primer is 20 nucleotides.

In one example, the at least one primer has a sequence identity of at least 70%, at least 75%, at least 80%, at least 85%, at least 90%, at least 95%, at least 96%, at least 97%, at least 98% or at least 99% to at least one sequence selected from the group consisting of AGATCTAAGGCCGGGAGAGG (SEQ ID NO.: 2), GGAATAAGCCCCCAGACAGG (SEQ ID NO.: 12), AGGAAGCGGGTCTATGGTTG (SEQ ID NO.: 13), CGCCCATTCGCCTCTAAAGT (SEQ ID NO.: 3), TTACGTAAACGCGCTGGACT (SEQ ID NO.: 14), and GACTGAGAAGGTGGCCTAGC (SEQ ID NO.: 15), or a complementary sequence thereof. In one example, the primer comprises one sequence selected from the group consisting of AGATCTAAGGCCGGGAGAGG (SEQ ID NO.: 2), GGAATAAGCCCCCAGACAGG (SEQ ID NO.: 12), AGGAAGCGGGTCTATGGTTG (SEQ ID NO.: 13), CGCCCATTCGCCTCTAAAGT (SEQ ID NO.: 3), TTACGTAAACGCGCTGGACT (SEQ ID NO.: 14), and GACTGAGAAGGTGGCCTAGC (SEQ ID NO.: 15), or a complementary sequence thereof.

In another example, the amplification of the target sequence comprises the use of at least one pair of oligonucleotide primers. The at least one pair of oligonucleotide primers can comprise one forward primer and one reverse primer. In some examples, the at least one pair of oligonucleotide primers can be selected from the group consisting of: forward primer AGATCTAAGGCCGGGAGAGG (SEQ ID NO.: 2) and reverse primer CGCCCATTCGCCTCTAAAGT (SEQ ID NO.: 3); forward primer GGAATAAGCCCCCAGACAGG (SEQ ID NO.: 12) and reverse primer TTACGTAAACGCGCTGGACT (SEQ ID NO.: 14); forward primer AGGAAGCGGGTCTATGGTTG (SEQ ID NO.: 13) and reverse primer GACTGAGAAGGTGGCCTAGC (SEQ ID NO.: 15). In one specific example, the at least one pair of oligonucleotide primers are forward primer AGATCTAAGGCCGGGAGAGG (SEQ ID NO.: 2) and reverse primer CGCCCATTCGCCTCTAAAGT (SEQ ID NO.: 3).

In one example, the amplification of the target sequence comprises the use of a probe capable of binding to the target sequence. The probe binds within a region from between 9 to 500 nucleotides, or from between 10 to 490 nucleotides, or from between 20 to 480 nucleotides, or from between 30 to 470 nucleotides, or from between 40 to 460 nucleotides, or from between 50 to 450 nucleotides, or from between 60 to 440 nucleotides, or from between 70 to 430 nucleotides, or from between 80 to 420 nucleotides, or from between 90 to 410 nucleotides, or from between 100 to 400 nucleotides, or from between 110 to 390 nucleotides, or from between 120 to 380 nucleotides, or from between 130 to 370 nucleotides, or from between 140 to 360 nucleotides, or from between 150 to 350 nucleotides, or from between 160 to 340 nucleotides, or from between 170 to 330 nucleotides, or from between 180 to 320 nucleotides, or from between 190 to 310 nucleotides, or from between 200 to 300 nucleotides, or from between 210 to 290 nucleotides, or from between 220 to 280 nucleotides, or from between 230 to 270 nucleotides, or from between 240 to 260 nucleotides, or 8 nucleotides, 15 nucleotides, 25 nucleotides, or 35 nucleotides, or 45 nucleotides, or 55 nucleotides, or 65 nucleotides, or 80 nucleotides, or 90 nucleotides, or 100 nucleotides, or 110 nucleotides, or 120 nucleotides, or 130 nucleotides, or 140 nucleotides, or 150 nucleotides, or 160 nucleotides, or 170 nucleotides, or 180 nucleotides, or 190 nucleotides, or 200 nucleotides, or 210 nucleotides, or 220 nucleotides, or 230 nucleotides, or 240 nucleotides, or 250 nucleotides, or 260 nucleotides, or 270 nucleotides, or 280 nucleotides, or 290 nucleotides, or 300 nucleotides, or 310 nucleotides, or 320 nucleotides, or 330 nucleotides, or 340 nucleotides, or 350 nucleotides, or 360 nucleotides, or 370 nucleotides, or 380 nucleotides, or 390 nucleotides, or 400 nucleotides, or 410 nucleotides, or 420 nucleotides, or 430 nucleotides, or 440 nucleotides, or 450 nucleotides, or 460 nucleotides, or 470 nucleotides, or 480 nucleotides, or 490 nucleotides, or 500 nucleotides from a specific nucleotide of the target sequence. The specific nucleotide can be nucleotide at position number 1, 5, 10, 15, 20, 25, 30, 35, 40, 45, 50, 52, 54, 56, 58, 60, 62, 64, 66, 68, 70, 72, 74, 76, 78, 80, 82, 84, 86, 88, 90, 92, 94, 96, 98, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490 or 500 of the target sequence. The length of a probe can be between 5 to 40 nucleotides, or between 10 to 35 nucleotides, or between 15 to 30 nucleotides, or between 20 to 25 nucleotides, or 8 nucleotides, or 9 nucleotides, or 10 nucleotides, or 12 nucleotides, or 14 nucleotides, or 16 nucleotides, or 18 nucleotides, or 20 nucleotides, or 22 nucleotides, or 24 nucleotides, or 26 nucleotides, or 28 nucleotides, or 30 nucleotides, or 32 nucleotides, or 34 nucleotides, or 36 nucleotides, or 38 nucleotides, or 40 nucleotides.

In one example, the probe has a sequence identity of at least 70%, at least 75%, at least 80%, at least 85%, at least 90%, at least 95%, at least 96%, at least 97%, at least 98% or at least 99% to a sequence selected from the group consisting of CTCTGGTAGTGATTTGGACCCGAAATCTG (SEQ ID NO.: 16), CCACCTTCTCAGTCCAGCGCGTTT (SEQ ID NO.: 17), GTGACTTCACCAAAGGTCAGGGCCC (SEQ ID NO.: 18), GGTGGTAAGCGGTTCACCTTCAGGG (SEQ ID NO.: 19), and complementary sequences thereof. In one example, the probe comprises the sequence selected from the group consisting of CTCTGGTAGTGATTTGGACCCGAAATCTG (SEQ ID NO.: 16), CCACCTTCTCAGTCCAGCGCGTTT (SEQ ID NO.: 17), GTGACTTCACCAAAGGTCAGGGCCC (SEQ ID NO.: 18), GGTGGTAAGCGGTTCACCTTCAGGG (SEQ ID NO.: 19) and complementary sequences thereof. In one specific example, the probe comprises the sequence of CTCTGGTAGTGATTTGGACCCGAAATCTG (SEQ ID NO.: 16).

In one example, the amplification of the target sequence comprises the use of at least one oligonucleotide primer and one probe as defined above. In another example, the amplification of the target sequence comprises the use of at least one pair of oligonucleotide primers and one probe as defined above.

In one aspect, there is provided a method for detecting and/or quantifying the presence of a target nucleic acid sequence of Epstein-Barr virus (EBV) in a sample obtained from a subject, comprising amplifying a target sequence in the *Bam*HI-W region of EBV, wherein the target sequence comprises the sequence of AGATCTAAGGCCGGGAGAGGCAGCCCCAAAGCGGGTGCAGTAACAGGTAATCTC TGGTAGTGATTTGGACCCGAAATCTGACACTTTAGAGCTCTGGAGGACTTTAAAA CTCTAAAAATCAAAACTTTAGAGGCGAATGGGCG (SEQ ID NO.: 1), wherein amplifying the target sequence comprises the use of a pair of oligonucleotide primers and a probe, wherein the first oligonucleotide primer comprises the sequence of 5'-AGATCTAAGGCCGGGAGAGG-3' (SEQ ID NO.: 2), and the second oligonucleotide primer comprises the sequence of 5'-CGCCCATTCGCCTCTAAAGT-3' (SEQ ID NO.: 3), and wherein the probe comprises the sequence of 5'-(6-FAM)CTCTGGTAGTGATTTGGACCCGAAATCTG(TAMRA)-3' (SEQ ID NO.: 4), and wherein the method is a quantitative polymerase chain reaction (qPCR).

In some examples, the method of detecting and/or quantifying the presence of a target nucleic acid sequence of Epstein-Barr virus (EBV) in a sample obtained from a subject further comprises amplifying a control.

The term "control" or "internal control" as used herein refers to a reference sequence which can be used to indicate whether the amplification system is functioning.

When a control is included in the amplification system but is not successfully amplified, it indicates that the amplification result is false-negative. When there is no control spiked in samples, it is not possible to differentiate true negative and false negative. When a control is spiked and successfully amplified but no target signal in the tested samples, the results are true negative. When internal control is spiked and no signals from both internal control and tested samples, the results are false negative. Thus using a control allows one to distinguish false negative results which are common in clinical samples in which many PCR inhibitors are present. False negative results have great impacts on patients' lives, such as failure of disease diagnosis which will delay treatment and might result in treatment failure.

In some examples, the control includes a target sequence of TTAGCAGCGACGAAGATCATGCGCTCACGCTCTCGGTGTCCTCATTCATCAGTTA TTCACAACGCTATGCTGTAACTCGACCTGACAAGACTGTACCTATGAGAAGGCA CTTGCTACCTTATGCAAGCGTCAGCCCGCGGTATCGCTTGG (SEQ ID NO.: 80), a complementary sequence, a fragment or a variant thereof.

In some examples, amplifying the control includes the use of a pair of oligonucleotide primers. Examples of a forward primer sequence is 5'-CGCTCTCGGTGTCCTCATTC-3' (SEQ ID NO.: 81), a complementary sequence, a fragment or a variant thereof. Examples of a reverse primer sequence is 5'-GGCTGACGCTTGCATAAGGT-3' (SEQ ID NO.: 82), a complementary sequence, a fragment or a variant thereof.

In some other examples, amplifying the control further includes the use of a probe capable of binding to the target sequence of the control. In one example, the probe comprises the sequence of CACAACGCTATGCTGTAACTCGACCTGAC (SEQ ID NO.: 89), a complementary sequence, a fragment or a variant thereof. One specific example of a probe is 5'-VIC-CACAACGCTATGCTGTAACTCGACCTGAC-TAMRA-3' (SEQ ID NO.: 83).

In one example, the microorganism of which a target nucleic acid sequence is to be detected and/or quantified using the method as disclosed herein is a bacterium. One specific example of such a bacterium is *Mycobacterium*

*tuberculosis.*

The following are some non-exclusive examples of CpG islands in the genomic nucleic acid sequences of *Mycobacterium tuberculosis* as predicted using CpG island analytical software:

| CpG island serial no. | Start position (nucleotide number) | End position (nucleotide number) |
|---|---|---|
| 1 | 250 | 485,607 |
| 2 | 486,395 | 598,721 |
| 3 | 598,929 | 889,723 |
| 4 | 890,230 | 1,103,130 |
| 5 | 1,103,439 | 1,691,493 |
| 6 | 1,691,745 | 1,697,185 |
| 7 | 1,697,464 | 1,779,283 |
| 8 | 1,779,789 | 2,268,374 |
| 9 | 2,268,597 | 2,367,062 |
| 10 | 2,367,073 | 2,680,742 |
| 11 | 2,681,249 | 2,807,592 |
| 12 | 2,807,742 | 3,587,732 |
| 13 | 3,588,238 | 3,791,614 |
| 14 | 3,791,647 | 3,792,550 |
| 15 | 3,793,135 | 3,798,679 |
| 16 | 3,798,717 | 3,964,564 |
| 17 | 3,965,008 | 4,411,274 |

The above identified 17 CpG islands accounts for 99.9% of the genomic nucleic acid sequence of *Mycobacterium tuberculosis.*

Some non-limiting examples of the target sequences to be used for the detection and/or quantification of *Mycobacterium tuberculosis* are:

**>ST-E00142:243:HVLMVCCXX:8:1101:23043:33111/1**

GGTCGCCGCGGGCAGGCTCAACCCGGAGCGGATCACCGAATCCACGATCGCCCG
CCACCTGCAGCGACCCGACATTCCCGACGTTGACCTCTTCCTGCGGACCTCGGGT
GAGCAGCGCTCCAGCAACTTCATGCTGTGGCAGGCGGCCTA (SEQ ID NO.: 20)

(Nucleotide 2,642,836 - 2,642,687)

**>ST-E00142:243:HVLMVCCXX:8:1101:23043:33111/2**

CCTCGCAGGCCGCCCACAAGTCGCGGCGGTCATAGTCGGGCCAGAGCTTGTCCT
GGAATATGTATTCAGCGTAGGCCGCCTGCCACAGCATGAAGTTGCTGGAGCGCT
GCTCACCCGAGGTCCGCAGGAAGAGGTCAACGTCGGGAATGT (SEQ ID NO.: 21)

(Nucleotide 2,642,616 - 2,642,765)

**>ST-E00142:243:HVLMVCCXX:8:1101:24068:32601/1**

GGTCGCCGCGGGCAGGCTCAACCCGGAGCGGATCACCGAATCCACGATCGCCCG CCACCTGCAGCGACCCGACATTCCCGACGTTGACCTCTTCCTGCGGACCTCGGGT GAGCAGCGCTCCAGCAACTTCATGCTGTGGCAGGCGGCCTA (SEQ ID NO.: 22)

(Nucleotide 2,642,836 - 2,642,687)
**>ST-E00142:243:HVLMVCCXX:8:1101:24068:32601/2**

CCTCGCAGGCCGCCCACAAGTCGCGGCGGTCATAGTCGGGCCAGAGCTTGTCCT GGAATATGTATTCAGCGTAGGCCGCCTGCCACAGCATGAAGTTGCTGGAGCGCT GCTCACCCGAGGTCCGCAGGAAGAGGTCAACGTCGGGAATGT (SEQ ID NO.: 23)

(Nucleotide 2,642,616 - 2,642,765)
**>ST-E00142:243:HVLMVCCXX:8:1103:21836:42622/1**

TGGTGGTCGGCTCGCGGCCGGTGTCGAGATCACCGAACCGCAAAGAAGTGTGGT CGTCTTCGACACCGCACCCACCGCCCTGCTGCGGGTTTACCGCGACAAGCTTCC (SEQ ID NO.: 24)

(Nucleotide 3,355,782 - 3,355,889)
**>ST-E00142:243:HVLMVCCXX:8:1103:21836:42622/2**

GGAAGCTTGTCGCGGTAAACCCGCAGCAGGGCGGTGGGTGCGGTGTCGAAGACG ACCACACTTCTTTGCGGTTCGGTGATCTCGACACCGGCCGCGAGCCGACCACCA (SEQ ID NO.: 25)

(Nucleotide 3,355,889 - 3,355,782)
**>ST-E00142:243:HVLMVCCXX:8:1111:4980:34799/1**

GATGTCCGGGCTCACCCCGGCGTGGTCGGCGGCGAACAACGCGCCGGTGCGGCC GAAGCCGGTGGCGATCTCATCGAAGATCAGCAGCACCTCGTAACGGCGGCAGAT GTCCCGCAGGTCGTGCAGATAGCGCGGGTCGTGAAAACGCAT (SEQ ID NO.: 26)

(Nucleotide 1,776,225 - 1,776,076)
**>ST-E00142:243:HVLMVCCXX:8:1111:4980:34799/2**

GCACGCCGGCGAGCTGGCCGCGGTGGTCGTGGAGCCGGTCGTGCAGGGTGCGGG CGGTATGCGTTTTCACGACCCGCGCTATCTGCACGACCTGCGGGACATCTGCCGC CGTTACGAGGTGCTGCTGATCTTCGATGAGATCGCCACCGG (SEQ ID NO.: 27)

(Nucleotide 1,776,018 - 1,776,167)
**>ST-E00142:243:HVLMVCCXX:8:1203:1895:39335/1**

GGTATGGTCGCCGCCGCGACGCCGCTGCCGGTGATCGGGGTGCCCGTACCGCTG GGCAGGCTGGACGGGCTTGACTCCCTGCTGTCGATCGTGCAAACGCCGGCCGGG GGTCCGGTGGCCACGCTCCCCATCGGGGGCGCCGGTAAGGCC (SEQ ID NO.: 28)

(Nucleotide 3,658,407 - 3,658,258)
**>ST-E00142:243:HVLMVCCXX:8:1203: 1895:39335/2**

CCTCGGGCAGCTCGAACAGATCGAACGACGGGTTTCCGGCCCATCCGACCATCTT GGAGCCCTCCTAATCTCCGGGCTAGTCGCGGGTTAACTTACCCGGCGGCCGCTCC ACTTCCGCATCCTTGGCCGCCACGCCGTCGGCGAGCCGGT (SEQ ID NO.: 29)

(Nucleotide 3658038 - 3658187)
**>ST-E00142:243:HVLMVCCXX:8:1203:25976:49127/1**

GTCATGCTGATCGCGGTGCTCGCTCGGCTGATGATGCGCGGCTGGCGGCGCCGTT CG (SEQ ID NO.: 30)

(Nucleotide 1555525 - 1555581)
**>ST-E00142:243:HVLMVCCXX:8:1203:25976:49127/2**

CGAACGGCGCCGCCAGCCGCGCATCATCAGCCGAGCGAGCACCGCGATCAGCAT GAC (SEQ ID NO.: 31)

(Nucleotide 1555581 - 1555525)
**>ST-E00142:243:HVLMVCCXX:8:1206:20740:34570/1**

TGCATGGCTGTCGTCAGCTCGTGTCGTGAGATGTTGGGTTAAGTCCCGCAACGAG CGCAACCCTTGTCATTAGTTGC (SEQ ID NO.: 32)

(Nucleotide 1472888 - 1472955)
**>ST-E00142:243:HVLMVCCXX:8:1207:9587:30334/1**

CCGGCTGGCGCAGCCGATACACCTCGCGCCACATCATGCCGGCCTCGAAGTCGG CTCCGGCGAACGACTTT (SEQ ID NO.: 33)

(Nucleotide 4010375 - 4010445)
**>ST-E00142:243:HVLMVCCXX:8:1207:9587:30334/2**

AAAGTCGTTCGCCGGAGCCGACTTCGAGGCCGGCATGATGTGGCGCGAGGTGTA TCGGCTGCGCCAGCCGG (SEQ ID NO.: 34)

(Nucleotide 4010445 - 4010375)
**>ST-E00142:243:HVLMVCCXX:8:1211:11373:3366/1**

ACCCGCGGATACGGGTCCGGTACCGCCAACCGCGGCGCCGACGGCCGCCGGCGC CGCGGCGGCAGCTTCGGCGGCCTCGGGACCGATCCATCCCAGCGCCCGCCACGA TGTAATAAGGCTGTTGCCAATACCAATGGCGAAATATGGCAA (SEQ ID NO.: 35)

(Nucleotide 1931752 - 1931901)
**>ST-E00142:243:HVLMVCCXX:8:1211:11373:3366/2**

CCGGGGTGGCTGGAATGGTTCATCAACTGGTATCTGCCGATATCACAGCTGTTTT ACAACACCGTGGGTTTGCCATATTTCGCCATTGGTATTGGCAACAGCCTTATTAC ATCGTGGCGGGCGCTGGGATGGATCGGTCCCGAGGCCGCC (SEQ ID NO.: 36)

(Nucleotide 1931970 - 1931821)
**>ST-E00142:243:HVLMVCCXX:8:1211:29498:4280/1**

CAGGTAGCCGGTCAGCCGGATTGGGTCTCGTTGCGCCGGCAGGTGACGGTCGCG CAGCGAAAAAGCGACCTGCGGGCCGCCGAGGATCCGATCGACGCCGTCGTATGC GCC (SEQ ID NO.: 37)

(Nucleotide 869238 - 869128)
**>ST-E00142:243:HVLMVCCXX:8:1211:29498:4280/2**

GGCGCATACGACGGCGTCGATCGGATCCTCGGCGGCCCGCAGGTCGCTTTTTCGC TGCGCGACCGTCACCTGCCGGCGCAACGAGACCCAATCCGGCTGACCGGCTACC TG (SEQ ID NO.: 38)

(Nucleotide 869128 - 869238)
**>ST-E00142:243:HVLMVCCXX:8:1211:3518:26255/1**

TCACATCCGGGTGGCGGGCAGCCGTCGTCTGCAACGAGGCCGACGGTAGGCGAT GGGTGAGGCCATGGAGGGCAAATGTCCCCATGGTGTATCGATTACGTACCCTCTT ACTTCGTCGGGGTCGCATCGGCATTCGCCTTGCCCGCCTGT (SEQ ID NO.: 39)

(Nucleotide 1934277 - 1934128)
**>ST-E00142:243:HVLMVCCXX:8:1211:3518:26255/2**

CGGGCGACACACCGTCCCGATACATGTCAGCAACCGGGTCGATCGTGGTGAATG CACAGGCGGGCAAGGCGAATGCCGATGCGACCCCGACGAAGTAAGAGGGTACG TAATCGATACACCATGGGGACATTTGCCCTCCATGGCCTCACC (SEQ ID NO.: 40)

(Nucleotide 2023240 - 2023389)
**>ST-E00142:243:HVLMVCCXX:8:1214:4706:5950/1**

ACCGGTGTCTTCTGCCATCAACAGGCCTCGACCGCGGGGCAGCGGGCCGCCCTTC ATCTTGCCGCGAATGAAGCCCTCGTCGGGATCGGCGTCCATCACCAGCAGTGGC GCATTGGCCTGATGCAGGGCCCGCAACATCGGGTCGCTGCC (SEQ ID NO.: 41)

(Nucleotide 2023393 - 2023244)
**>ST-E00142:243:HVLMVCCXX:8:1214:4706:5950/2**

AGCCGGCAGCGACCCGATGTTGCGGGCCCTGCATCAGGCCAATGCGCCACTGCTGGTGATGGACGCCGATCCCGACGAGGGCTTCATTCGCGGCAAGATGAAGGGCGGCCCGCTGCCCCGCGGTCGAGGCCTGTTGATGGCAGAAGACAC (SEQ ID NO.: 42)

(Nucleotide 2023240 - 2023389)
**>ST-E00142:243:HVLMVCCXX:8:1215:29812:28664/1**

AGAACACCGTCGAATGCATGCAAGCCGGTGCGGTGTTCGGCTTCGCCGGGCTGGTAGACGGGTTGGTAG (SEQ ID NO.: 43)

(Nucleotide 4043309 - 4043241)
**>ST-E00142:243:HVLMVCCXX:8:1215:29812:28664/2**

CTACCAACCCGTCTACCAGCCCGGCGAAGCCGAACACCGCACCGGCTTGCATGCATTCGACGGTGTTCT (SEQ ID NO.: 44)

(Nucleotide 4043241 - 4043309)
**>ST-E00142:243:HVLMVCCXX:8:1222:8197:67234/1**

CGGGGTCATGGTCGCTCTATGCCTCGGCGGCGGCGTTTTCGGGCTGAGCCTCGGCAAGCACGTCACGCAGAGCGGCTTCTACGACGACGGCAGCCAATCGGTGCAAGCATCG (SEQ ID NO.: 45)

(Nucleotide 247268 - 247157)
**>ST-E00142:243:HVLMVCCXX:8:1222:8197:67234/2**

CGATGCTTGCACCGATTGGCTGCCGTCGTCGTAGAAGCCGCTCTGCGTGACGTGCTTGCCGAGGCTCAGCCCGAAAACGCCGCCGCCGAGGCATAGAGCGACCATGACCCCG (SEQ ID NO.: 46)

(Nucleotide 247157 - 247268)
**>ST-E00142:243:HVLMVCCXX:8:1223:32826:24216/1**

ACGATCTGCCGGGCGGCGGCATGTGTGCGCGGCGCGATGCCATCGACGAGGCGCGCCAACCGCGGCGCCGGAACCGCCAGGATGACGGCGTCGGCCTGCCAGCGGCCGCCGGTTTCGTCGCGCAGCACCCAGCCGCGTTCGAGCTGGACC (SEQ ID NO.: 47)

(Nucleotide 2993118 - 2993267)
**>ST-E00142:243:HVLMVCCXX:8:1223:32857:24233/1**

ACGATCTGCCGGGCGGCGGCATGTGTGCGCGGCGCGATGCCATCGACTAGGCGCGCCAACCGCGGCGCCGGAACCGCCAGGATGACGGCGTCGGCCTGCCAGCGGCCGCCGGTTTCGTCGCGCAGCACCCAGCCGCGTTCGAGCTGGACC (SEQ ID NO.: 48)

(Nucleotide 2993118 - 2993267)
**>ST-E00142:243:HVLMVCCXX:8:2105:14519:48757/1**

TCACATCCGGGTGGCGGGCAGCCGTCGTCTGCAACGAGGCCGACGGTAGGCGAT GGGTGAGGCCATGGAGGGCAAATGTCCCCATGGTGTATCGATTACGTACCCTCTT ACTTCGTCGGGGTCGCATCGGCATTCGCCTTGCCCGCCTGT (SEQ ID NO.: 49)

(Nucleotide 1934277 - 1934128)
**>ST-E00142:243:HVLMVCCXX:8:2105:14519:48757/2**

CGGGCGACACACCGTCCCGATACATGTCAGCAACCGGGTCGATCGTGGTGAATG CACAGGCGGGCAAGGCGAATGCCGATGCGACCCCGACGAAGTAAGAGGGTACG TAATCGATACACCATGGGGACATTTGCCCTCCATGGCCTCACC (SEQ ID NO.: 50)

(Nucleotide 1934073 - 1934222)
**>ST-E00142:243:HVLMVCCXX:8:2111:10490:53276/1**

GCACGCACCCACCTGCACATCGAAATCGTGCCCGGCCTGGCCGCCAGCAGCGCG GTCCCGACCTATGCCGGGTTGCCGCTGGGTTCGTCGCACACCGTCGCCGACGTGC GTATC (SEQ ID NO.: 51)

(Nucleotide 603248 - 603361)
**>ST-E00142:243:HVLMVCCXX:8:2111:10490:53276/2**

GATACGCACGTCGGCGACGGTGTGCGACGAACCCAGCGGCAACCCGGCATAGGT CGGGACCGCGCTGCTGGCGGCCAGGCCGGGCACGATTTCGATGTGCAGGTGGGT GCGTGC (SEQ ID NO.: 52)

(Nucleotide 603361 - 603248)
**>ST-E00142:243:HVLMVCCXX:8:2111:10500:53258/1**

GCACGCACCCACCTGCACATCGAAATCGTGCCCGGCCTGGCCGCCAGCAGCGCG GTCCCGACCTATGCCGGGTTGCCGCTGGGTTCGTCGCACACCGTCGCCGACGTGC GTATC (SEQ ID NO.: 53)

(Nucleotide 603248 - 603361)
**>ST-E00142:243:HVLMVCCXX:8:2111:10500:53258/2**

GATACGCACGTCGGCGACGGTGTGCGACGAACCCAGCGGCAACCCGGCATAGGT CGGGACCGCGCTGCTGGCGGCCAGGCCGGGCACGATTTCGATGTGCAGGTGGGT GCGTGC (SEQ ID NO.: 54)

(Nucleotide 603361 - 603248)
**>ST-E00142:243:HVLMVCCXX:8:2111:19461:17342/1**

CCACGTGGCTACCCATGACCGCGCTCTCAGCGATCTTGTCTTTCTTGACGTAGTG GAAGTATTTCGGTGTGTCGCTGCCGGTCCCGTCGTCGACGCGTTCGTCGGCGTCG GTACGTTCAATCGTCTGGGTCTGCATACCTGACATTGTGC (SEQ ID NO.: 55)

(Nucleotide 3021652 - 3021801)
**>ST-E00142:243:HVLMVCCXX:8:2111:19461:17342/2**

TGCCAAGGGCACAATGTCAGGTATGCAGACCCAGACGATTGAACGTACCGACGC CGACGAACGCGTCGACGACGGGACCGGCAGCGACACACCGAAATACTTCCACTA CGTCAAGAAAGACAAGATCGCTGAGAGCGCGGGCATGGGTAG (SEQ ID NO.: 56)

(Nucleotide 3021809 - 3021660)
**>ST-E00142:243:HVLMVCCXX:8:2116:2676:63085/1**

CTTCCGATAAATAGTTAGCCGAATTATATCCTCAGGCATCAATCTCAGCTCGTCC AATCGAGCA (SEQ ID NO.: 57)

(Nucleotide 1566814 - 1566751)
**>ST-E00142:243:HVLMVCCXX:8:2116:2676:63085/2**

TGCTCGATTGGACGAGCTGAGATTGATGCCTGAGGATATAATTCGGCTAACTATT TATCGGAAG (SEQ ID NO.: 58)

(Nucleotide 1566751 - 1566814)
**>ST-E00142:243:HVLMVCCXX:8:2117:2960:14564/1**

TGATGGCGCTGGTGGAATACTCCGCCGACGAAATCAGAGAAGTGTTCTCCGACTT CCCCGATCTGGAGGTGTGTGTCTACGCCGCGCCC (SEQ ID NO.: 59)

(Nucleotide 4258616 - 4258528)
**>ST-E00142:243:HVLMVCCXX:8:2117:2960:14564/2**

GGACGCGGCGTAGACACACACCTCCAGATCGGGGAAGTCGGAGAACACTTCTCT GATTTCGTCGGCGGAGTATTCCACCAGCGCCATCA (SEQ ID NO.: 60)

(Nucleotide 4258528 - 4258616)
**>ST-E00142:243:HVLMVCCXX:8:2117:6827 :70275/1**

GGTCGTGCTGGCGGCCACGCCGTGATCGCCAGCTCGAAGACAACGCGCCACGCG TGAAGCGGCTCGATCTGGTCGCCGGGCCCAACGGCGCCG (SEQ ID NO.: 61)

(Nucleotide 444868 - 444776)
**>ST-E00142:243:HVLMVCCXX:8:2117:6827 :70275/2**

CGGCGCCGTTGGGCCCGGCGACCAGATCGAGCCGCTTCACGCGTGGCGCGTTGT CTTCGAGCTGGCGATCACGGCGTGGCCGCCAGCACGACC (SEQ ID NO.: 62)

(Nucleotide 444776 - 444868)

**>ST-E00142:243:HVLMVCCXX:8:2118: 14539:66865/1**

CAAAGGATTACCAGCGCGAGGACCTGAACCCTGAGTTCTTCGCGGCGTGTTCTCG GCATCTGCATGGACGTAGCAGACTGTGGTTGTTCCGCTACCAGGGCACGCCAATT GCCTTCTTTTTGAACGTTTGGGGTGCGGATGAGAACTACA (SEQ ID NO.: 63)

(Nucleotide 34929 - 35078)
**>ST-E00142:243:HVLMVCCXX:8:2118:14539:66865/2**

CAGTATGTAGTTCTCATCCGCACCCCAAACGTTCAAAAAGAAGGCAATTGGCGT GCCCTGGTAGCGGAACAACCACAGTCTGCTACGTCCATGCAGATGCCGAGAACA CGCCGCGAAGAACTCAGGGTTCAGGTCCTCGCGCTGGTAATC (SEQ ID NO.: 64)

(Nucleotide 35083 - 34934)
**>ST-E00142:243:HVLMVCCXX:8:2119:2209:19627/1**

CGAGGGTGACGACCCGTCGATGACCAACCCCTACATGTACGACGTCGTCGACAC CAAGCGCGGGGCCCGCAAAAGCTACACCGAAGCCCTGATCGGACGTGGCGACAT CTCGATGAAAGAGGCCGAGGACGCGCTGCGCGACTACCAGGG (SEQ ID NO.: 65)

(Nucleotide 1390797 - 1390648)
**>ST-E00142:243:HVLMVCCXX:8:2203:18254:2522/1**

TTAGCCCACCGTATCGGGCGGCGGGGACTTGCCGGAAACCGACGAACGCCTCGG CATCCATCCAAATGGCGACGAAAGATCACGGAATTGTCGCGAAACGAACG (SEQ ID NO.: 66)

(Nucleotide 4082867 - 4082764)
**>ST-E00142:243:HVLMVCCXX:8:2203:24525:40249/1**

CCTCGAGCAAAAACGTCTTCACCGGTGTTGCCCGCACCCGTAGCCGCCAACAAC GCGGCATCCAGATCGCGCTGTTGGCTGG (SEQ ID NO.: 67)

(Nucleotide 687626 - 687545)
**>ST-E00142:243:HVLMVCCXX:8:2203:24525:40249/2**

CCAGCCAACAGCGCGATCTGGATGCCGCGTTGTTGGCGGCTACGGGTGCGGGCA ACACCGGTGAAGACGTTTTTGCTCGAGG (SEQ ID NO.: 68)

(Nucleotide 687545 - 687626)
**>ST-E00142:243:HVLMVCCXX:8:2209:22871:18573/1**

CAAAGGATTACCAGCGCGAGGACCTGAACCCTGAGTTCTTCGCGGCGTGTTCTCG GCATCTGCATGGACGTAGCAGACTGTGGTTGTTCCGCTACCAGGGCACGCCAATT GCCGTCTTTTTGAACGGTTGGGGTGCGGATGAGACCTACA (SEQ ID NO.: 69)

(Nucleotide 34929 - 35078)
**>ST-E00142:243:HVLMVCCXX:8:2209:22871:18573/2**

CAGTATGTAGTTCTCATCCGCACCCCAAACGTTCAAAAAGAAGGCAATTGGCGT
GCCCTGGTAGCGGAACAACCACAGTCTGCTACGTCCATGCAGATGCCGAGAACA
CGCCGCGAAGAACTCAGGGGTCAGGTCCTCGCGCTGGTAATC (SEQ ID NO.: 70)

(Nucleotide 35083 - 34934)
**>ST-E00142:243:HVLMVCCXX:8:2210:28919:55034/2**

GGCTAGGCGCGAAAAGCGCGTGTTCGACATGTTCGGAGATCGCATGAATAAAAA
CGATGTGAGATTCCTGGATTCGCCCGGTGTCGCGTGACGGGACGTTGATCAAGA
AATCTGCGAATTCTGCCAGCTGGCCGCCGGAGTCGCCCGTCA (SEQ ID NO.: 71)

(Nucleotide 137904 - 137755)
**>ST-E00142:243:HVLMVCCXX:8:2221:11617:15637/1**

CCACTTCGGGTTCACCCTCGGCGGCAACGAGCGTGTCGAGCATCCGTTCACACTG
CGCCGCGGTGATGGCGTAGCCGTCGGGT (SEQ ID NO.: 72)

(Nucleotide 4178708 - 4178626)
**>ST-E00142:243:HVLMVCCXX:8:2221:11617:15637/2**

ACCCGACGGCTACGCCATCACCGCGGCGCAGTGTGAACGGATGCTCGACACGCT
CGTTGCCGCCGAGGGTGAACCCGAAGTGG (SEQ ID NO.: 73)

(Nucleotide 4178626 - 4178708)

The above identified target sequences in the genomic nucleic acid sequence of *Mycobacterium tuberculosis* are derived from whole genome sequencing from the plasma sample of a patient who has pulmonary tuberculosis. It is worth noting that it is generally unexpected to detect tuberculosis DNA signal from circulating plasma through whole genome sequencing. It is also to be noted that all of these target sequences fall within the above 17 CpG islands identified for *Mycobacterium tuberculosis.*

Examples of primers that can be used for the amplification of a target sequence in the CpG island of the genomic nucleic acid sequence of *Mycobacterium tuberculosis* include but are not limited to: forward primer GGCTGTGGGTAGCAGACC (SEQ ID NO.: 74) and ACCTGAAAGACGTTATCCACCAT (SEQ ID NO.: 75), reverse primer CGGGTCCAGATGGCTTGC (SEQ ID NO.: 76) and CGGCTAGTGCATTGTCATAGGA (SEQ ID NO.: 77).

The amplification of a target sequence in the CpG island of the genomic nucleic acid sequence of *Mycobacterium tuberculosis* may also comprise the use of a probe. In some examples, the probe used comprises the sequence of TGTCGACCTGGGCAGGGTTCG (SEQ ID NO.: 78) and TCCGACCGCGCTCCGACCGACG (SEQ ID NO.: 79).

In one example, the probe used comprises a component comprises at least one detectable label. In one example, the detectable label is capable of producing an optical signal. In one example, the detectable label comprises a fluorophore. Examples of fluorophores include but are not limited to fluorescent proteins, for example GFP (green fluorescent protein), YFP (yellow fluorescent protein), RFP (red fluorescent protein); non-protein fluorophores selected from the group consisting of xanthene derivatives (for example, fluorescein, rhodamine, Oregon green, eosin, 6-carboxyfluorescein and Texas red); cyanine derivatives (for example, cyanine, indocarbocyanine, oxacarbocyanine, thiacarbocyanine, and merocyanine), squaraine derivatives and ring-substituted squaraines, including Seta, SeTau, and Square dyes, naphthalene derivatives (dansyl and prodan derivatives), coumarin derivatives, oxadiazole derivatives (for example pyridyloxazole, nitrobenzoxadiazole and benzoxadiazole), anthracene derivatives (for example anthraquinones, DRAQ5, DRAQ7 and CyTRAK Orange), pyrene derivatives(for example cascade blue), oxazine derivatives (for example, Nile red, Nile blue, cresyl violet, oxazine 170), acridine derivatives (for example proflavin, acridine orange, acridine yellow), arylmethine derivatives (for example auramine, crystal violet, malachite green), tetrapyrrole derivatives (for example porphin, phthalocyanine, bilirubin) and derivatives thereof. In some specific examples, the fluorophore is selected from the group consisting of FAM (carboxyfluorescein), TET (carboxy-2',4,7,7' - tetrachlorofluorescein succinimidyl ester), HEX (carboxy-2,4,4,5,7,7 -hexachlorofluorescein succinimidyl ester), ROX (carboxy-X-rhodamine) and NED. In one specific example, the fluorophore is FAM (carboxyfluorescein), or 6-FAM (6-carboxyfluorescein).

In one example, the at least one detectable label is capable of producing a changeable signal. The changeable signal may be produced upon the hybridization of the probe to the target sequence. For example, the signal may be detectable before the probe binds to the target sequence, and upon the hybridization of the probe to the target sequence, the signal is reduced in strength or becomes completely undetectable. In another example, the detectable signal may be produced only upon the hybridization of the probe to the target sequence, or the strength of the detectable signal may be increased upon the hybridization of the probe to the target sequence.

In one example, the component comprises two detectable labels. In one example, the two detectable labels function independently, while in another example, the two detectable labels are an interactive pair of labels. The interactive pair of labels are capable of generating a changeable signal. For example, the signal may be detectable before the probe binds to the target sequence, and upon the hybridization of the probe to the target sequence, the signal is reduced in strength or becomes completely undetectable. In another example, the detectable signal may be produced only upon the hybridization of the probe to the target sequence, or the strength of the detectable signal may be increased upon the hybridization of the probe to the target sequence. In one specific example, the detectable signal is not generated when both detectable labels are linked together by the probe sequence. Once at least one detectable label is cleaved from the probe, the detectable signal is generated.

In some examples, the interactive pair of labels may comprise a fluorophore and a quencher pair. In one specific example, the fluorophore is located at the 5' end of the probe, and the quencher is located at the 3'end of the probe. Examples of quenchers include but are not limited to TAMRA (tetramethylrhodamine), TaqMan® MGB (minor groove binder) and BHQ™ (Black Hole Quencher™). In one specific example, the fluorophore is FAM (carboxyfluorescein), more particularly 6-FAM (6-carboxyfluorescein), and the quencher is TAMRA (tetramethylrhodamine).

The amplification of the target sequence in the above methods may be carried out via a polymerase chain reaction (PCR). Examples of PCRs include but are not limited to real-time polymerase chain reaction, digital polymerase chain reaction, quantitative polymerase chain reaction, qualitative polymerase chain reaction, quantitative real-time polymerase chain reaction, or quantitative reverse transcription polymerase chain reaction.

Real-time PCR monitors the amplification of a targeted DNA molecule during the PCR, i.e. in real-time, and not at its end, as in conventional PCR. Real-time PCR can be used quantitatively (Quantitative real-time PCR), and semi-quantitatively, i.e. above/below a certain amount of DNA molecules (Semi quantitative real-time PCR). Quantitative Real-Time PCR (qrt-PCR) methods use fluorescent dyes or fluorophore-containing DNA probes to measure the amount of amplified product as the amplification progresses.

Digital PCR (dPCR) simultaneously amplifies thousands of samples, each in a separate droplet within an emulsion.

Quantitative PCR (qPCR) is used to measure the specific amount of target DNA (or RNA) in a sample. By measuring amplification only within the phase of true exponential increase, the amount of measured product more accurately reflects the initial amount of target. Special thermal cyclers are used that monitor the amount of product during the amplification.

Qualitative PCR refers to a PCR method used to detect the present or absence of target DNA (RNA) in a sample without quantifying the amount present.

Reverse Transcription PCR is used to reverse-transcribe and amplify RNA to cDNA. PCR is preceded by a reaction using reverse transcriptase, an enzyme that converts RNA into cDNA. The two reactions may be combined in a tube, with the initial heating step of PCR being used to inactivate the transcriptase. RT-PCR is widely used in expression profiling, which detects the expression of a gene. It can also be used to obtain sequence of an RNA transcript, which may aid the determination of the transcription start and termination sites and facilitate mapping of the location of exons and introns in a gene sequence.

The amplified product obtained using the methods described above can be purified and the resulting purified product can be quantified using conventional nucleic acid purification methods and quantification methods. Examples of nucleic acid purification methods include but are not limited to gel electrophoresis followed by gel extraction, and silica based membrane technologies. Examples of methods to quantify the purified nucleic acid include but are not limited to spectrophotometric analysis and analysis using fluorescent dye tagging. Various kits and systems are commercially available for the purification and quantification of amplified, nucleic acid products.

The copy number of *Bam*HI-W in the amplified product can be calculated using the following formula:

$$\text{Copy Number of } BamHI - W = \frac{DNA\ Quantity\ (ng)\ x\ Avogradro's\ Number}{88390.29\ (\text{Da})\ x\ 10^{9}}$$

The methods described herein have better sensitivity and specificity compared to the currently known methods of detecting microorganisms. Specifically, for the detection of EBV, in terms of sensitivity, the lowest concentration of EBVs in a sample that can be detected using the methods described herein is 100 International Unity (IU)/ml or sample, or 90 IU/ml of sample, or 80 IU/ml of sample, or 70 IU/ml of sample, or 60 IU/ml of sample, or 50 IU/ml of sample, or 40 IU/ml of sample, or 30 IU/ml of sample, or 20 IU/ml of sample, or 10 IU/ml of sample, or 5 IU/ml of sample, or 1 IU/ml of sample.

The term "International Unit" or "IU" as used herein refers to the first WHO International Standard for Epstein-Barr Virus for Nucleic Acid Amplification Techniques defined by the National Institute for Biological Standards and Control (NIBSC) (NIBSC Code No. 09/260).

As described above, the genome of an EBV typically contains six to twenty copies of the *Bam*HI-W sequence. Therefore higher sensitivity can be achieved when the detection of EBV is based on the detection of the *Bam*HI-W region. However, the variability of *Bam*HI-W copy numbers in different EBV strains has been considered as a challenged in assay comparison and standardization between laboratories. Therefore, a new method of standardizing the number of copies of *Bam*HI-W to the amount of EBV in a sample has been developed in the present disclosure to solve this problem. The sequence of *Bam*HI-W standard was incorporated as an insert in plasmid which was propagated in competent bacteria cells, such as *E.coli.* Single colony was picked and grown for scale-up production of the plasmids. Bacteria were harvested and plasmids were extracted and quantified.

The *Bam*HI-W standard plasmid obtained carries only *Bam*HI-W sequence of EBV whereas NIBSC standard is the whole genome sequence of EBV. The presence of other genes in EBV genome might interfere with the amplification of *Bam*HI-W or generate higher background signals as compared to the *Bam*HI-W standard plasmid. In addition, the *Bam*HI-W standard plasmid obtained carries one *Bam*HI-W copy, allowing absolute quantification of *Bam*HI-W. This is not feasible in the case of NIBSC standards because number of *Bam*HI-W copies is unknown in EBV genome.

Using the constructed standard plasmid of *Bam*HI-W, it has been derived that 1 IU (International Unit) of EBV as defined by the NIBSC standard equals to about 1.38 copies of *Bam*HI-W. This conversion allows standardization between *Bam*HI-W assay of the present disclosure and other assays, allowing comparison of test results across various laboratories that use different types of assay for EBV quantification.

The method of detecting and/or quantifying a target nucleic acid sequence of the EBV can be used alone or in combination with other available methods of detecting and/or quantifying EBV.

In another aspect, there is provided a method of detecting a disease associated with microorganism infection, or risk of developing a disease associated with microorganism infection in a subject, comprising detecting and/or quantifying the presence of a nucleic acid sequence of the microorganism using the method of the present invention in a sample obtained from the subject, wherein the presence of the nucleic acid sequence of the microorganism in the sample indicates that the subject has a disease associated with microorganism infection or is at risk of developing a disease associated with microorganism infection.

In a further aspect, there is provided a method of detecting and treating a disease associated with microorganism infection, comprising: (i) detecting and/or quantifying the presence of a nucleic acid sequence of the microorganism using the method of the present invention in a sample obtained from the subject, wherein the presence of the nucleic acid sequence of the microorganism in the sample indicates that the subject has a disease associated with microorganism; (ii) administering to the subject a medicament suitable for the treatment of the disease associated with the microorganism.

In yet a further aspect, there is provided a method of predicting the treatment outcome of a disease associated with microorganism infection in a patient, comprising: (i) quantifying the nucleic acid sequence of the microorganism in a sample collected from the patient before treatment or before a treatment step, and quantifying the nucleic acid sequence of the microorganism in a sample collected from the same patient after treatment or after a treatment step; (ii) comparing the amount of the nucleic acid sequence of the microorganism in the sample before and after treatment or a treatment step, wherein a decrease in the amount of the nucleic acid sequence of the microorganism in the sample after treatment or a treatment step indicates that treatment outcome of the disease associated with microorganism infection in the patient is positive, wherein the quantifying of the nucleic acid sequence of the microorganism in the sample is performed according to the method of the present invention.

The term "disease associated with microorganism infection" as used herein refers to any disease that can be caused by a microorganism, in particular a pathogenic microorganism as described herein.

In one specific example, the disease is EBV-associated disease, in particular EBV-associated cancers. Examples of EBV-associated cancers include but are not limited to nasopharyngeal carcinoma (NPC), gastric cancer, Hodgkin's lymphoma and Burkitt's lymphoma. In one specific example, the EBV-associated cancer is NPC.

The term "sample" used herein refers to a biological sample, or a sample that comprises at least some biological materials such as nucleic acid molecules, more particularly cell free DNAs (cfDNAs). The biological samples may include liquid samples, such as whole blood, blood serum, blood plasma, buffy coat, peripheral blood mononuclear cells (PBMCs), cerebrospinal fluid, central spinal fluid, lymph fluid, cystic fluid, sputum, stool, pleural effusion, mucus, pleural fluid, ascitic fluid, amniotic fluid, peritoneal fluid, saliva, bronchial washes and urine. In specific examples, the biological sample is a blood sample or blood plasma sample. In some other specific examples, the biological sample is not a urine sample. Nucleic acids can be extracted from a biological sample using any method known to those of skill in the art.

In one specific example, the subject from which the sample is obtained is of Asian ethnicity.

The term cell free DNA (cfDNA) is used herein to refer to DNA that is found in the circulating system of a subject. cfDNAs can be microorganism cfDNAs, or cfDNAs directly released from mammalian cells, in particular abnormal cells such as cancer cells. Microorganism cfDNAs may be released from the circulating cell free microorganisms, or from microorganisms present in mammalian cells. cfDNAs directly released from mammalian cells could have been incorporated into the DNAs of the mammalian cells as a result of mammalian cells infection caused by the microorganisms.

cfDNAs which are residing within the CpG island is more stable and less susceptible to degradation and hence more likely to be detected. Therefore when a sample contains both microorganism cfDNAs and human cfDNAs, targeting microorganism cfDNAs residing within the CpG island makes the microorganism cfDNAs more likely to be detected amongst the presence of human cfDNAs.

In some examples, it is preferred to select target sequences within a nucleic acid sequence that occurs in multiple repeats in a microorganism, in order to make detection and/or quantification more feasible, especially in sample with low content of the microorganism.

The presence of the DNA sequence of a microorganism in a sample from a subject can also be used as an indication of the stage of the disease associated with the microorganism. In one specific example, the presence of an EBV DNA sequence in a sample from a subject can be used as an indication of the stage of the EBV-associated cancers.

The term "treatment" as used herein refers to any methods or substances or combination thereof, which remedy a disease state or symptoms, prevent the establishment of disease, or otherwise prevent, hinder, retard, or reverse the progression of disease or other undesirable symptoms. In particular, the disease is an EBV-associated disease, such as EBV-associated cancers, which include but are not limited to nasopharyngeal carcinoma, gastric cancer, Hodgkin's lymphoma and Burkitt's lymphoma. Types of cancer treatment generally include chemotherapy, radiation therapy, immunotherapy, and targeted therapy.

The term "decrease" or "reduce" and their grammatically variance refers to a decrease in the level of EBV in a sample collected from the patient after treatment or a treatment step as compared to the level of EBV in a sample collected from the same patient before treatment or before a treatment step. In some examples, the level of EBVs in the sample collected after treatment or a treatment step is reduced by at least 10%, at least 20%, at least 30%, at least 40%, at least 50%, at least 60%, at least 70%, at least 80%, at least 90% or at least 95% as compared to the level of EBVs in a sample collected from the same patient before treatment or before a treatment step.

The present disclosure also provides a kit for detecting and/or quantifying the presence of a target nucleic acid of a microorganism in a sample, where the kit can be used according to the methods of the present invention.

In one aspect, there is provided a kit for detecting and/or quantifying the nucleic acid sequence of a microorganism in a sample obtained from a subject, comprising a pair of oligonucleotide primers specific for the amplification of a target sequence in a CpG island of the nucleic acid of the microorganism.

In one example, the kit further comprises a probe capable of binding to the target sequence. In some examples, the probe is any probe as described above.

In yet a further example, there is provided one or more oligonucleotide primers for the amplification of a target sequence in the CpG island of the nucleic acid of a microorganism.

The sequences as described herein are from the 5' to the 3' direction, unless specified otherwise.

## EXPERIMENTAL SECTION

### Example 1 -- Comparison of sensitivity and specificity between EBV cfDNA assays

Benchmarking of the EBV cfDNA was conducted using comparison against results from a College of American Pathologists (CAP)-accredited laboratory as well as WHO-approved international EBV standards.

The clinical sensitivity and specificity of the three EBV cfDNA assays was benchmarked against an in-house EBV cfDNA assay targeting *EBNA1* in a College of American Pathologists (CAP)-accredited clinical-grade laboratory at the Singapore General Hospital (SGH), with known analytical performance reported as a sensitivity of 79% and specificity of 100%. Out of 46 NPC patients (Table 1), 31 (69%) were reported to be EBV-positive, and 14 (31%) were reported to be EBV-negative (1 case was not reported due to logistic reasons). Of 31 EBV-positive patients on the clinical-grade assay, both *Bam*HI-W qPCR and *EBNA1*-dPCR assays showed 100% matching positivity, whereas the *EBNA1*-qPCR assay showed 80% match. Of the 14 EBV-negative patients, the *Bam*HI-W qPCR, *EBNA1*-dPCR and *EBNA1*-qPCR assay reported 9, 7, and 5 positive cases. Overall, all three EBV cfDNA assays demonstrate high clinical sensitivity and specificity, with particularly high sensitivity shown at baseline for the *Bam*HI-W qPCR assay.

**Table 2. Patient characteristics**

| Characteristic | No. of patients (%) |
|---|---|
| **Total** | 46 (100.0) |
| **Gender** | |
| **Male** | 38 (82.6) |
| **Female** | 8 (17.4) |
| **Age (median,50; range,23-80)** | |
| **≤50** | 24 (52.2) |
| **>50** | 22 (47.8) |
| **T-classification** | |
| **1** | 16 (34.8) |
| **2** | 5 (10.9) |
| **3** | 19 (41.3) |
| **4** | 6 (13.0) |
| **N-classification** | |
| **0** | 8 (17.4) |
| **1** | 17 (37.0) |
| **2** | 15 (32.6) |
| **3** | 6 (13.0) |
| **M-classification** | |
| **0** | 43 (93.5) |
| **1** | 3 (6.5) |
| **AJCC 7$^{th}$ Stage** | |
| **I** | 8 (17.4) |
| **II** | 8 (17.4) |
| **III** | 18 (39.1) |
| **IV** | 12 (26.1) |
| **Treatment** | |
| **Radiotherapy alone** | 16 (34.8) |
| **Chemo- Radiotherapy** | 27 (58.7) |
| **Unknown** | 3 (6.5) |
| **Adjuvant Chemotherapy** | |
| **Yes** | 3 (6.5) |
| **No** | 43 (93.5) |

Abbreviations: AJCC, American Joint Committee on Cancer

The only available WHO-approved international EBV standard was used to benchmark the sensitivity and specificity of the three EBV cfDNA assays. The *Bam*HI-W qPCR assay demonstrated the highest reproducible sensitivity. The lowest EBV concentration detected in triplicates was 100 IU/mL for *Bam*HI-W qPCR assay and 1,000 IU/mL for both *EBNA1* assays (Table 2). The *Bam*HI-W qPCR assay was also able to detect positive signal in one replicate of the standard containing 1 IU/mL, whereas *EBNA1* assays were not able to. In addition, all assays produced no false-positive detection in five EBV-free standards, indicating their high specificity against EBV cfDNA.

**Table 3. Sensitivity and Specificity of EBV cfDNA Quantitative Assays**

| Spike-in Standards (IU/mL) | Total Number of Samples | Number of Positive Spike-in Standards | | |
|---|---|---|---|---|
| | | ***Bam*HI-W qPCR Assay** | ***EBNA1*-qPCR Assay** | ***EBNA1*-dPCR Assay** |
| **1,000,000** | 3 | 3 | 3 | 3 |
| **1,000** | 3 | 3 | 3 | 3 |
| **100** | 3 | 3 | 1 | 2 |

(continued)

| Spike-in Standards (IU/mL) | Total Number of Samples | Number of Positive Spike-in Standards | | |
|---|---|---|---|---|
| | | *Bam*HI-W qPCR Assay | *EBNA1*-qPCR Assay | *EBNA1*-dPCR Assay |
| **10** | 3 | 2 | 1 | 0 |
| **1** | 3 | 1 | 0 | 0 |
| **0** | 3 | 0 | 0 | 0 |
| **Blank** | 2 | 0 | 0 | 0 |
| Abbreviations: EBV, Epstein-Barr virus | | | | |

The IU of NIBSC standards is derived from a mean value of highly variable EBV copy number measured by various qPCR assays of 28 laboratories in the world. These assays employ different DNA extraction methods, and target a wide range of genes, including a single-copy gene, *EBNA1,* and a multiple-repeat gene, *Bam*HI-W. However, since dPCR was not included in the evaluation, the relationship between EBV copy number as obtained by dPCR and IU is less clear. Moreover, since the number of *Bam*HI-W fragments varies in different EBV isolates, a fixed conversion ratio of *Bam*HI-W copies to IU will not be always accurate in different patients' sample. Therefore, the NIBSC standards were only used in this study for comparison of sensitivity and specificity between EBV cfDNA assays. The subsequent data were to be reported in copy number of respective EBV targets.

**Example 2 -- Relationship between NPC circulating biomarkers in pre-treatment samples**

Among EBV cfDNA quantitation approaches, *Bam*HI-W qPCR assay yielded the highest concentration of EBV cfDNA levels: 2.4 to 37.7-fold higher than *EBNA1*-qPCR assay and 2.2 to 25.5-fold higher than *EBNA1*-dPCR assay (Table 3). All samples detected EBV-positive by both *EBNA1* assays were also detected positive for EBV by *Bam*HI-W assay. The detection rates of canonical CTCs and potential CTCs are 76% and 94% in pre-treatment samples respectively. Overall, potential CTC count was higher and weakly correlated to canonical CTC count ($r^2$ = 0.21, *P*-value = < 0.01). No correlation was observed between each type of CTC count and EBV cfDNA levels quantified by different assays. In contrast, among the EBV cfDNA assays, strong correlation was observed between *Bam*HI-W qPCR and *EBNA1*-dPCR assays ($r^2$ = 0.99, *P*-value < 0.0001), but not between *Bam*HI-W and *EBNA1*-qPCR assays ($r^2$ = 0.03, *P*-value = 0.29) nor between *EBNA1*-qPCR and -dPCR assays ($r^2$ = 0.06, *P*-value = 0.11). This result corresponded with the similar detection rate of *Bam*HI-W qPCR (89%) and *EBNA1*-dPCR (85%) assays, with the detection rate of *EBNA1*-qPCR assay being 67%.

**Table 4. Quantitative levels of NPC circulating biomarkers in 46 pre-treatment samples**

| Patient ID | AJCC 7th Stage | Status on Follow-up | Pre-Treatment | | | | |
|---|---|---|---|---|---|---|---|
| | | | *Bam*HI-W qPCR Assay (copies/mL) | *EBNA1*-qPCR Assay (copies/mL) | *EBNA1*-dPCR Assay (copies/mL) | Canonical CTCs (cells/mL) | Potential CTCs (cells/mL) |
| **001** | III | NED | 70,569 | 5,416 | 9,484 | 0 | 3 |
| **002** | III | NED | 9,728 | 385 | 1,109 | 0 | 12 |
| **003** | III | NED | 10,631 | 855 | 1,376 | NA | NA |
| **004** | III | NED | 507 | 30 | 43 | 0 | 0 |
| **005** | IV | NED | 1,324 | 80 | 168 | 5 | 13 |
| **006** | I | NED | 0 | 0 | 0 | 4 | 13 |
| **007** | I | NED | 21 | 0 | 9 | 0 | 20 |
| **008** | I | NED | 107 | 0 | 0 | 0 | 14 |
| **009** | III | DOD | 18,572 | 5,425 | 3,636 | NA | NA |
| **010** | IV | AWD | 1,249 | 36 | 132 | 3 | 4 |
| **011** | II | NED | 23,507 | 2,838 | 3,656 | 2 | 1 |
| **012** | IV | DOD | 99,379 | 18,816 | 14,199 | 6 | 146 |
| **013** | II | NED | 301 | 0 | 28 | 0 | 0 |
| **014** | I | NED | 0 | 0 | 0 | 1 | 12 |

(continued)

| Patient ID | AJCC 7th Stage | Status on Follow-up | Pre-Treatment | | | | |
|---|---|---|---|---|---|---|---|
| | | | *Bam*HI-W qPCR Assay (copies/mL) | *EBNA1*-qPCR Assay (copies/mL) | *EBNA1*-dPCR Assay (copies/mL) | Canonical CTCs (cells/mL) | Potential CTCs (cells/mL) |
| **017** | II | NED | 67 | 0 | 21 | 50 | 134 |
| **018** | IV | NED | 441,316 | 13,565 | 50,081 | 11 | 29 |
| **019** | I | NED | 162 | 0 | 13 | 18 | 44 |
| **020** | III | NED | 4,860 | 0[a] | 804 | 16 | 17 |
| **021** | III | NED | 1,236 | 33 | 49 | 37 | 76 |
| **022** | III | NED | 44,918 | 1,964 | 3,949 | NA | NA |
| **023** | III | NED | 29,006 | 777 | 3,272 | NA | NA |
| **024** | I | NED | 0 | 49 | 0 | 31 | 63 |
| **025** | IV | NA | 290,961 | 16,727 | 53,740 | 5 | 68 |
| **026** | IV | NED | 1,157 | 121 | 230 | 16 | 83 |
| **027** | III | NED | 6,687 | 431 | 1,356 | 4 | 21 |
| **028** | I | NED | 360 | 0 | 53 | 1 | 7 |
| **029** | III | NED | 6,072 | 303 | 816 | 17 | 47 |
| **030** | III | NED | 8,226 | 714 | 1,095 | 0 | 15 |
| **031** | IV | NED | 9,507 | 442 | 670 | 4 | 19 |
| **032** | II | NED | 92 | 0 | 42 | 3 | 67 |
| **033** | IV | DOD | 1,743,700 | 0[a] | 193,125 | 6 | 44 |
| **034** | III | NED | 9,043 | 447 | 1,279 | 1 | 63 |
| **035** | II | NED | 146 | 21 | 0 | 1 | 14 |
| **036** | III | NED | 105 | 0 | 0 | 0 | 182 |
| **037** | II | NED | 669 | 0 | 63 | 3 | 13 |
| **038** | IV | AWD | 81 | 26 | 105 | 3 | 31 |
| **039** | III | DOD | 6,613 | 439 | 780 | 1 | 15 |
| **040** | III | NED | 5,106 | 623 | 1,125 | 1 | 14 |
| **041** | II | NED | 331 | 84 | 46 | NA | NA |
| **042** | III | NED | 2,156 | 171 | 241 | NA | NA |
| **043** | III | NED | 56,490 | 8,829 | 9,894 | NA | NA |
| **044** | IV | DOD | 88,432 | 12,074 | 16,850 | NA | NA |
| **045** | IV | NED | 33,057 | 6,014 | 5,319 | NA | NA |
| **046** | I | NED | 131 | 55 | 7 | NA | NA |
| **047** | IV | NED | 0 | 0 | 0 | NA | NA |
| **048** | II | NED | 0 | 0 | 7 | NA | NA |

Abbreviations: NPC, nasopharyngeal carcinoma; AJCC, American Joint Committee on Cancer; CTCs, circulating tumour cells;; NED, no evidence of disease; AWD, alive with disease; DOD, dead of disease; NA, data are not available
[a] PCR inhibition

**Example 3-- Relationship between NPC circulating biomarkers and clinical stage**

The clinical stages were re-classified to three groups; stage I, stage II-III, and stage IV (Table 4). The combination of stage-II and -III NPC patients was in the light of long-term 5-year follow-up data from Singapore showing similar survival outcomes using modern treatment approaches[13]. The EBV cfDNA levels in three assays strongly correlated with clinical stages. In contrast, there was no statistically significant relationship between CTCs and clinical stages. These results indicated a strong association between NPC clinical stage and EBV cfDNA, but not CTCs.

**Table 5. Relationship between NPC circulating biomarkers and clinical stages in pre-treatment samples**

| NPC circulating biomarkers | Mean Values | | | LR Chi-Square Values[a] | Degree of Freedom | *P*-Values[a] |
|---|---|---|---|---|---|---|
| | Stage I | Stage II-III | Stage IV | | | |
| ***BamHI-W*** **qPCR Assay (copies/mL)** | 98 | 12,140 | 225,847 | 14.15 | 1 | 0.0002[b] |
| ***EBNA1*****-qPCR Assay (copies/mL)** | 13 | 1,146 | 5,658 | 10.84 | 1 | 0.0010[b] |
| ***EBNA1*****-dPCR Assay (copies/mL)** | 10 | 1,699 | 27,885 | 14.52 | 1 | 0.0001[b] |
| **Canonical CTC Enumeration (cells/mL)** | 8 | 8 | 7 | 0.05 | 1 | 0.8250 |
| **Potential CTC Enumeration (cells/mL)** | 25 | 39 | 49 | 1.07 | 1 | 0.3000 |

Abbreviations: NPC, nasopharyngeal carcinoma; CTCs, circulating tumour cells
[a] Likelihood ratio Chi-square and *P*-values were determined using logistic ordinal regression for the prediction of NPC clinical stage, given the levels of NPC circulation biomarkers
[b] *P*-values <0.05 were considered statistically significant.

**Example 4 - Relationship between NPC circulating biomarkers and treatment outcome**

Decreased EBV cfDNA levels were observed in all EBV-positive patients following treatment, strongly correlating with the local radiological response (Table 5). To evaluate the predictive value of NPC circulating biomarkers for short-term radiological response, we determined that EBV cfDNA levels were significantly reduced after treatment (Wilcoxon's signed rank testing p-value<0.001 for all three techniques *Bam*HI-W qPCR, *EBNA1*-dPCR and *EBNA1*-qPCR assay). In contrast, for both canonical and potential CTCs, decrease was not significant (p=0.07 and 0.54 respectively). The stratified analysis performed on patients undergoing radiotherapy and chemo-radiotherapy showed the magnitude of decrease of canonical CTCs pre- and post-treatment in each group remains insignificant (Table 6). Overall, our results show that EBV cfDNA level correlation with short-term radiological response was much stronger than that of potential or canonical CTC counts.

**Table 6. Quantitative levels of NPC circulating biomarkers in 28 matched samples**

| Patient ID | AJCC 7th Stage | Post-Treatment Radiological Response | Status on Follow-up | *Bam*HI-W qPCR Assay (copies/mL) | | *EBNA1*-qPCR Assay (copies/mL) | | *EBNA1*-dPCR Assay (copies/mL) | | Canonical CTCs (cells/mL) | | Potential CTCs (cells/mL) | |
|---|---|---|---|---|---|---|---|---|---|---|---|---|---|
| | | | | Pre-Treatment | Post-Treatment | Pre-Treatment | Post-Treatment | Pre-Treatment | Post-Treatment | Pre-Treatment | Post-Treatment | Pre-Treatment | Post-Treatment |
| **006** | I | CR | NED | 0 | 0 | 0 | 0 | 0 | 0 | 4 | 3 | 13 | 88 |
| **014** | I | CR | NED | 0 | 0 | 0 | 0 | 0 | 0 | 1 | 5 | 12 | 5 |
| **024** | I | PR | NED | 0 | 0 | 49 | 0 | 0 | 0 | 31 | 1 | 63 | 3 |
| **007** | I | CR | NED | 21 | 0 | 0 | 0 | 9 | 0 | 0 | 0 | 20 | 103 |
| **008** | I | CR | NED | 107 | 0 | 0 | 0 | 0 | 0 | 0 | 3 | 14 | 14 |
| **019** | I | nCR | NED | 162 | 42 | 0 | 0 | 13 | 0 | 18 | 22 | 44 | 148 |
| **028** | I | CR | NED | 360 | 0 | 0 | 0 | 53 | 14 | 1 | 0 | 7 | 13 |
| **017** | II | PR | NED | 67 | 0 | 0 | 0 | 21 | 7 | 50 | 16 | 134 | 220 |
| **032** | II | PR | NED | 92 | 0 | 0 | 0 | 42 | 7 | 3 | 0 | 67 | 15 |
| **035** | II | nCR | NED | 146 | 0 | 21 | 0 | 0 | 0 | 1 | 0 | 14 | 23 |
| **013**[b] | II | CR | NED | 301 | 0 | 0 | 0 | 28 | 0 | 0 | 3 | 0 | 26 |
| **011**[b] | II | CR | NED | 23,507 | 27 | 2,838 | 0 | 3,656 | 0 | 2 | 6 | 1 | 9 |
| **004**[b] | III | CR | NED | 507 | 0 | 30 | 0 | 43 | 0 | 0 | 4 | 0 | 26 |
| **021**[b] | III | nCR | NED | 1,236 | 0 | 33 | 0 | 49 | 0 | 37 | 2 | 76 | 33 |
| **029**[b] | III | nCR | NED | 6,072 | 0 | 303 | 0 | 816 | 0 | 17 | 0 | 47 | 6 |
| **027**[b] | III | CR | NED | 6,687 | 0 | 431 | 0 | 1,356 | 0 | 4 | 1 | 21 | 18 |
| **030**[b] | III | nCR | NED | 8,226 | 0 | 714 | 0 | 1,095 | 0 | 0 | 0 | 15 | 0 |
| **002**[b] | III | nCR | NED | 9,728 | 0 | 385 | 0 | 1,109 | 7 | 0 | 0 | 12 | 150 |
| **003**[b] | III | CR | NED | 10,631 | 0 | 855 | 0 | 1,376 | 0 | NA | NA | NA | NA |
| **009** | III | PD | DOD | 18,572 | 131 | 5,425 | 0 | 3,636 | 35 | NA | NA | NA | NA |
| **001**[b] | III | CR | NED | 70,569 | 0 | 5,416 | 0 | 9,484 | 0 | NA | NA | NA | NA |
| **023**[b] | III | CR | NED | 29,006 | 47 | 777 | 0 | 3,272 | 6 | NA | NA | NA | NA |
| **022**[b] | III | nCR | NED | 44,918 | 0 | 1,964 | 0 | 3,949 | 13 | NA | NA | NA | NA |
| **026**[b] | IV | PR | NED | 1,157 | 0 | 121 | 0 | 230 | 7 | 16 | 2 | 83 | 6 |
| **010**[b] | IV | PR | AWD | 1,249 | 0 | 36 | 0 | 132 | 0 | 3 | 0 | 4 | 3 |
| **005**[b] | IV | nCR | NED | 1,324 | 0 | 80 | 0 | 168 | 0 | 5 | 1 | 13 | 27 |
| **012**[b] | IV | PR | DOD | 99,379 | 24,577 | 18,816 | 2,529 | 14,199 | 5,107 | 6 | 1 | 146 | 35 |
| **018**[b] | IV | PR | NED | 441,316 | 0 | 13,565 | 0 | 50,081 | 0 | 11 | 6 | 29 | 61 |
| **Mean** | | | | 27,690 | 887 | 1,852 | 90 | 3,386 | 186 | 9 | 3 | 36 | 45 |
| ***P*-Values**[a] | | | | <0.001 | | <0.001 | | <0.001 | | 0.07 | | 0.54 | |

Abbreviations: NPC, nasopharyngeal carcinoma; AJCC, American Joint Committee on Cancer; CTCs, circulating tumour cells; nCR, near complete response; CR, complete response; PR, partial response; PD, progressive disease; NED, no evidence of disease; AWD, alive with disease; DOD, dead of disease; NA, data are not available

[a] *P*-Values were calculated using the Wilcoxon's signed rank testing and values <0.05 were considered statistically significant.

**Table 7. Stratified analysis of CTC enumeration in NPC pre- and post-treatment samples**

| | Radiotherapy (n=10) | | | | Chemo-Radiotherapy (n=13) | | | | All Treatment (n=23) | | | |
|---|---|---|---|---|---|---|---|---|---|---|---|---|
| | Canonical CTCs | | Potential CTCs | | Canonical CTCs | | Potential CTCs | | Canonical CTCs | | Potential CTCs | |
| | Pre | Post | Pre | Post | Pre | Post | Pre | Post | Pre | Post | Pre | Post |
| **Mean** | 11 | 5 | 38 | 63 | 8 | 2 | 34 | 31 | 9 | 3 | 36 | 45 |
| **P-value** | 0.58 | | 0.19 | | 0.07 | | 0.78 | | 0.07 | | 0.54 | |

**Example 5 - Relationship between NPC circulating biomarkers and overall survival**

Survival analysis demonstrated that there was a stronger correlation between EBV cfDNA and overall survival, as compared to that between CTC counts and overall survival. All three EBV cfDNA techniques showed prognostic value on survival analysis: *BamHI-W* qPCR, *EBNA1*-dPCR and *EBNA1*-qPCR assays yielded corresponding p-values of 0.03, 0.02 and 0.0002 by log-rank testing respectively, whereas canonical CTC and potential CTC counts were not associated with overall survival (p=0.66 and 0.13 respectively). Kaplan-Meier plots are also shown for dichotomized biomarker variables (Fig. 1).

**Example 6 - Derivation of Internal Control for the amplification of EBV**

**Table 8. Derivation of Internal Control (IC) for the amplification of EBV**

| **Master Mix** | **1** | | **2** | | **3** | | **4** | | **5** | |
|---|---|---|---|---|---|---|---|---|---|---|
| **EBV Primers/Probe** | + | | + | | + | | + | | - | |
| **IC Primers/Probe** | + | | + | | - | | - | | + | |
| **IC Oligo** | + | | - | | - | | + | | + | |
| **Mean Ct*** | **EBV¹** | **IC²** | **EBV¹** | **IC²** | **EBV¹** | **IC²** | **EBV¹** | **IC²** | **EBV¹** | **IC²** |
| **C666-1** | 24.61 | 22.21 | 24.81 | U | 24.63 | U | 25.29 | U | U | 22.39 |
| **RKO** | U | 22.13 | U | U | U | U | U | U | U | 22.43 |
| **Healthy Donor#** | U | 22.16 | U | U | U | U | U | U | U | 22.43 |
| **NTC** | U | 22.13 | U | U | U | U | U | U | U | 22.39 |

* Triplicate
#Buffy coat DNA
1FAM signal
2VIC signal
U: Undetermined signal
EBV: Epstein-Barr Virus
IC: Internal Control

The nucleic acid sequence of the Internal Control (IC) selected is TTAGCAGCGACGAAGATCATGCGCT-CACGCTCTCGGTGTCCTCATTCATCAGTTAT TCACAACGCTATGCTGTAACTCGACCTGACAAGACTGTACCTAT-GAGAAGGCACTT GCTACCTTATGCAAGCGTCAGCCCGCGGTATCGCTTGG (SEQ ID NO.: 80). The sequences of the primers and probes used to amplify the IC are: forward Primer: 5'-CGCTCTCGGTGTCCTCATTC-3' (SEQ ID NO.: 81), Reverse Primer: 5'-GGCTGACGCTTGCATAAGGT-3' (SEQ ID NO.: 82), and Probe: 5'-VIC-CACAACGCTATGCTGTAACTCGACCTGAC-TAMRA-3' (SEQ ID NO.: 83).

Concentration of IC primers and probe is 400nM and 100nM respectively (same as *Bam*HI-W primers and probe). As shown in Table 8, Master mix 1 contains IC and all the components of the duplex assay. Therefore, both FAM and VIC signals should be seen in C666-1, an EBV-positive cell line. RKO, buffy coat of healthy donor and no-template control are EBV-negative, thus, will only emit VIC signal. Master mix 2, 3, 4 contain EBV primers and probe without IC Primers and probes or IC sequence or both. So VIC signal should not be present in all samples. This is to test if any component of the IC assay interfere the EBV assay. The last master mix only contains the IC sequence, primers and probe. VIC signal should be positive whereas FAM signal should be negative for all samples. And the results are as expected. The mean Ct values of both FAM and VIC signals in different setups are about the same. The duplex assay is then applied on a serial dilution of EBV sample with or without IC. As shown in Fig. 4, the two straight lines are almost overlapping, indicating no interference between EBV and IC assays.

The duplex assay is tested in clinical samples, including 5 EBV-positive NPC samples, and 5 healthy donor's samples. As illustrated in Fig. 5 and shown in Table 9, in setup 1, no IC is spiked, thus, VIC signal should be negative in all samples. In setup 2 and 3, IC is spiked to extracted DNA or samples respectively, so VIC signal will be seen in both setups. And once again, the results are as expected. The small SD of mean Ct in each NPC sample indicates consistent measurement of EBV regardless whether IC is spiked or which step it is spiked. If can be noticed that the mean Ct of IC in setup 3 is smaller than the one in setup 2. This is because the quantity of IC spiked in setup 3 was 10 times higher than the one in setup 2. This was done to accommodate the possible DNA loss during extraction process, however, as shown in Table 9, most of the IC was recovered very well.

**Table 9. Impact of IC assay on EBV quantification**

| Setup | Mean Ct* | NPC Pre-Treatment Samples | | | | | Healthy Donor Samples | | | | |
|---|---|---|---|---|---|---|---|---|---|---|---|
| | | 1 | 2 | 3 | 4 | 5 | 6 | 7 | 8 | 9 | 10 |
| 1 | EBV[1] | 30.30 | 25.47 | 27.83 | 27.18 | 28.59 | U | U | U | U | U |
| | IC[2] | U | U | U | U | U | U | U | U | U | U |
| 2 | EBV[1] | 30.65 | 25.81 | 28.02 | 27.41 | 28.55 | U | U | U | U | U |
| | IC[2] | 20.72 | 20.76 | 20.73 | 20.81 | 20.80 | 20.80 | 20.80 | 20.88 | 20.9 | 20.86 |
| 3 | EBV[1] | 30.98 | 26.35 | 28.29 | 28.19 | 29.08 | U | U | U | U | U |
| | IC[2] | 17.73 | 18.15 | 17.12 | 18.24 | 17.39 | 18.50 | 18.57 | 17.90 | 18.04 | 18.35 |

*Triplicate
[1] FAM signal
[2] VIC signal
U: Undetermined signal
EBV: Epstein-Barr Virus
IC: Internal Control

The IC plasmids were spiked to plasma samples and underwent DNA extraction whereas EBV standard plasmids were added directly to the PCR reactions. As shown in Fig. 6, successful amplification of both EBV standard and IC indicates plasmids can be amplified as whole or fragments. As shown in Table 10, in 6 clinical samples, IC were detected, confirming true negative detection of EBV in sample 2 and 6. Sample 6 was indeed negative control, from healthy donor whereas sample 5 was positive control, from an NPC sample with known EBV positive result in plasma. Samples 1 to 4 were also derived from NPC patients but EBV levels in plasma were unknown.

| Samples | 1 | 2 | 3 | 4 | 5 | 6 |
|---|---|---|---|---|---|---|
| Mean EBV Standard Plasmid Copies* | 84 | 0 | 325 | 3 | 239 | 0 |
| Standard Deviation | 13 | N.A. | 26 | 1 | 39 | N.A. |
| IC Signal | + | + | + | + | + | + |

*Triplicate

**Table 10. Plasmid validation in clinical samples**

**Example 7 - Derivation of a new standard for quantifying EBV based on the number of copies of *BamHI-W***

Despite being a powerful tool in NPC prognosis, the quantification of EBV cfDNA faces challenges of standardization. The NIBSC standards, which are derived from whole EBV produced by B95-8 cells provide a consensus estimate of EBV IU, but are not ideal for standardization of *Bam*HI-W copy number. In addition, the NIBSC spike-in standards do not truly represent the NPC plasma samples. Naturally occurring cfDNA has a size of less than 181 bp in NPC plasma whereas DNA obtained from NIBSC was genomic DNA with a size of 170 kb. The differences in DNA size influence the choice of DNA extraction kit, which in turn has meaningful impact on DNA recovery, and subsequently DNA quantification.

To solve these problems, a new standard for the quantification of EBV cfDNA is developed in the present study.

The sequence of *Bam*HI-W standard was incorporated as an insert in plasmid which was propagated in competent bacteria cells (such as *E.coli).* Single colony was picked and further grown in Lysogeny *Broth (LB) for scale-up production of the plasmids. Bacteria were harvested and plasmids were extracted using the* QIAprep Spin Miniprep Kit (Qiagen) and quantified using Quantus Fluorometer (Promega). Sequence of *Bam*HI-W in the newly produced plasmids was confirmed by Sanger Sequencing.

*Bam*HI-W standard plasmids were prepared with known copy number (preferably 10-time serial dilution) then added directly to the PCR well. Standard curve was plotted based on the Ct values obtained from the *Bam*HI-W standard plasmids, which were used to calculate sample's *Bam*HI-W copy number. In the conversion factor experiment, NIBSC standards were set at standard curve to which *Bam*HI-W standard plasmids were calibrated.

### Example 8 - Detection of target nucleic acid sequence of *Mycobacterium tuberculosis*

Two targets sequences were tested for the amplification of nucleic acid sequence of Mycobacterium tuberculosis in samples collected from a patient known to have tuberculosis and a healthy subject as control. The target sequences are: (1) nucleotide sequences from nucleotide position 1542511 to nucleotide position 1542349; and (2) nucleotide sequences from nucleotide position 1542328 to nucleotide position 1542215 of the genomic sequence of *Mycobacteriaum tuberculosis.* The primers and probes used for the amplification of target sequence (1) are: forward primer 5'-GGCTGTGGGTAGCAGACC-3' (SEQ ID NO.: 74), reverse primer 5'-CGGGTCCAGATGGCTTGC-3' (SEQ ID NO.: 76) and probe 5'-FAM-TGTCGACCTGGGCAGGGTTCG-TAMRA-3' (SEQ ID NO.: 84),. The primers and probes used for the amplification of target sequence (2) are: forward primer 5'-ACCTGAAAGACGTTATCCACCAT-3'(SEQ ID NO.: 75), reverse primer 5'-CGGCTAGTGCATTGTCATAGGA- 3' (SEQ ID NO.: 77), and probe 5'-FAM-TCCGACCGCGCTCCGACCGACG-TAMRA-3' (SEQ ID NO.: 85). From the PCR amplification results shown in Fig. 11, it is concluded that both target sequences of *Mycobacterium tuberculosis* are present in the sample from the patient with tuberculocis, but absent in the sample from the healthy subject control.

### Discussion

Non-invasive approaches of NPC diagnosis have been available for the past decade via the detection of immunoglobulin A antibody against EBV antigens in patients' serum. However, these techniques are inefficient in NPC prognosis and relapse prediction. There is considerable ongoing research into EBV cfDNA in NPC patients for prediction of post-treatment outcomes, and its role in selecting patients for additional adjuvant treatment following definitive therapy.

In the present study, good correlation between EBV cfDNA and clinicopathologic outcomes was consistently demonstrated regardless of approach undertaken: *Bam*HI-W qPCR, *EBNA1*-qPCR or *EBNA1*-dPCR assays. Decreased EBV cfDNA levels are commonly observed in almost all patients undergoing treatment, corresponding generally to the short-term post-treatment radiological response, which is commonly a complete or near-complete response. Overall, the results demonstrated that EBV cfDNA yielded better results in comparison with circulating tumor cell (CTC) count as a circulating biomarker for NPC. Regardless of approach, cfDNA showed far stronger correlation with tumor stage, short-term radiological response as well as overall survival, in comparison with CTC counts.

The detection rate of the *Bam*HI-W qPCR assay in the present study was 89%. In comparison with clinically validated assays, the *Bam*HI-W qPCR assay demonstrated better performance. The detection rate of the CE-IVD *EBNA1*-qPCR assay reported in this study was 67%, despite its claimed clinical sensitivity of 100%, based on 80 EBV-positive samples. Moreover, EBV positive cases reported by the *Bam*HI-W qPCR assay were matched with the ones reported by the SGH assay, which had clinical sensitivity of 79%.

In the present study, by targeting the multiple-repeat *Bam*HI-W fragments, the *Bam*HI-W qPCR assay yielded the highest detection rate in NPC pre-treatment samples. It also yielded the highest sensitivity in measurement of NIBSC spike-in standards despite the possible DNA losses due to the DNA extraction method potentially not optimized to genomic DNA. On the other hand, regardless of being different in fundamental techniques of quantification and EBV targets, *Bam*HI-W qPCR and *EBNA1*-dPCR assays were strongly correlated in the measurement of EBV levels in pre-treatment samples. This correlation could possibly be aided by the same extraction process from which the cfDNA used in *Bam*HI-W qPCR and *EBNA1*-dPCR assays was extracted. Altogether, in our interpretation, the *Bam*HI-W qPCR and *EBNA1*-dPCR assays are more likely to quantify the true values of EBV cfDNA level in pre-treatment samples of NPC patients.

The evidence of EBV cfDNA existing in the form of short and freely-floating fragments in the plasma had led to a conclusion that they were released from apoptotic NPC cells. In other words, the NPC cells releasing EBV cfDNA lysed before they had the chance to enter the bloodstream. This phenomenon could explain the non-correlation between NPC CTC counts and EBV cfDNA levels measured by various assays.

The results of the present study demonstrated that by targeting the multiple-repeat *BamHI-W,* higher detection rate and sensitivity were achieved.

Further, plasma sample of NPC patients contains both human cfDNA and EBV cfDNA. The two major challenges in detection of EBV cfDNA, and in general, microorganism cfDNA in clinical samples are the degradation of EBV cfDNA and the abundant presence of human genomic DNA which hinders the signals from EBV cfDNA. In order to overcome these challenges, a target region (*Bam*HI-W region) in EBV that is preserved and present in high copy number was selected, in particular the region within the CpG island and near to the 5' end of CpG island was selected for the following reasons: 1. *Bam*HI-W region occurs in multiple repeats per EBV genome, making detection and quantification more feasible, especially in sample with low EBV copy number and/or limited input volume; 2. The region is near to the 5' location which would allow for preferential preservation during exonuclease III degradation of the EBV dsDNA; and 3. cfDNA residing within CpG islands is more stable and thus less susceptible to degradation and more likely to be detected amongst the presence of human cfDNA.

In the other example, *Mycobacterium tuberculosis,* plasma sample was selected from a tuberculosis patient because there were bacterium and human cfDNA present in the plasma and the genome of *Mycobacterium* is rich in GC content. Our analysis showed CpG islands cover 99.9% of the *Mycobacterium tuberculosis* genome. cfDNA was extracted from the tuberculosis plasma sample and undergone whole genome sequencing (WGS). All reads were mapped to *Mycobacterium tuberculosis* (Reference genome M.TB H37Rv) and aligned to the CpG islands data mentioned earlier. The results showed all reads belong to CpG islands on the *Mycobacterium tuberculosis* genome. In order words, highly fragmented and rare *Mycobacterium tuberculosis* cfDNA within CpG islands can be sequenced despite the abundant presence of human cfDNA in plasma sample. These results imply the advantage of designing cfDNA assay targeting CpG islands in microorganisms.

**Materials and Methods**

**Clinical samples**

The study was approved by the Centralised Institutional Review Board, SingHealth (Reference number: 2013/354/B) and all methods were carried out *in accordance with* the approved guidelines. A total of 46 NPC patients, all of Asian ethnicity, who provided informed written consent, were recruited into the study between June 2013 and October 2014 (Table 1). 20 mL of blood was collected in EDTA tube (BD Biosciences) at baseline and one month after treatment. All stage-I and most of stage-II patients received only radiotherapy whereas most patients from stage III and IV received combined chemo-radiotherapy. Only 3 patients received adjuvant chemotherapy. A total of 28 matched serial samples, pre- and post-treatment, were collected. The post-treatment radiological response of all patients was based on their first magnetic resonance imaging / computed tomography scan after treatment (Table 5). The median follow-up was 18.7 months.

**Participating laboratories and clinic**

Institute of Bioengineering and Nanotechnology (IBN) served as the centralised laboratory of the study. Blood samples were collected from consenting NPC patients at National Cancer Centre Singapore, and sent to IBN within the same day of their visits within 4 hours. For each sample, whole blood was used for immediate CTC enumeration, and plasma was obtained, assigned blinded IDs and stored at -80°C until further use. Each plasma assay had its individually optimized volumes. 250 µL of frozen plasma was distributed to Singapore General Hospital (SGH) where cfDNA extraction and quantification was performed using the *Sentosa*® SA EBV Quantitative PCR Test (Vela Diagnostics) following manufacturer's requirements. At IBN, 1 mL of thawed plasma was used for cfDNA extraction of which half was quantified by the in-house *Bam*HI-W assay. The other half of the extracted cfDNA was sent to JN Medsys where cfDNA quantification was conducted using the Clarity™ Digital PCR System (JN Medsys).

***Bam*HI-W qPCR assay**

50 µL of cfDNA was extracted from 1 mL of thawed plasma using the QIAamp Circulating Nucleic Acid Kit (Qiagen). The *Bam*HI-W7 primers (Sigma Aldrich) and dual-labelled *Bam*HI-W7 hydrolysis probe (Life Technologies) were designed for the amplification of a 143-bp region of *Bam*HI-W. Each 20-µL reaction consisted of 1x Taqman® Fast Advanced Master Mix (Life Technologies), 400 nM *Bam*HI-W7 primers (sense 5'-*AGATCTAAGGCCGGGAGAGG-3'* (SEQ ID NO.: 2) and antisense 5'-*CGCCCATTCGCCTCTAAAGT*-3') (SEQ ID NO.: 3), 100 nM *Bam*HI-W7 probe (5'-(6-FAM)*CTCTGGTAGTGATTTGGACCCGAAATCTG*(TAMRA)-3') (SEQ ID NO.:4) and 2 µL of DNA template, which was equivalent to 40 µl of plasma. Standard calibrators for *Bam*HI-W were generated with 8 dilutions of DNA derived from EBV-immortalised cell lines ranging from 1 to $10^7$ *Bam*HI-W copies per reaction. qPCR was performed using the ViiA™7 Real-time PCR System (Life Technologies). Each run included patients' cfDNA, standard calibrators, EBV-positive, -negative and no-template controls (NTCs). The reactions were run at 50°C for 2 min, followed by 95°C for 20 sec to activate Uracil N-Glycosylase (UNG) and AmpliTaq® Fast DNA Polymerase, respectively. Subsequently, the reactions underwent 40 two-step cycles of denaturation and annealing at 95°C for 1 sec, and 60°C for 20 sec, respectively. The *Bam*HI-W copy number was automatically calculated from ViiA™7 software based on the *Bam*HI-W standard calibrator of each run, with $R^2$ = 0.99, qPCR efficiency = 98-100%, m = (-3.315) - (-3.368). Initial optimization of the *Bam*HI-W assay was conducted by conventional PCR using EBV-positive C666-1 DNA (see Fig. 12). *Bam*HI-W specificity for healthy controls has been previously determined to be high and testing of 30 healthy donors also showed no signal.

**_EBNA1_-qPCR assay**

The *Sentosa*® SA EBV Quantitative PCR Test (Vela Diagnostics) was applied for quantification of EBV cfDNA with the aid of the integrated *Sentosa*® SX101 (Vela Diagnostics) and Rotor-Gene® Q MDx 5-plex HRM (Qiagen) instruments. 60 µL of DNA was automatically extracted from 200 µL of plasma using the *Sentosa*® SX Virus Total Nucleic Acid Kit v2.0 (Vela Diagnostics). 10 µL of purified DNA, equivalent to 33 µL of plasma was used for each reaction. The PCR master mix contained reagents and enzymes for the amplification of a 79-bp fragment of *EBNA1,* as well as a second set of primers/probes designed to detect EC3, a control for PCR inhibition and cfDNA extraction. The concentration of *EBNA1* was automatically calculated based on the imported standard curve, with $R^2$ = 0.99, qPCR efficiency = 98%, m = (-3.367). The clinical sensitivity and specificity of the assay was reported as 100% and 98.8% respectively.

**_EBNA1_-dPCR assay**

The Clarity™ Digital PCR System (JN Medsys) was used. The assay was designed to amplify a 118-bp fragment of *EBNA1.* Each 15-µL reaction consisted of IX FastStart Essential DNA Probes Master (Roche), 200 nM *EBNA1* primers (sense 5'-*TCATCATCATCCGGGTCTCC-3'* (SEQ ID NO.: 86) and antisense 5'-*GCTCACCATCTGGGCCAC-3')* (SEQ ID NO.: 87), 200 nM probe (5'-(6-FAM)*CCTCCAGGTAGAAGGCCATTTTTCCACCCTGTAG*(IABKFQ)-3') (SEQ ID NO.: 88) (Integrated DNA Technologies), IX Clarity™ JN Solution (JN Medsys), 0.15 U UNG (Roche) and 3 µL of plasma DNA or controls. The equivalent plasma volume per reaction was 60 µL. Each reaction mix was incubated at 40°C for 10 min to allow UNG to degrade carry-over PCR products, followed by 95°C for 10 min for UNG inactivation. The reaction mix was partitioned into approximately 10,000 individual reactions in the Clarity™ Digital PCR tube-strip (JN Medsys). Thereafter, the tube-strips were stabilised for 2 min, sealed with 230 µL sealing fluid and subjected to thermal cycling using the following parameters: 1 cycle at 95°C for 5 min, 40 cycles at 95°C for 50 sec and 58°C for 1.5 min. Afterward, the tube-strips were transferred to the Clarity™ Reader (JN Medsys), which detected and quantified fluorescence signals from all partitions. Absolute copy number of *EBNA1* in each reaction was determined by the Clarity™ Software (JN Medsys) after analysis of the ratio of positive partitions (i.e. those that contained amplified products) over the total number of partitions, using Poisson statistics.

**Determination of sensitivity and specificity of EBV cfDNA assays**

All three EBV cfDNA assays were benchmarked against the EBV qPCR assay routinely performed by the College of American Pathologists (CAP)-certified laboratory in SGH. The clinical sensitivity and clinical specificity of the SGH assay was reported as 79% and 100% respectively, based on 66 untreated nasopharyngeal carcinoma patients and 30 normal volunteers. In addition, sensitivity and specificity of EBV cfDNA assays were benchmarked against the 1st World Health Organization (WHO) International Standards for EBV, code 09/260; from National Institute for Biological *Standards* and Control (NIBSC). The NIBSC standards and nuclease-free water were spiked into EBV-free plasma to obtain 18 standards of 6 known EBV concentrations, ranging from 0 to 1,000,000 IU/mL. In addition, two aliquots of EBV-free plasma served as blank standards. The protocol of DNA extraction, sample distribution and EBV cfDNA assays of spike-in standards was identical to the one for clinical plasma samples.

**Enumeration of NPC CTCs**

CTCs from 1 mL of whole blood were captured using the microsieve technology and enumerated with the aid of biomarker characterization. The microsieve technology is a size-based method capable of isolating both epithelial and mesenchymal CTCs, unlike the affinity system, which only captures EpCAM-expressed CTCs. Cell counting, and image analysis were performed subject to sample availability, using the MetaMorph software (Molecular Devices) and manually verified by trained laboratory technicians. Cytokeratin-positive and CD45-negative nucleated cells were classified as canonical CTCs. Other nucleated cells that were negative for both cytokeratin and CD45 biomarkers were defined as potential CTCs. All nucleated cells with CD45-positive were classified as white blood cells.

**Statistical analysis**

Correlation study was carried out to correlate EBV levels amongst the NPC circulating biomarkers assays. Logistic ordinal regression modelling was used to evaluate pretreatment circulating biomarker quantitation relative to the dependent variable of clinical stage. Wilcoxon's signed-rank test with continuity correction (R.3.0.0) was conducted to compare paired pre and post-treatment levels of NPC circulating biomarkers. Correlation was performed using Microsoft Excel and the logistic ordinal regression model was performed using the "orm {rms}" library package in R. Alpha was set to 0.05 throughout. Survival analysis was performed using R 3.0.0 *survival* package to study survival distributions of continuous pretreatment levels of NPC circulating biomarkers and overall survival (Table 3), using log-rank testing to determine significance at a threshold of 0.05. 1 patient (Patient-025) was omitted from survival analysis, as the patient sought follow-up elsewhere.

## Claims

1. A method for detecting and/or quantifying the presence of a target nucleic acid sequence of a microorganism in a sample obtained from a subject, wherein the sample is a blood plasma or blood serum sample; wherein the sample comprises cell free DNAs (cfDNAs); and wherein said method comprises amplifying a target sequence in a CpG island of the nucleic acid of the microorganism, irrespective of the methylation status of the CpG island, and wherein the target nucleic acid sequence of the microorganism is cfDNA.

2. The method of claim 1, wherein the target sequence is within the 5' end of the nucleic acid of the CpG island of the nucleic acid of the microorganism.

3. The method of claim 1 or 2, wherein the method is a polymerase chain reaction (PCR); optionally wherein the polymerase chain reaction is selected from the group consisting of quantitative polymerase chain reaction, digital polymerase chain reaction, real-time polymerase chain reaction, and traditional polymerase chain reaction.

4. The method of any one of claims 1 to 3, wherein the microorganism is a virus, a bacterium, a fungus or a parasite;
optionally wherein the virus is Epstein-Barr virus (EBV);
optionally wherein the bacterium is a Mycobacterium; and
optionally wherein the Mycobacterium is *Mycobacterium tuberculosis*.

5. The method of claim 4, wherein the target sequence is in a CpG island of the *Bam*HI-W region of EBV;
optionally wherein the CpG island of the *Bam*HI-W region of EBV is selected from the group consisting of:

CTCCTCTCCAACCTTCGCTCCACCCTAGACCCCAGCTTCTGGCCTCCCCGGGTCCA
CCAGGCCAGCCGGAGGGACCCCGGCAGCCCGGGCGAGTCGCCTTCCCTCTCCCC
TGGCCTCTCCTTCCCGCCTCCCACCCGAGCCCCCTCAGCTTGCCTCCCCACCGGGT
CCATCAGGCCGGCCGGAGGGACCCCGGCGGCCCGGTGTCA (SEQ ID NO.: 6),

AGGCCATGCGCGCCCTGTCACCAGGCCTGCCAAAGAGCCAGATCTAAGGCCGGG
AGAGGCAGCCCCAAAGCGGGTGCAGTAACAGGTAATCTCTGGTAGTGATTTGGA
CCCGAAATCTGACACTTTAGAGCTCTGGAGGACTTTAAAACTCTAAAAATCAAA
ACTTTAGAGGCGAATGGGCGCCATTTTGTCCCCACGCGCGCATAATGGCGGACCT
AGGCCTAAAACCCCCAGGAAGCGGGTCTATGGTTGGCTGCGCTGCTGCTATCTTT
AGAGGGGAAAAGAGGAATAAGCCCCCAGACAGGGGAGTGGGCTTGTTTGTGACT
TCACCAAAGGTCAGGGCCCAAGGGGGTTCGCGTTGCTAGGCCACCTTCTCAGTC
CAGCGCGTTTACGTAAGC (SEQ ID NO.: 7),

CTGGTATAAAGTGGTCCTGCAGCTATTTCTGGTCGCATCAGAGCGCCAGGAGTCC
ACACAAATGTAAGAGGGGGTCTTCTACCTCTCCCTAGCCCTCCGCCCCCTCCAAG
GACTCGGGCCCAGTTTCTAACTTTTCCCCTTCCCTCCCTCGTCTTGCCCTGCGCCC
GGGGCCACCTTCATCACCGTCGCTGACTCCGCCATCCAAGCCTAGGGGAGACCG
AAGTGAAGGCCCTGGACCAACCCGGCCCGGGCCCCCCGGTATCGGGCCAGAGGT
AAGTGGACTTTAATTTTTTCTGCTAAGCCCAACACTCCACCACACCCAGGCACAC
ACTACACACACCCACCCGTCTCAGGGTCCCCTCGGA (SEQ ID NO.: 8), and

CGAGGAGGCGCCCGGAGTGGGGCCGGTCGGCTGGGCTGGCCGAGCCCGGGTCTG
GGAGGTCTGGGGTGGCGAGCCTGCTGTCTCAGGAGGGGCCTGGCTCCGCCGGGT
GGCCCTGGGGTAAGTCTGGGAGGCAGAGGGTCGGCCTAGGCCCGGGGAAGTGG
AGGGGGATCGCCCGGGTCTCTGTTGGCAGAGTCCGGGCGATCCTCTGAGACCCTC
CGGGCCCGGACGGTCGCCCTCAGCCCCCCAGACAGACCCCAGGGTCTCCAGGCA
GGGTCCGGCATCTTCAGGGGCAGCAGGCTCACCACCACAGGCCCCCCAGACCCG
GGTCTCGGCCAGCCGAGCCGACCGGCCCCGCGCCTGGCGCCTCCTCGGGGCCAG
CCGCCGGGGTTGGTTCTGCCCCTCTCTCTGTCCTTCAGAGGAACCAGGGACCTCG
GGCACCCCAGAGCCCCTCGGGCCCGCCTCCAGGCGCCCTCCTGGTCTCCGCTCCC
CTCTGAGCCCCGTTAAACCCAAAGAATGTCTGAGGGGAGCCACCCTCGG (SEQ ID NO.: 9);

optionally wherein the target sequence comprises the sequence selected from the group consisting of:

AGATCTAAGGCCGGGAGAGGCAGCCCCAAAGCGGGTGCAGTAACAGGTAATCTC TGGTAGTGATTTGGACCCGAAATCTGACACTTTAGAGCTCTGGAGGACTTTAAAA CTCTAAAAATCAAAACTTTAGAGGCGAATGGGCG (SEQ ID NO.: 1),

GGAATAAGCCCCCAGACAGGGGAGTGGGCTTGTTTGTGACTTCACCAAAGGTCA GGGCCCAAGGGGGTTCGCGTTGCTAGGCCACCTTCTCAGTCCAGCGCGTTTACGT AA (SEQ ID NO.: 10),

AGGAAGCGGGTCTATGGTTGGCTGCGCTGCTGCTATCTTTAGAGGGGAAAAGAG GAATAAGCCCCCAGACAGGGGAGTGGGCTTGTTTGTGACTTCACCAAAGGTCAG GGCCCAAGGGGGT-TCGCGTTGCTAGGCCACCTTCTCAGTC (SEQ ID NO.:11), a complementary sequence, a fragment and a variant thereof; optionally wherein amplifying the target sequence comprises the use of a pair of oligonucleotide primers, wherein the first oligonucleotide primer comprises a sequence selected from the group consisting of: 5'-AGATCTAAGGCCGGGAGAGG-3' (SEQ ID NO.:2), 5'-GGAATAAGCCCCCAGACAGG-3' (SEQ ID NO.:12), 5'-AGGAAGCGGGTCTATGGTTG-3' (SEQ ID NO.:13), a fragment and a variant thereof, and the second oligonucleotide primer comprises a sequence selected from the group consisting of 5'-CGCCCATTCGCCTCTAAAGT-3' (SEQ IDNO.:3), 5'-TTACGTAAACGCGCTGGACT-3' (SEQ ID NO.:14), 5'-GACTGAGAAGGTGGCCTAGC-3' (SEQ ID NO.: 15), a fragment and a variant thereof.

**6.** The method of claim 4 or 5, wherein amplifying the target sequence further comprises the use of a probe capable of binding to the target sequence;
optionally wherein the probe comprises a sequence selected from the group consisting of 5'-CTCTGGTAGTGATTTGGACCCGAAATCTG-3' (SEQ ID NO.: 16), 5'-CCACCTTCTCAGTCCAGCGCGTTT-3' (SEQ ID NO.: 17), 5'-GTGACTTCACCAAAGGTCAGGGCCC-3' (SEQ ID NO.: 18), 5'-GGTGGTAAGCGGTTCACCTTCAGGG-3' (SEQ ID NO.: 19), a fragment and variant thereof.

**7.** The method of any one of claims 4 to 6, further comprising calculating the copy number of *Bam*HI-W region in the amplified product, wherein the copy number is calculated using the following formula:

$$\text{Copy Number of } BamHI - W = \frac{DNA\ Quantity\ (ng)\ x\ Avogradro's\ Number}{88390.29\ (\text{Da})\ x\ 10^9}$$

**8.** The method of any one of claims 4 to 7, wherein the method is capable of detecting the lowest concentration of EBV of 100 IU (International Unit)/ml of sample, or 10 IU/ml of sample, or 1 IU/ml of sample.

**9.** A method for detecting and/or quantifying the presence of a target nucleic acid sequence of Epstein-Barr virus (EBV) in a sample obtained from a subject, wherein the sample is a blood plasma or blood serum sample; wherein the sample comprises cell free DNAs (cfDNAs); and wherein said method comprises amplifying a target sequence in the *Bam*HI-W region of EBV, wherein the target nucleic acid sequence of the Epstein-Barr virus is cfDNA, wherein the target sequence comprises the sequence of AGATCTAAGGCCGGGAGAGGCAGCCCCAAAGCGGGTGCAGTAACAGGTAATCTC TGGTAGTGATTTGGACCCGAAATCTGACACTTTAGAGCTCTGGAGGACTTTAAAA CTCTAAAAATCAAAACTTTAGAGGCGAATGGGCG (SEQ ID NO.: 1), wherein amplifying the target sequence comprises the use of a pair of oligonucleotide primers and a probe, wherein the first oligonucleotide primer comprises the sequence of 5'-AGATCTAAGGCCGGGAGAGG-3' (SEQ ID NO.: 2), and the second oligonucleotide primer comprises the sequence of 5'-CGCCCATTCGCCTCTAAAGT-3' (SEQ ID NO.:3), and wherein the probe comprises the sequence of 5'-(6-FAM)CTCTGGTAGTGATTTGGACCCGAAATCTG(TAMRA)-3' (SEQ ID NO.: 4),
and wherein the method is a quantitative polymerase chain reaction (qPCR).

**10.** The method of any one of claims 4 to 9, wherein the method further comprises amplifying a control comprising a target sequence of TTAGCAGCGACGAAGATCATGCGCTCACGCTCTCGGTGTCCTCATTCATCAGTTA TTCACAACGCTATGCTGTAACTCGACCTGACAAGACTGTACCTATGAGAAGGCA CTTGCTACCTTATGCAAGCGTCAGCCCGCGGTATCGCTTGG (SEQ ID NO.: 80), a complementary sequence, a fragment or a variant thereof;
optionally wherein amplifying the control comprises the use of a pair of oligonucleotide primers, wherein the first oligonucleotide primer comprises the sequence of 5'-CGCTCTCGGTGTCCTCATTC-3' (SEQ ID NO.: 81), a complementary sequence, a fragment or a variant thereof, and the second oligonucleotide primer comprises the sequence of 5'-GGCTGACGCTTGCATAAGGT-3' (SEQ ID NO.: 82), a complementary sequence, a fragment or a variant thereof;
optionally wherein amplifying the control further comprises the use of a probe capable of binding to the target sequence of the control, wherein the probe comprises the sequence of 5'-VIC-CACAACGCTATGCTGTAACTCGACCTGAC-TAMRA-3' (SEQ ID NO.: 83).

**11.** A method of detecting a disease associated with microorganism infection, or risk of developing a disease associated with microorganism infection in a subject, comprising detecting and/or quantifying the presence of a nucleic acid sequence of the microorganism using the method of any one of claims 1 to 10 in a sample obtained from the subject, wherein the presence of the nucleic acid sequence of the microorganism in the sample indicates that the subject has a disease associated with microorganism infection or is at risk of developing a disease associated with microorganism infection;
optionally wherein the disease associated with microorganism infection is selected from the group consisting of Alzheimer's disease, amyotrophic lateral sclerosis, anorexia nervosa, anxiety disorder, asthma, atherosclerosis, autoimmune diseases, cancers, chronic obstructive pulmonary disease, Crohn's disease, coronary heart disease, dementia, diabetes mellitus type 1, diabetes mellitus type 2, dilated cardiomyopathy, epilepsy, Guillain-Barre syndrome, irritable bowel syndrome, lupus, multiple sclerosis, myocardial infarction, Parkinson's disease, psoriasis, rheumatoid arthritis, sarcoidosis, schizophrenia, stroke, thromboangiitis obliterans, tourette syndrome and vasculitis;
optionally wherein the disease associated with microorganism infection is cancer;
optionally wherein the cancer is EBV-associated cancer;
optionally wherein the EBV-associated cancer is selected from the group consisting of nasopharyngeal carcinoma (NPC), gastric cancer, Hodgkin's lymphoma and Burkitt's lymphoma; and
optionally wherein the EBV-associated cancer is NPC.

**12.** A method of predicting the treatment outcome of a disease associated with microorganism infection in a patient, comprising:

(i) quantifying the nucleic acid sequence of the microorganism in a sample collected from the patient before treatment or before a treatment step, and quantifying the nucleic acid sequence of the microorganism in a sample collected from the same patient after treatment or after a treatment step;
(ii) comparing the amount of the nucleic acid sequence of the microorganism in the sample before and after treatment or a treatment step, wherein a decrease in the amount of the nucleic acid sequence of the microorganism in the sample after treatment or a treatment step indicates that treatment outcome of the disease associated with microorganism infection in the patient is positive,

wherein the quantifying of the nucleic acid sequence of the microorganism in the sample is performed according to the method of any one of claims 1-10,
optionally wherein the disease associated with microorganism infection is selected from the group consisting of Alzheimer's disease, amyotrophic lateral sclerosis, anorexia nervosa, anxiety disorder, asthma, atherosclerosis, autoimmune diseases, cancers, chronic obstructive pulmonary disease, Crohn's disease, coronary heart disease, dementia, diabetes mellitus type 1, diabetes mellitus type 2, dilated cardiomyopathy, epilepsy, Guillain-Barre syndrome, irritable bowel syndrome, lupus, multiple sclerosis, myocardial infarction, Parkinson's disease, psoriasis, rheumatoid arthritis, sarcoidosis, schizophrenia, stroke, thromboangiitis obliterans, tourette syndrome and vasculitis;
optionally wherein the disease associated with microorganism infection is cancer;
optionally wherein the cancer is EBV-associated cancer;
optionally wherein the EBV-associated cancer is selected from the group consisting of nasopharyngeal carcinoma (NPC), gastric cancer, Hodgkin's lymphoma and Burkitt's lymphoma; and
optionally wherein the EBV-associated cancer is NPC.

**13.** A kit for detecting and/or quantifying the nucleic acid sequence of a microorganism in a sample obtained from a subject, wherein the sample is a blood plasma or blood serum sample; wherein the sample comprises cell free DNAs (cfDNAs); and wherein the kit comprises a pair of oligonucleotide primers specific for the amplification of a target sequence in a CpG island of the nucleic acid of the microorganism.

**14.** The kit of claim 13, wherein the target sequence is within the 5'end of the CpG island of the nucleic acid of the microorganism.

**15.** The kit of claim 13 or 14, wherein the microorganism is a virus, a bacterium, a fungus or a parasite; optionally wherein the virus is Epstein-Barr virus (EBV).

**16.** The kit of claim 15, wherein the target sequence is in a CpG island of the *Bam*HI-W region of EBV; optionally wherein the target sequence comprises the sequence selected from the group consisting of:

AGATCTAAGGCCGGGAGAGGCAGCCCCAAAGCGGGTGCAGTAACAGGTAATCTC TGGTAGTGATTTGGACCCGAAATCTGACACTTTAGAGCTCTGGAGGACTTTAAAA CTCTAAAAATCAAAACTTTAGAGGCGAATGGGCG (SEQ ID NO.: 1),

GGAATAAGCCCCCAGACAGGGGAGTGGGCTTGTTTGTGACTTCACCAAAGGTCA GGGCCCAAGGGGGTTCGCGTTGCTAGGCCACCTTCTCAGTCCAGCGCGTTTACGT AA (SEQ ID NO.: 10),

AGGAAGCGGGTCTATGGTTGGCTGCGCTGCTGCTATCTTTAGAGGGGAAAAGAG GAATAAGCCCCCAGACAGGGGAGTGGGCTTGTTTGTGACTTCACCAAAGGTCAG GGCCCAAGGGGGT-TCGCGTTGCTAGGCCACCTTCTCAGTC (SEQ ID NO.: 11), a complementary sequence, a fragment and a variant thereof;

optionally wherein the first oligonucleotide primer comprises a sequence selected from the group consisting of: 5'-AGATCTAAGGCCGGGAGAGG-3' (SEQ ID NO.: 2), 5'-GGAATAAGCCCCCAGACAGG-3' (SEQ ID NO:12), 5'-AGGAAGCGGGTCTATGGTTG-3' (SEQ ID NO.:13), a fragment and a variant thereof, and the second oligonucleotide primer comprises a sequence selected from the group consisting of 5'-CGCCCATTCGCCTCTAAAGT-3' (SEQ ID NO.:3), 5'-TTACGTAAACGCGCTGGACT-3' (SEQ ID NO.:14), 5'-GACTGAGAAGGTGGCCTAGC-3' (SEQ ID NO.:15), a fragment and a variant thereof.

**17.** The kit of any one of claims 13 to 16, further comprises a probe capable of binding to the target sequence; optionally wherein the probe comprises a sequence selected from the group consisting of 5'-CTCTGGTAGTGATTTGGACCCGAAATCTG-3' (SEQ ID NO.: 16), 5'-CCACCTTCTCAGTCCAGCGCGTTT-3' (SEQ ID NO.: 17), 5'-GTGACTTCACCAAAGGTCAGGGCCC-3' (SEQ ID NO.: 18), 5'-GGTGGTAAGCGGTTCACCTTCAGGG-3' (SEQ ID NO.: 19), a fragment and variant thereof.

## Patentansprüche

**1.** Verfahren zum Nachweisen und/oder Quantifizieren der Gegenwart einer Zielnucleinsäuresequenz eines Mikroorganismus in einer von einem Individuum erhaltenen Probe, wobei die Probe eine Blutplasma- oder Blutserumprobe ist; wobei die Probe zellfreie DNA (cfDNA) umfasst; und wobei das Verfahren das Amplifizieren einer Zielsequenz in einer CpG-Insel der Nucleinsäure des Mikroorganismus, unabhängig von dem Methylierungszustand der CpG-Insel, umfasst und wobei die Zielnucleinsäuresequenz des Mikroorganismus cfDNA ist.

**2.** Verfahren nach Anspruch 1, wobei die Zielsequenz innerhalb des 5'-Endes der Nucleinsäure der CpG-Insel der Nucleinsäure des Mikroorganismus liegt.

**3.** Verfahren nach Anspruch 1 oder 2, wobei das Verfahren eine Polymerase-Kettenreaktion (PCR) ist, wobei die Polymerase-Kettenreaktion gegebenenfalls aus der aus quantitativer Polymerase-Kettenreaktion, digitaler Polymerase-Kettenreaktion, Echtzeit-Polymerase-Kettenreaktion und traditioneller Polymerase-Kettenreaktion bestehenden Gruppe ausgewählt ist.

**4.** Verfahren nach einem der Ansprüche 1 bis 3, wobei der Mikroorganismus ein Virus, ein Bakterium, ein Pilz oder ein Parasit ist;
wobei das Virus gegebenenfalls das Epstein-Barr-Virus (EBV) ist;
wobei das Bakterium gegebenenfalls ein Mykobakterium ist; und
wobei das Mykobakterium gegebenenfalls Mycobacterium tuberculosis ist.

**5.** Verfahren nach Anspruch 4, wobei die Zielsequenz in einer CpG-Insel der BamHI-W-Region von EBV liegt;
wobei die CpG-Insel der BamHI-W-Region von EBV gegebenenfalls aus folgender Gruppe ausgewählt ist:

CTCCTCTCCAACCTTCGCTCCACCCTAGACCCCAGCTTCTGGCCTCCCCGGGTCCA
CCAGGCCAGCCGGAGGGACCCCGGCAGCCCGGGCGAGTCGCCTTCCCTCTCCCC
TGGCCTCTCCTTCCCGCCTCCCACCCGAGCCCCCTCAGCTTGCCTCCCCACCGGGT
CCATCAGGCCGGCCGGAGGGACCCCGGCGGCCCGGTGTCA (SEQ ID NO: 6),
AGGCCATGCGCGCCCTGTCACCAGGCCTGCCAAAGAGCCAGATCTAAGGCCGGG
AGAGGCAGCCCCAAAGCGGGTGCAGTAACAGGTAATCTCTGGTAGTGATTTGGA
CCCGAAATCTGACACTTTAGAGCTCTGGAGGACTTTAAAACTCTAAAAATCAAA

ACTTTAGAGGCGAATGGGCGCCATTTTGTCCCCACGCGCGCATAATGGCGGACCT
AGGCCTAAAACCCCCAGGAAGCGGGTCTATGGTTGGCTGCGCTGCTGCTATCTTT
AGAGGGGAAAAGAGGAATAAGCCCCCAGACAGGGGAGTGGGCTTGTTTGTGACT
TCACCAAAGGTCAGGGCCCAAGGGGGTTCGCGTTGCTAGGCCACCTTCTCAGTC
CAGCGCGTTTACGTAAGC (SEQ ID NO: 7),
CTGGTATAAAGTGGTCCTGCAGCTATTTCTGGTCGCATCAGAGCGCCAGGAGTCC
ACACAAATGTAAGAGGGGGTCTTCTACCTCTCCCTAGCCCTCCGCCCCCTCCAAG
GACTCGGGCCCAGTTTCTAACTTTTCCCCTTCCCTCCCTCGTCTTGCCCTGCGCCC
GGGGCCACCTTCATCACCGTCGCTGACTCCGCCATCCAAGCCTAGGGGAGACCG
AAGTGAAGGCCCTGGACCAACCCGGCCCGGGCCCCCCGGTATCGGGCCAGAGGT
ACTACACACACCCACCCGTCTCAGGGTCCCCTCGGA (SEQ ID NO: 8) und
CGAGGAGGCGCCCGGAGTGGGGCCGGTCGGCTGGGCTGGCCGAGCCCGGGTCTG
GGAGGTCTGGGGTGGCGAGCCTGCTGTCTCAGGAGGGGCCTGGCTCCGCCGGGT
GGCCCTGGGGTAAGTCTGGGAGGCAGAGGGTCGGCCTAGGCCCGGGGAAGTGG
AGGGGGATCGCCCGGGTCTCTGTTGGCAGAGTCCGGGCGATCCTCTGAGACCCTC
CGGGCCCGGACGGTCGCCCTCAGCCCCCCAGACAGACCCCAGGGTCTCCAGGCA
GGGTCCGGCATCTTCAGGGGCAGCAGGCTCACCACCACAGGCCCCCCAGACCCG
GGTCTCGGCCAGCCGAGCCGACCGGCCCCGCGCCTGGCGCCTCCTCGGGGCCAG
CCGCCGGGGTTGGTTCTGCCCCTCTCTCTGTCCTTCAGAGGAACCAGGGACCTCG
GGCACCCCAGAGCCCCTCGGGCCCGCCTCCAGGCGCCCTCCTGGTCTCCGCTCCC
CTCTGAGCCCCGTTAAACCCAAAGAATGTCTGAGGGGAGCCACCCTCGG (SEQ ID NO:
9);

wobei die Zielsequenz gegebenenfalls die Sequenz umfasst, die aus folgender Gruppe ausgewählt ist:

AGATCTAAGGCCGGGAGAGGCAGCCCCAAAGCGGGTGCAGTAACAGGTAATCTC TGGTAGTGATTTGGACCCGAAATCTGACACTTTAGAGCTCTGGAGGACTTTAAAA CTCTAAAAATCAAAACTTTAGAGGCGAATGGGCG (SEQ ID NO: 1),
GGAATAAGCCCCCAGACAGGGGAGTGGGCTTGTTTGTGACTTCACCAAAGGTCA GGGCCCAAGGGGGTTCGCGTTGCTAGGCCACCTTCTCAGTCCAGCGCGTTTACGTAA (SEQ ID NO: 10),

AGGAAGCGGGTCTATGGTTGGCTGCGCTGCTGCTATCTTTAGAGGGGAAAAGAG GAATAAGCCCCCAGACAGGGGAGTGGGCTTGTTTGTGACTTCACCAAAGGTCAG GGCCCAAGGGGGTTCG-CGTTGCTAGGCCACCTTCTCAGTC (SEQ ID NO: 11) oder eine komplementäre Sequenz, ein Fragment oder eine Variante davon;
wobei das Amplifizieren der Zielsequenz gegebenenfalls die Verwendung eines Paars Oligonucleotid-Primer umfasst, wobei der erste Oligonucleotid-Primer eine Sequenz umfasst, die aus folgender Gruppe ausgewählt ist: 5'-AGATCTAAGGCCGGGAGAGG-3' (SEQ ID NO: 2), 5'-GGAATAAGCCCCCAGACAGG-3' (SEQ ID NO:12), 5'-AGGAAGCGGGTCTATGGTTG-3' (SEQ ID NO: 13), ein Fragment oder eine Variante davon und der zweite Oligonucleotid-Primer eine Sequenz umfasst, die aus folgender Gruppe ausgewählt ist: 5'-CGCCCATTCGCCTCTAAAGT-3' (SEQ ID NO: 3), 5'-TTACGTAAACGCGCTGGACT-3' (SEQ ID NO: 14), 5'-GACTGAGAAGGTGGCCTAGC-3' (SEQ ID NO: 15), ein Fragment oder eine Variante davon.

**6.** Verfahren nach Anspruch 4 oder 5, wobei das Amplifizieren der Zielsequenz außerdem die Verwendung einer Sonde umfasst, die zur Bindung an die Zielsequenz in der Lage ist;
wobei die Sonde gegebenenfalls eine Sequenz umfasst, die aus folgender Gruppe ausgewählt ist: 5'-CTCTGGTAGTGATTTGGACCCGAAATCTG-3' (SEQ ID NO: 16), 5'-CCACCTTCTCAGTCCAGCGCGTTT-3' (SEQ ID NO: 17), 5'-GTGACTTCACCAAAGGTCAGGGCCC-3' (SEQ ID NO: 18), 5'-GGTGGTAAGCGGTTCACCTTCAGGG-3' (SEQ ID NO: 19), ein Fragment oder eine Variante davon.

**7.** Verfahren nach einem der Ansprüche 4 bis 6, das außerdem das Berechnen der Kopienanzahl der BamHI-W-Region in dem amplifizierten Produkt umfasst, wobei die Kopienanzahl unter Verwendung der folgenden Formel berechnet wird:

$$\text{Kopienanzahl von BamHI-W} = \frac{DNA\text{–}Menge(ng)xAvogrado\text{–}Konstante}{88390{,}29(Da)x10^9}$$

**8.** Verfahren nach einem der Ansprüche 4 bis 7, wobei das Verfahren in der Lage ist, die geringste Konzentration von EBV von 100 IU (Internationale Einheit)/ml Probe oder 10 IU/ml Probe oder 1 IU/ml Probe nachzuweisen.

**9.** Verfahren zum Nachweisen und/oder Quantifizieren der Gegenwart einer Zielnucleinsäuresequenz von Epstein-Barr-Virus (EBV) in einer von einem Individuum erhaltenen Probe, wobei die Probe eine Blutplasma- oder Blutserumprobe ist; wobei die Probe zellfreie DNA (cfDNA) umfasst; und wobei das Verfahren das Amplifizieren einer Zielsequenz in der BamHI-W-Region von EBV umfasst, wobei die Zielnucleinsäuresequenz des Epstein-Barr-Virus cfDNA ist, wobei die Zielsequenz die Sequenz von AGATCTAAGGCCGGGAGAGGCAGCCCCAAAGCGGGTGCAGTAACAGGTAATCTC TGGTAGTGATTTGGACCCGAAATCTGACACTTTAGAGCTCTGGAGGACTTTAAAA CTCTAAAAATCAAAACTTTAGAGGCGAATGGGCG (SEQ ID NO: 1) umfasst,
wobei das Amplifizieren der Zielsequenz die Verwendung eines Paars Oligonucleotid-Primer und einer Sonde umfasst, wobei der erste Oligonucleotid-Primer die Sequenz von 5'-AGATCTAAGGCCGGGAGAGG-3' (SEQ ID NO: 2) umfasst und der zweite Oligonucleotid-Primer die Sequenz von 5'-CGCCCATTCGCCTCTAAAGT-3' (SEQ ID NO: 3) umfasst und wobei die Sonde die Sequenz von 5'-(6-FAM)CTCTGGTAGTGATTTGGACCCGAAATCTG(TAMRA)-3' (SEQ ID NO: 4) umfasst und wobei das Verfahren eine quantitative Polymerase-Kettenreaktion (qPCR) ist.

**10.** Verfahren nach einem der Ansprüche 4 bis 9, wobei das Verfahren außerdem das Amplifizieren einer Kontrolle umfasst, die eine Zielsequenz von TTAGCAGCGACGAAGATCATGCGCTCACGCTCTCGGTGTCCTCATTCAT-CAGTTAT TCACAACGCTATGCTGTAACTCGACCTGACAAGACTGTACCTATGAGAAGGCACTT GCTACCT-TATGCAAGCGTCAGCCCGCGGTATCGCTTGG (SEQ ID NO: 80), eine komplementäre Sequenz, ein Fragment oder eine Variante davon umfasst;
wobei das Amplifizieren der Kontrolle gegebenenfalls die Verwendung eines Paars Oligonucleotid-Primer umfasst, wobei der erste Oligonucleotid-Primer die Sequenz von 5'-CGCTCTCGGTGTCCTCATTC-3' (SEQ ID NO: 81), eine komplementäre Sequenz, ein Fragment oder eine Variante davon umfasst und der zweite Oligonucleotid-Primer die Sequenz von 5'-GGCTGACGCTTGCATAAGGT-3' (SEQ ID NO: 82), eine komplementäre Sequenz, ein Fragment oder eine Variante davon umfasst;
wobei das Amplifizieren der Kontrolle gegebenenfalls außerdem die Verwendung einer Sonde umfasst, die zur Bindung an die Zielsequenz der Kontrolle in der Lage ist, wobei die Sonde die Sequenz von 5'-VIC-CACAACGCTATGCTGTAACTCGACCTGAC-TAMRA-3' (SEQ ID NO: 83) umfasst.

**11.** Verfahren zum Nachweisen einer Erkrankung, die mit einer Mikroorganismus-Infektion in Zusammenhang steht, oder einer Gefahr der Entwicklung einer Erkrankung, die mit einer Mikroorganismus-Infektion in Zusammenhang steht, in einem Individuum, das das Nachweisen und/oder Quantifizieren der Gegenwart einer Nucleinsäuresequenz des Mikroorganismus unter Verwendung eines Verfahrens nach einem der Ansprüche 1 bis 10 in einer von dem Individuum erhaltenen Probe umfasst, wobei die Gegenwart der Nucleinsäuresequenz des Mikroorganismus in der Probe anzeigt, dass das Individuum eine Erkrankung aufweist, die mit einer Mikroorganismus-Infektion in Zusammenhang steht, oder bei dem die Gefahr einer Entwicklung einer Erkrankung besteht, die mit einer Mikroorganismus-Infektion in Zusammenhang steht;
wobei die mit einer Mikroorganismus-Infektion in Zusammenhang stehende Erkrankung aus der aus Alzheimer-Krankheit, amyotropher Lateralsklerose, Anorexia nervosa, Angststörungen, Asthma, Atherosklerose, Autoimmunerkrankungen, Krebserkrankungen, chronisch obstruktiver Lungenerkrankung, Morbus Crohn, koronarer Herzkrankheit, Demenz, Diabetes mellitus Typ 1, Diabetes mellitus Typ 2, dilatativer Kardiomyopathie, Epilepsie, Guillain-Barre-Syndrom, Reizdarmsyndrom, Lupus, multipler Sklerose, Myokardinfarkt, Morbus Parkinson, Psoriasis, rheumatoider Arthritis, Sarkoidose, Schizophrenie, Schlaganfall, Thromboangiitis obliterans, Tourette-Syndrom und Vaskulitis bestehenden Gruppe ausgewählt ist;
wobei die mit einer Mikroorganismus-Infektion in Zusammenhang stehende Erkrankung gegebenenfalls eine Krebserkrankung ist;
wobei die Krebserkrankung gegebenenfalls eine Krebserkrankung ist, die mit EBV in Zusammenhang steht;
wobei die mit EBV in Zusammenhang stehende Krebserkrankung gegebenenfalls aus der aus Nasopharynxkarzinom (NPC), Magenkrebs, Hodgkin-Lymphom und Burkitt-Lymphom bestehenden Gruppe ausgewählt ist; und
wobei die mit EBV in Zusammenhang stehende Krebserkrankung gegebenenfalls NPC ist.

**12.** Verfahren zur Prognose des Behandlungsergebnisses einer mit einer Mikroorganismus-Infektion in Zusammenhang stehenden Erkrankung bei einem Patienten, das Folgendes umfasst:

(i) das Quantifizieren der Nucleinsäuresequenz des Mikroorganismus in einer Probe, die dem Patienten vor der Behandlung oder vor einem Behandlungsschritt entnommen wurde, und das Quantifizieren der Nucleinsäuresequenz des Mikroorganismus in einer Probe, die dem gleichen Patienten nach der Behandlung oder nach einem Behandlungsschritt entnommen wurde;
(ii) das Vergleichen der Menge der Nucleinsäuresequenz des Mikroorganismus in der Probe vor und nach der Behandlung oder einem Behandlungsschritt, wobei eine Abnahme der Menge der Nucleinsäuresequenz des Mikroorganismus in der Probe nach der Behandlung oder einem Behandlungsschritt anzeigt, dass das Behandlungsergebnis der mit einer Mikroorganismus-Infektion in Zusammenhang stehenden Erkrankung bei dem Patienten positiv ist,

wobei das Quantifizieren der Nucleinsäuresequenz des Mikroorganismus in der Probe gemäß einem Verfahren nach einem der Ansprüche 1 bis 10 durchgeführt wird,
wobei die mit einer Mikroorganismus-Infektion in Zusammenhang stehende Erkrankung gegebenenfalls aus der aus Alzheimer-Krankheit, amyotropher Lateralsklerose, Anorexia nervosa, Angststörungen, Asthma, Atherosklerose, Autoimmunerkrankungen, Krebserkrankungen, chronisch obstruktiver Lungenerkrankung, Morbus Crohn, koronarer Herzkrankheit, Demenz, Diabetes mellitus Typ 1, Diabetes mellitus Typ 2, dilatativer Kardiomyopathie, Epilepsie, Guillain-Barre-Syndrom, Reizdarmsyndrom, Lupus, multipler Sklerose, Myokardinfarkt, Morbus Parkinson, Psoriasis, rheumatoider Arthritis, Sarkoidose, Schizophrenie, Schlaganfall, Thromboangiitis obliterans, Tourette-Syndrom und Vaskulitis bestehenden Gruppe ausgewählt ist;
wobei die mit einer Mikroorganismus-Infektion in Zusammenhang stehenden Erkrankung gegebenenfalls eine Krebserkrankung ist;
wobei die Krebserkrankung gegebenenfalls eine Krebserkrankung ist, die mit EBV in Zusammenhang steht;
wobei die mit EBV in Zusammenhang stehende Krebserkrankung gegebenenfalls aus der aus Nasopharynxkarzinom (NPC), Magenkrebs, Hodgkin-Lymphom und Burkitt-Lymphom bestehenden Gruppe ausgewählt ist; und
wobei die mit EBV in Zusammenhang stehende Krebserkrankung gegebenenfalls NPC ist.

**13.** Set zum Nachweisen und/oder Quantifizieren der Nucleinsäuresequenz eines Mikroorganismus in einer von einem Individuum erhaltenen Probe, wobei die Probe eine Blutplasma- oder Blutserumprobe ist; wobei die Probe zellfreie DNA (cfDNA) umfasst; und wobei das Set ein Paar Oligonucleotid-Primer umfasst, die für die Amplifikation einer Zielsequenz in einer CpG-Insel der Nucleinsäure des Mikroorganismus spezifisch sind.

**14.** Set nach Anspruch 13, wobei die Zielsequenz innerhalb des 5'-Endes der CpG-Insel der Nucleinsäure des Mikroorganismus liegt.

**15.** Set nach Anspruch 13 oder 14, wobei der Mikroorganismus ein Virus, ein Bakterium, ein Pilz oder ein Parasit ist; wobei das Virus gegebenenfalls das Epstein-Barr-Virus (EBV) ist.

**16.** Set nach Anspruch 15, wobei die Zielsequenz in einer CpG-Insel der BamHI-W-Region von EBV liegt;
wobei die Zielsequenz gegebenenfalls die Sequenz umfasst, die aus folgender Gruppe ausgewählt ist:

AGATCTAAGGCCGGGAGAGGCAGCCCCAAAGCGGGTGCAGTAACAGGTAATCTC
TGGTAGTGATTTGGACCCGAAATCTGACACTTTAGAGCTCTGGAGGACTTTAAAA
CTCTAAAAATCAAAACTTTAGAGGCGAATGGGCG (SEQ ID NO: 1),

GGAATAAGCCCCCAGACAGGGGAGTGGGCTTGTTTGTGACTTCACCAAAGGTCA
GGGCCCAAGGGGGTTCGCGTTGCTAGGCCACCTTCTCAGTCCAGCGCGTTTACGTAA
(SEQ ID NO: 10),

AGGAAGCGGGTCTATGGTTGGCTGCGCTGCTGCTATCTTTAGAGGGGAAAAGAG
GAATAAGCCCCCAGACAGGGGAGTGGGCTTGTTTGTGACTTCACCAAAGGTCAG GGCCCAAGGGGGTTCG-CGTTGCTAGGCCACCTTCTCAGTC (SEQ ID NO: 11), eine komplementäre Sequenz, ein Fragment oder eine Variante davon;
wobei der erste Oligonucleotid-Primer gegebenenfalls eine Sequenz umfasst, die aus folgender Gruppe ausgewählt ist: 5'-AGATCTAAGGCCGGGAGAGG-3' (SEQ ID NO: 2), 5'-GGAATAAGCCCCCAGACAGG-3' (SEQ ID NO: 12), 5'-AGGAAGCGGGTCTATGGTTG-3' (SEQ ID NO: 13), ein Fragment oder eine Variante davon und der zweite Oligonucleotid-Primer eine Sequenz umfasst, die aus folgender Gruppe ausgewählt ist: 5'-CGCCCATTCGCCTCTAAAGT-3' (SEQ ID NO: 3), 5'-TTACGTAAACGCGCTGGACT-3' (SEQ ID NO: 14), 5'-GACTGAGAAGGTGGCCTAGC-3' (SEQ ID NO: 15), ein Fragment oder eine Variante davon.

**17.** Set nach einem der Ansprüche 13 bis 16, das außerdem eine Sonde umfasst, die zur Bindung an die Zielsequenz in der Lage ist:
wobei die Sonde gegebenenfalls eine Sequenz umfasst, die aus folgender Gruppe ausgewählt ist: 5'-CTCTGGTAGTGATTTGGACCCGAAATCTG-3' (SEQ ID NO: 16), 5'-CCACCTTCTCAGTCCAGCGCGTTT-3' (SEQ ID NO: 17), 5'-GTGACTTCACCAAAGGTCAGGGCCC-3' (SEQ ID NO: 18), 5'-GGTGGTAAGCGGTTCACCTTCAGGG-3' (SEQ ID NO: 19), ein Fragment oder eine Variante davon.

## Revendications

**1.** Procédé pour détecter et/ou quantifier la présence d'une séquence d'acide nucléique cible d'un micro-organisme dans un échantillon obtenu chez un sujet, dans lequel l'échantillon est un échantillon de plasma sanguin ou de sérum sanguin ; dans lequel l'échantillon comprend des ADN libres circulants (ADNlc) ; et dans lequel ledit procédé comprend l'amplification d'une séquence cible dans un îlot CpG de l'acide nucléique du micro-organisme, sans tenir compte de l'état de méthylation de l'îlot CpG, et dans lequel la séquence d'acide nucléique cible du micro-organisme

est de l'ADNlc.

2. Procédé selon la revendication 1, dans lequel la séquence cible est comprise dans l'extrémité 5' de l'acide nucléique de l'îlot CpG de l'acide nucléique du micro-organisme.

3. Procédé selon la revendication 1 ou 2, dans lequel le procédé est une réaction en chaîne par polymérase (PCR) ; facultativement dans lequel la réaction en chaîne par polymérase est sélectionnée dans le groupe consistant en une réaction en chaîne par polymérase quantitative, une réaction en chaîne par polymérase numérique, une réaction en chaîne par polymérase en temps réel, et une réaction en chaîne par polymérase traditionnelle.

4. Procédé selon l'une quelconque des revendications 1 à 3, dans lequel le micro-organisme est un virus, une bactérie, un champignon ou un parasite ;
facultativement dans lequel le virus est le virus d'Epstein-Barr (EBV) ;
facultativement dans lequel la bactérie est une mycobactérie ; et
facultativement dans lequel la mycobactérie est *Mycobacterium tuberculosis.*

5. Procédé selon la revendication 4, dans lequel la séquence cible est dans un îlot CpG de la région *Bam*HI-W de l'EBV ; facultativement dans lequel l'îlot CpG de la région *Bam*HI-W de l'EBV est sélectionné dans le groupe consistant en :

CTCCTCTCCAACCTTCGCTCCACCCTAGACCCCAGCTTCTGGCCTCCCCGGGTCCA
CCAGGCCAGCCGGAGGGACCCCGGCAGCCCGGGCGAGTCGCCTTCCCTCTCCCC
TGGCCTCTCCTTCCCGCCTCCCACCCGAGCCCCCTCAGCTTGCCTCCCCACCGGGT
CCATCAGGCCGGCCGGAGGGACCCCGGCGGCCCGGTGTCA

(SEQ ID NO: 6),

AGGCCATGCGCGCCCTGTCACCAGGCCTGCCAAAGAGCCAGATCTAAGGCCGGG
AGAGGCAGCCCCAAAGCGGGTGCAGTAACAGGTAATCTCTGGTAGTGATTTGGA
CCCGAAATCTGACACTTTAGAGCTCTGGAGGACTTTAAAACTCTAAAAATCAAA
ACTTTAGAGGCGAATGGGCGCCATTTTGTCCCCACGCGCGCATAATGGCGGACCT
AGGCCTAAAACCCCCAGGAAGCGGGTCTATGGTTGGCTGCGCTGCTGCTATCTTT
AGAGGGGAAAAGAGGAATAAGCCCCCAGACAGGGGAGTGGGCTTGTTTGTGACT
TCACCAAAGGTCAGGGCCCAAGGGGGTTCGCGTTGCTAGGCCACCTTCTCAGTC
CAGCGCGTTTACGTAAGC

(SEQ ID NO: 7),

CTGGTATAAAGTGGTCCTGCAGCTATTTCTGGTCGCATCAGAGCGCCAGGAGTCC
ACACAAATGTAAGAGGGGGTCTTCTACCTCTCCCTAGCCCTCCGCCCCCTCCAAG
GACTCGGGCCCAGTTTCTAACTTTTCCCCTTCCCTCCCTCGTCTTGCCCTGCGCCC
GGGGCCACCTTCATCACCGTCGCTGACTCCGCCATCCAAGCCTAGGGGAGACCG
AAGTGAAGGCCCTGGACCAACCCGGCCCGGGCCCCCCGGTATCGGGCCAGAGGT
AAGTGGACTTTAATTTTTTCTGCTAAGCCCAACACTCCACCACACCCAGGCACAC
ACTACACACACCCACCCGTCTCAGGGTCCCCTCGGA

(SEQ ID NO: 8), et

CGAGGAGGCGCCCGGAGTGGGGCCGGTCGGCTGGGCTGGCCGAGCCCGGGTCTG
GGAGGTCTGGGGTGGCGAGCCTGCTGTCTCAGGAGGGGCCTGGCTCCGCCGGGT
GGCCCTGGGGTAAGTCTGGGAGGCAGAGGGTCGGCCTAGGCCCGGGGAAGTGG
AGGGGGATCGCCCGGGTCTCTGTTGGCAGAGTCCGGGCGATCCTCTGAGACCCTC
CGGGCCCGGACGGTCGCCCTCAGCCCCCCAGACAGACCCCAGGGTCTCCAGGCA
GGGTCCGGCATCTTCAGGGGCAGCAGGCTCACCACCACAGGCCCCCCAGACCCG
GGTCTCGGCCAGCCGAGCCGACCGGCCCCGCGCCTGGCGCCTCCTCGGGGCCAG
CCGCCGGGGTTGGTTCTGCCCCTCTCTCTGTCCTTCAGAGGAACCAGGGACCTCG
GGCACCCCAGAGCCCCTCGGGCCCGCCTCCAGGCGCCCTCCTGGTCTCCGCTCCC
CTCTGAGCCCCGTTAAACCCAAAGAATGTCTGAGGGGAGCCACCCTCGG

(SEQ ID NO: 9) ;

facultativement dans lequel la séquence cible comprend la séquence sélectionnée dans le groupe consistant en :

AGATCTAAGGCCGGGAGAGGCAGCCCCAAAGCGGGTGCAGTAACAGGTAATCTC
TGGTAGTGATTTGGACCCGAAATCTGACACTTTAGAGCTCTGGAGGACTTTAAAA
CTCTAAAAATCAAAACTTTAGAGGCGAATGGGCG (SEQ ID NO.: 1),
GGAATAAGCCCCCAGACAGGGGAGTGGGCTTGTTTGTGACTTCACCAAAGGTCA
GGGCCCAAGGGGGTTCGCGTTGCTAGGCCACCTTCTCAGTCCAGCGCGTTTACGT
AA,

(SEQ ID NO: 10),

AGGAAGCGGGTCTATGGTTGGCTGCGCTGCTGCTATCITTAGAGGGGAAAAGAG
GAATAAGCCCCCAGACAGGGGAGTGGGCTTGTTTGTGACTTCACCAAAGGTCAG
GGCCCAAGGGGGTTCGCGTTGCTAGGCCACCTTCTCAGTC ,
(SEQ ID NO: 11), une séquence complémentaire, un fragment et un variant de celles-ci ; facultativement dans lequel l'amplification de la séquence cible comprend l'utilisation d'une paire d'amorces oligonucléotidiques, dans lequel la première amorce oligonucléotidique comprend une séquence sélectionnée dans le groupe consistant en : 5'-AGATCTAAGGCCGGGAGAGG-3' (SEQ ID NO: 2), 5'-GGAATAAGCCCCCAGACAGG-3' (SEQ ID NO: 12), 5'-AGGAAGCGGGTCTATGGTTG-3' (SEQ ID NO: 13), un fragment et un variant de celles-ci, et la seconde amorce oligonucléotidique comprend une séquence sélectionnée dans le groupe consistant en 5'-CGCCCATTCGCCTCTAAAGT-3' (SEQ ID NO: 3), 5'-TTACGTAAACGCGCTGGACT-3' (SEQ ID NO: 14), 5'-GACTGAGAAGGTGGCCTAGC-3' (SEQ ID NO: 15), un fragment et un variant de celles-ci.

**6.** Procédé selon la revendication 4 ou 5, dans lequel l'amplification de la séquence cible comprend en outre l'utilisation d'une sonde capable de se lier à la séquence cible ; facultativement dans lequel la sonde comprend une séquence sélectionnée dans le groupe consistant en 5'-CTCTGGTAGTGATTTGGACCCGAAATCTG-3' (SEQ ID NO: 16), 5'-CCACCTTCTCAGTCCAGCGCGTTT-3' (SEQ ID NO: 17), 5'-GTGACTTCACCAAAGGTCAGGGCCC-3' (SEQ ID NO: 18), 5'-GGTGGTAAGCGGTTCACCTTCAGGG-3' (SEQ ID NO: 19), un fragment et un variant de celles-ci.

**7.** Procédé selon l'une quelconque des revendications 4 à 6, comprenant en outre le calcul du nombre de copies de la région *Bam*HI-W dans le produit amplifié, dans lequel le nombre de copies est calculé en utilisant la formule suivante :

$$\text{Nombre de copies de } BamHI - W = \frac{Quantité\ d'ADN\ x\ Nombre\ d'Avogadro}{88390{,}29\ (Da)\ x\ 10^{9}}$$

8. Procédé selon l'une quelconque des revendications 4 à 7, dans lequel le procédé est capable de détecter la concentration la plus faible d'EBV de 100UI (unité internationale)/ml d'échantillon, ou 10 UI/ml d'échantillon, ou 1 IU/ml d'échantillon.

9. Procédé pour détecter et/ou quantifier la présence d'une séquence d'acide nucléique cible du virus d'Epstein-Barr (EBV) dans un échantillon obtenu chez un sujet, dans lequel l'échantillon est un échantillon de plasma sanguin ou de sérum sanguin ; dans lequel l'échantillon comprend des ADN libres circulants (ADNlc) ; et dans lequel ledit procédé comprend l'amplification d'une séquence cible dans la région *Bam*HI-W de l'EBV, dans lequel la séquence d'acide nucléique cible du virus d'Epstein-Barr est de l'ADNlc, dans lequel la séquence cible comprend la séquence de

AGATCTAAGGCCGGGAGAGGCAGCCCCAAAGCGGGTGCAGTAACAGGTAATCTC
TGGTAGTGATTTGGACCCGAAATCTGACACTTTAGAGCTCTGGAGGACTTTAAAA
CTCTAAAAATCAAAACTTTAGAGGCGAATGGGCG ,

(SEQ ID NO: 1),

dans lequel l'amplification de la séquence cible comprend l'utilisation d'une paire d'amorces oligonucléotidiques et d'une sonde, dans lequel la première amorce oligonucléotidique comprend la séquence de 5'-AGATCTAAGGCCGGGAGAGG-3' (SEQ ID NO: 2), et la seconde amorce oligonucléotidique comprend la séquence de 5'-CGCCCATTCGCCTCTAAAGT-3' (SEQ ID NO: 3), et dans lequel la sonde comprend la séquence de 5'-(6-FAM)CTCTGGTAGTGATTTGGACCCGAAATCTG(TAMRA)-3' (SEQ ID NO: 4), et dans lequel le procédé est une réaction en chaîne par polymérase quantitative (qPCR).

10. Procédé selon l'une quelconque des revendications 4 à 9, dans lequel le procédé comprend en outre l'amplification d'un contrôle comprenant une séquence cible de TTAGCAGCGACGAAGATCATGCGCTCACGCTCTCGGTGTCCTCATTCATCAGTTA TTCACAACGCTATGCTGTAACTCGACCTGACAAGACTGTACCTATGAGAAGGCA CTTGCTACCTTATGCAAGCGTCAGCCCGCGGTATCGCTTGG ,
(SEQ ID NO: 80), une séquence complémentaire, un fragment ou un variant de celle-ci ; facultativement dans lequel l'amplification du contrôle comprend l'utilisation d'une paire d'amorces oligonucléotidiques, dans lequel la première amorce oligonucléotidique comprend la séquence de 5'-CGCTCTCGGTGTCCTCATTC-3' (SEQ ID NO: 81), une séquence complémentaire, un fragment ou un variant de celle-ci, et la seconde amorce oligonucléotidique comprend la séquence de 5'-GGCTGACGCTTGCATAAGGT-3' (SEQ ID NO: 82), une séquence complémentaire, un fragment ou un variant de celle-ci ; facultativement dans lequel l'amplification du contrôle comprend en outre l'utilisation d'une sonde capable de se lier à la séquence cible du contrôle, dans lequel la sonde comprend la séquence de 5'-VIC-CACAACGCTATGCTGTAACTCGACCTGAC-TAMRA-3' (SEQ ID NO: 83).

11. Procédé de détection d'une maladie associée à une infection par un micro-organisme, ou d'un risque de développer une maladie associée à une infection par un micro-organisme chez un sujet, comprenant la détection et/ou la quantification de la présence d'une séquence d'acide nucléique du micro-organisme en utilisant le procédé selon l'une quelconque des revendications 1 à 10 dans un échantillon obtenu chez le sujet, dans lequel la présence de la séquence d'acide nucléique du micro-organisme dans l'échantillon indique que le sujet présente une maladie associée à une infection par le micro-organisme ou est à risque de développer une maladie associée à une infection par le micro-organisme ;
facultativement dans lequel la maladie associée à une infection par le micro-organisme est sélectionnée dans le groupe consistant en la maladie d'Alzheimer, la sclérose latérale amyotrophique, l'anorexie nerveuse, un trouble de l'anxiété, l'asthme, l'athérosclérose, des maladies auto-immunes, des cancers, la bronchopneumopathie chronique obstructive, la maladie de Crohn, une coronaropathie, la démence, le diabète de type 1, le diabète de type 2, la cardiomyopathie dilatée, l'épilepsie, le syndrome de Guillain-Barré, le syndrome du côlon irritable, le lupus, la sclérose en plaques, l'infarctus du myocarde, la maladie de Parkinson, le psoriasis, la polyarthrite rhumatoïde, la sarcoïdose, la schizophrénie, un accident vasculaire cérébral, la thromboangéite oblitérante, le syndrome de la Tourette et la vascularite ;

facultativement dans lequel la maladie associée à une infection par un micro-organisme est le cancer ;
facultativement dans lequel le cancer est un cancer associé à l'EBV ;
facultativement dans lequel le cancer associé à l'EBV est sélectionné dans le groupe consistant en un carcinome du nasopharynx (CNP), le cancer gastrique, le lymphome hodgkinien et le lymphome de Burkitt ; et
facultativement dans lequel le cancer associé à l'EBV est un CNP.

**12.** Procédé de prédiction du résultat du traitement d'une maladie associée à une infection par un micro-organisme chez un patient, comprenant :

(i) la quantification de la séquence d'acide nucléique du micro-organisme dans un échantillon prélevé chez le patient avant le traitement ou avant une étape du traitement, et la quantification de la séquence d'acide nucléique du micro-organisme dans un échantillon prélevé chez le même patient après le traitement ou après une étape du traitement ;
(ii) la comparaison de la quantité de la séquence d'acide nucléique du micro-organisme dans l'échantillon avant et après le traitement ou une étape du traitement, dans lequel une réduction de la quantité de la séquence d'acide nucléique du micro-organisme dans l'échantillons après le traitement ou une étape du traitement indique que le résultat du traitement de la maladie associée à une infection par un micro-organisme chez le patient est positif,

dans lequel la quantification de la séquence d'acide nucléique du micro-organisme dans l'échantillon est réalisée selon le procédé de l'une quelconque des revendications 1 à 10, facultativement dans lequel la maladie associée à une infection par le micro-organisme est sélectionnée dans le groupe consistant en la maladie d'Alzheimer, la sclérose latérale amyotrophique, l'anorexie nerveuse, un trouble de l'anxiété, l'asthme, l'athérosclérose, des maladies auto-immunes, des cancers, la bronchopneumopathie chronique obstructive, la maladie de Crohn, une coronaropathie, la démence, le diabète de type 1, le diabète de type 2, la cardiomyopathie dilatée, l'épilepsie, le syndrome de Guillain-Barré, le syndrome du côlon irritable, le lupus, la sclérose en plaques, l'infarctus du myocarde, la maladie de Parkinson, le psoriasis, la polyarthrite rhumatoïde, la sarcoïdose, la schizophrénie, un accident vasculaire cérébral, la thromboangéite oblitérante, le syndrome de la Tourette et la vascularite ;
facultativement dans lequel la maladie associée à une infection par un micro-organisme est le cancer ;
facultativement dans lequel le cancer est un cancer associé à l'EBV ;
facultativement dans lequel le cancer associé à l'EBV est sélectionné dans le groupe consistant en un carcinome du nasopharynx (CNP), le cancer gastrique, le lymphome hodgkinien et le lymphome de Burkitt ; et
facultativement dans lequel le cancer associé à l'EBV est un CNP.

**13.** Kit pour détecter et/ou quantifier la séquence d'acide nucléique cible d'un micro-organisme dans un échantillon obtenu chez un sujet, dans lequel l'échantillon est un échantillon de plasma sanguin ou de sérum sanguin ; dans lequel l'échantillon comprend des ADN libres circulants (ADNlc) ; et dans lequel le kit comprend une paire d'amorces oligonucléotidiques spécifiques pour l'amplification d'une séquence cible dans un îlot CpG de l'acide nucléique du micro-organisme.

**14.** Kit selon la revendication 13, dans lequel la séquence cible est comprise dans l'extrémité 5' de l'îlot CpG de l'acide nucléique du micro-organisme.

**15.** Kit selon la revendication 13 ou 14, dans lequel le micro-organisme est un virus, une bactérie, un champignon ou un parasite ;
facultativement dans lequel le virus est le virus d'Epstein-Barr (EBV).

**16.** Kit selon la revendication 15, dans lequel la séquence cible est dans un îlot CpG de la région *Bam*HI-W de l'EBV ; facultativement dans lequel la séquence cible comprend la séquence sélectionnée dans le groupe consistant en :

AGATCTAAGGCCGGGAGAGGCAGCCCCAAAGCGGGTGCAGTAACAGGTAATCTC
TGGTAGTGATTTGGACCCGAAATCTGACACTTTAGAGCTCTGGAGGACTTTAAAA
CTCTAAAAATCAAAACTTTAGAGGCGAATGGGCG ,

(SEQ ID NO: 1),

GGAATAAGCCCCCAGACAGGGGAGTGGGCTTGTTTGTGACTTCACCAAAGGTCA GGGCCCAAGGGGGTTCGCGTTGCTAGGCCACCTTCTCAGTCCAGCGCGTTTACGT AA ,

(SEQ ID NO: 10),

AGGAAGCGGGTCTATGGTTGGCTGCGCTGCTGCTATCTTTAGAGGGGAAAAGAG GAATAAGCCCCCAGACAGGGGAGTGGGCTTGTTTGTGACTTCACCAAAGGTCAG

GGCCCAAGGGGGTTCGCGTTGCTAGGCCACCTTCTCAGTC ,

(SEQ ID NO: 11), une séquence complémentaire, un fragment et un variant de celles-ci ; facultativement dans lequel la première amorce oligonucléotidique comprend une séquence sélectionnée dans le groupe consistant en : 5'-AGATCTAAGGCCGGGAGAGG-3' (SEQ ID NO: 2), 5'-GGAATAAGCCCCCAGACAGG-3' (SEQ ID NO: 12), 5'-AGGAAGCGGGTCTATGGTTG-3' (SEQ ID NO: 13), un fragment et un variant de celles-ci, et la seconde amorce oligonucléotidique comprend une séquence sélectionnée dans le groupe consistant en 5'-CGCCCATTCGCCTCTAAAGT-3' (SEQ ID NO: 3), 5'-TTACGTAAACGCGCTGGACT-3' (SEQ ID NO: 14), 5'-GACTGAGAAGGTGGCCTAGC-3' (SEQ ID NO: 15), un fragment et un variant de celles-ci.

**17.** Kit selon l'une quelconque des revendications 13 à 16, comprenant en outre une sonde capable de se lier à la séquence cible ;
facultativement dans lequel la sonde comprend une séquence sélectionnée dans le groupe consistant en 5'-CTCTGGTAGTGATTTGGACCCGAAATCTG-3' (SEQ ID NO: 16), 5'-CCACCTTCTCAGTCCAGCGCGTTT-3' (SEQ ID NO: 17), 5'-GTGACTTCACCAAAGGTCAGGGCCC-3' (SEQ ID NO: 18), 5'-GGTGGTAAGCGGTTCACCTTCAGGG-3' (SEQ ID NO: 19), un fragment et un variant de celles-ci.

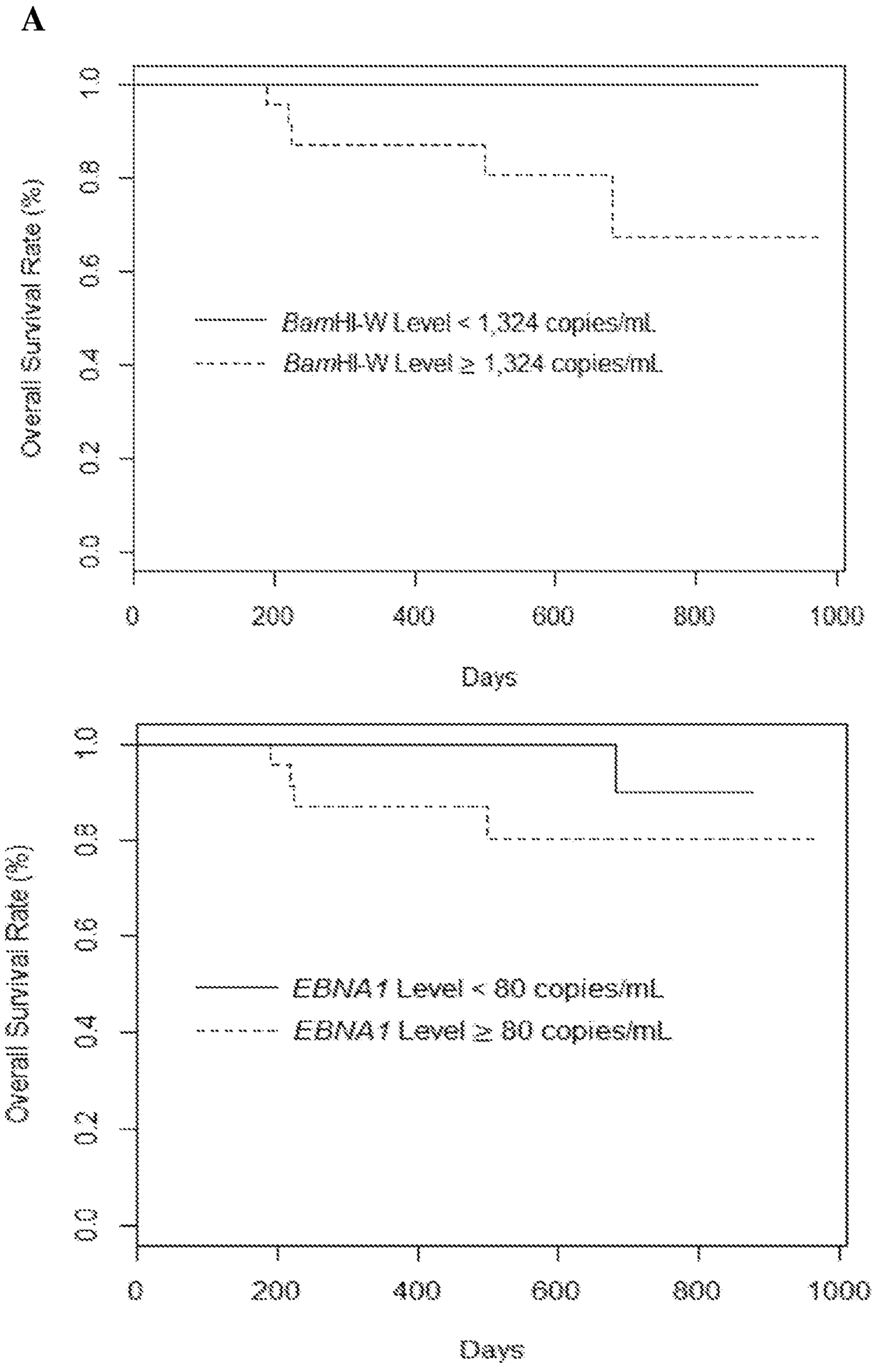
A
Overall Survival Rate (%)
1.0
0.8
0.6
0.4
0.2
0.0
BamHI-W Level < 1,324 copies/mL
BamHI-W Level ≥ 1,324 copies/mL
0
200
400
600
800
1000
Days
Overall Survival Rate (%)
EBNA1 Level < 80 copies/mL
EBNA1 Level ≥ 80 copies/mL
Days

Fig. 1

C

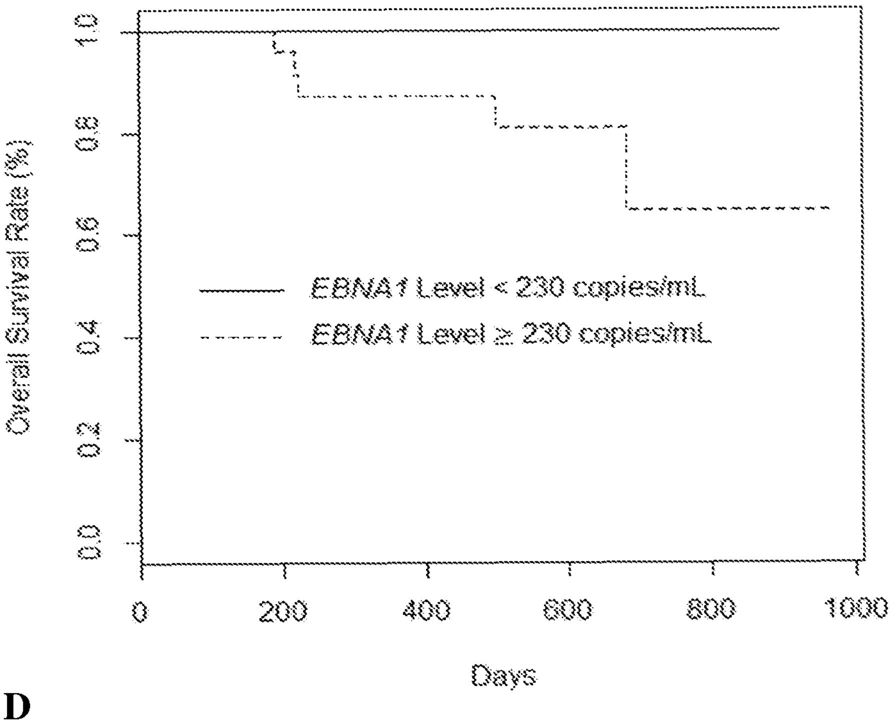
1.0
0.8
0.6
0.4
0.2
0.0
Overall Survival Rate (%)
EBNA1 Level < 230 copies/mL
EBNA1 Level ≥ 230 copies/mL
0
200
400
600
800
1000
Days

D

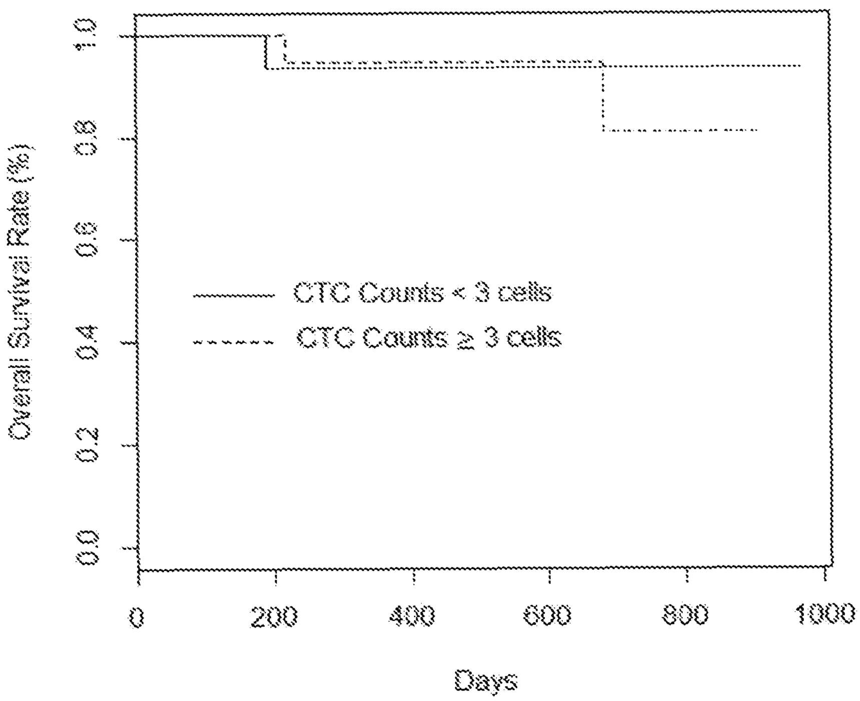
1.0
0.8
0.6
0.4
0.2
0.0
Overall Survival Rate (%)
CTC Counts < 3 cells
CTC Counts ≥ 3 cells
0
200
400
600
800
1000
Days

**Fig. 1 (Con't)**

E

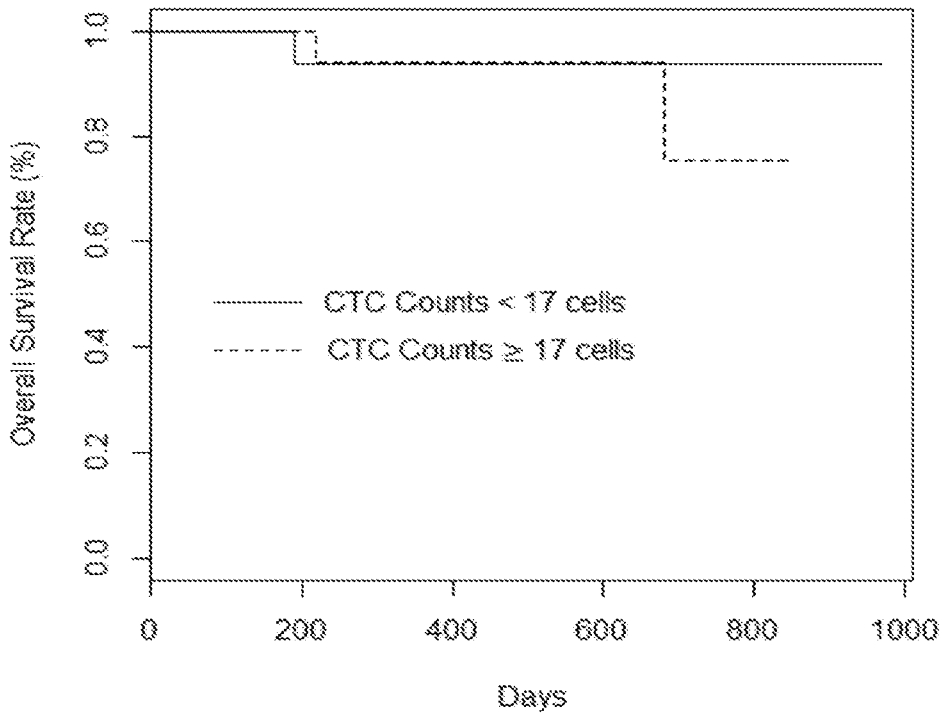
1.0
0.8
0.6
0.4
0.2
0.0
Overall Survival Rate (%)
CTC Counts < 17 cells
CTC Counts ≥ 17 cells
0
200
400
600
800
1000
Days

Fig. 1 (Con't)

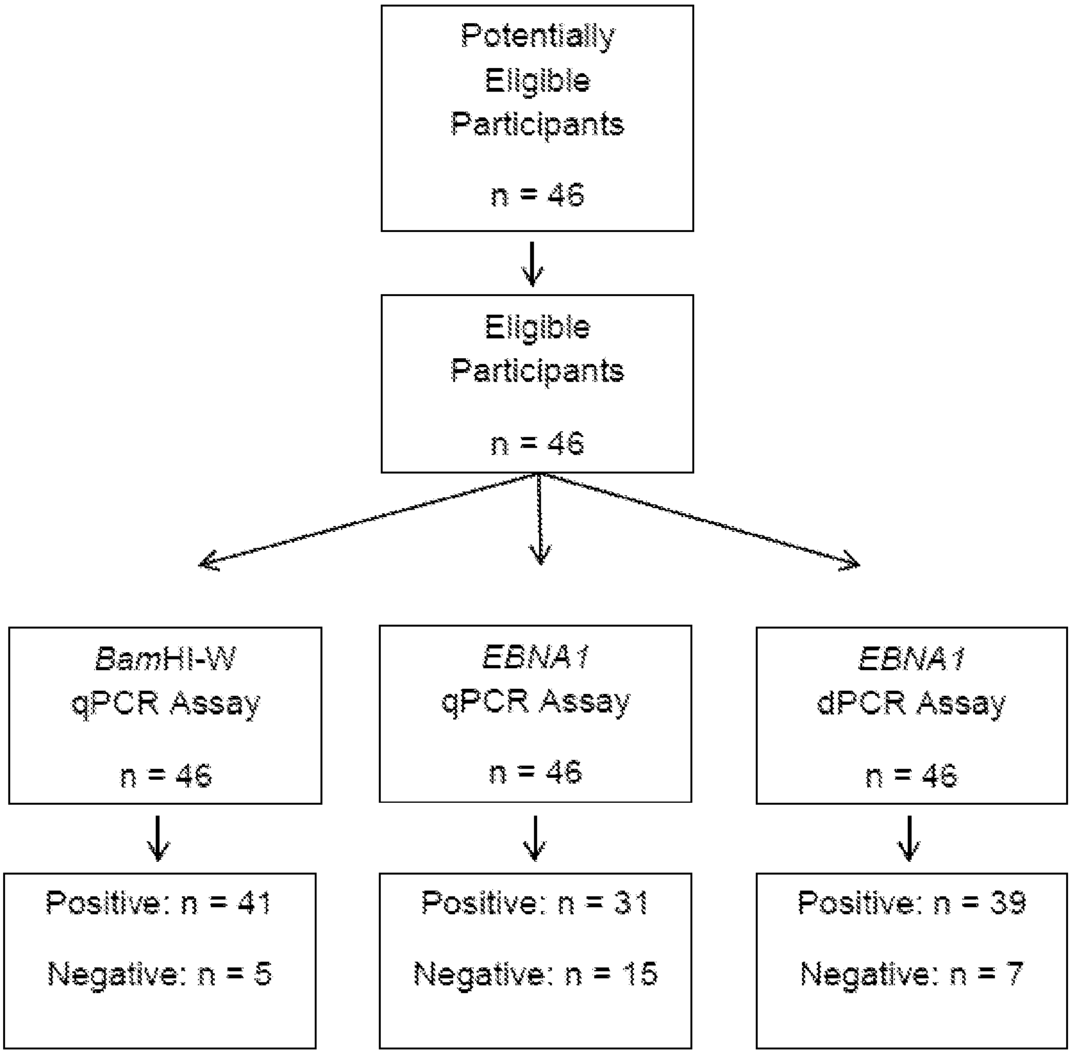
Potentially
Eligible
Participants
n = 46
Eligible
Participants
n = 46
*Bam*HI-*W*
qPCR Assay
n = 46
*EBNA1*
qPCR Assay
n = 46
*EBNA1*
dPCR Assay
n = 46
Positive: n = 41
Negative: n = 5
Positive: n = 31
Negative: n = 15
Positive: n = 39
Negative: n = 7

**Fig. 2**

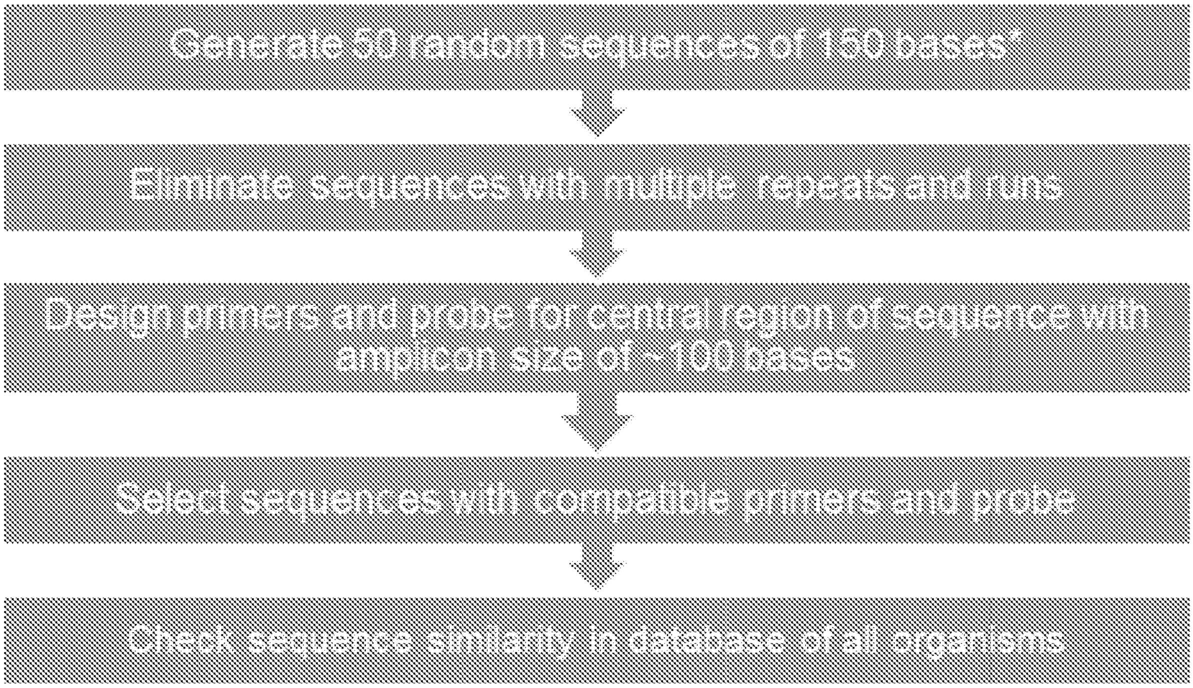
Generate 50 random sequences of 150 bases*
Eliminate sequences with multiple repeats and runs
Design primers and probe for central region of sequence with ampicon size of ~100 bases
Select sequences with compatible primers and probe
Check sequence similarity in database of all organisms

**Fig. 3**

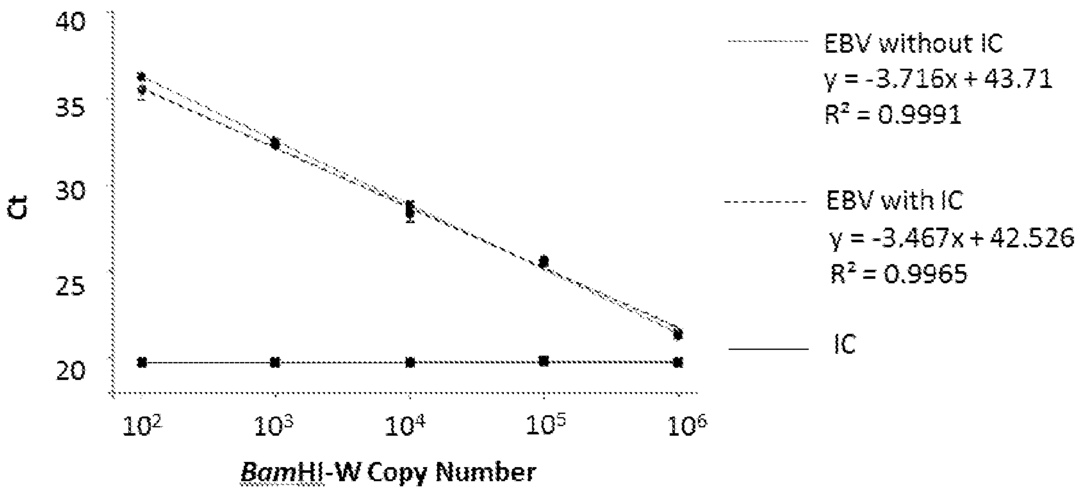
40
35
30
25
20
Ct
10^2
10^3
10^4
10^5
10^6
BamHI-W Copy Number
EBV without IC
y = -3.716x + 43.71
R² = 0.9991
EBV with IC
y = -3.467x + 42.526
R² = 0.9965
IC

**Fig. 4**

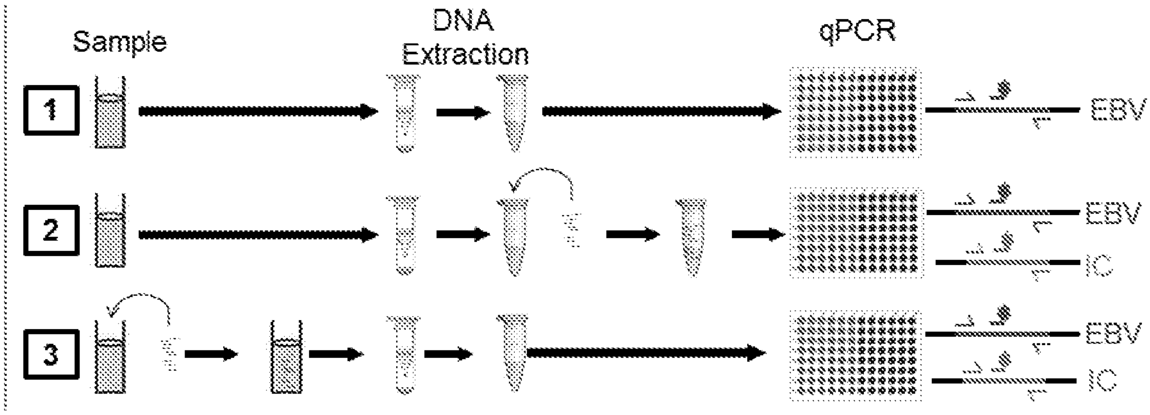
Sample
DNA Extraction
qPCR
1
2
3
EBV
EBV
IC
EBV
IC

Fig. 5

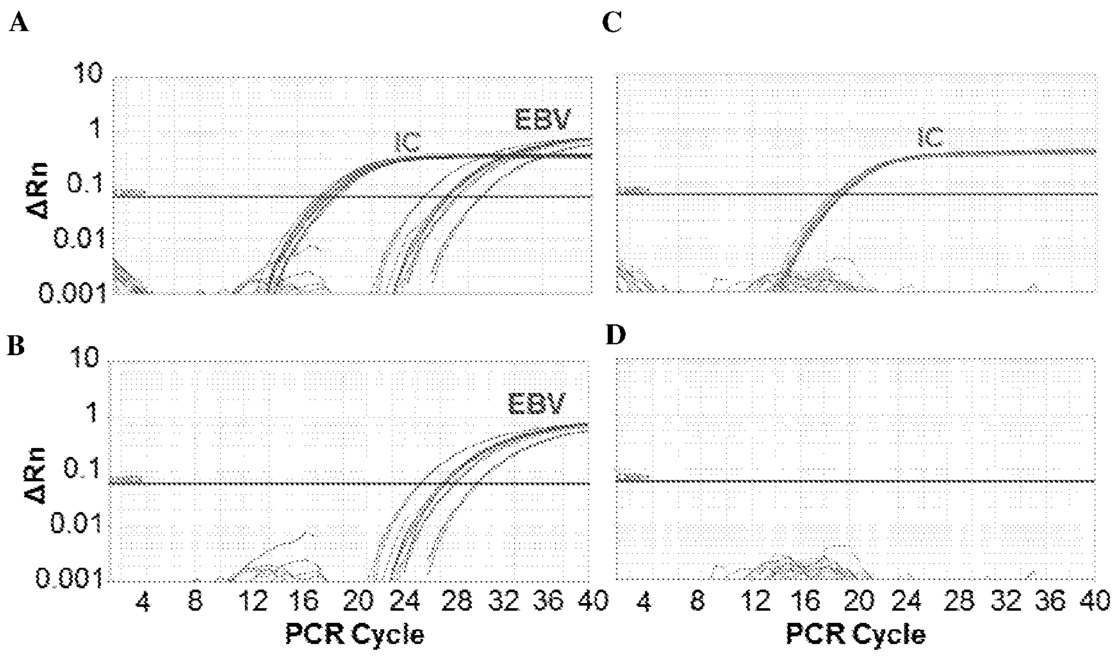
A
C
B
D
10
1
0.1
0.01
0.001
ΔRn
IC
EBV
IC
EBV
4 8 12 16 20 24 28 32 36 40
PCR Cycle
4 8 12 16 20 24 28 32 36 40
PCR Cycle

Fig. 6

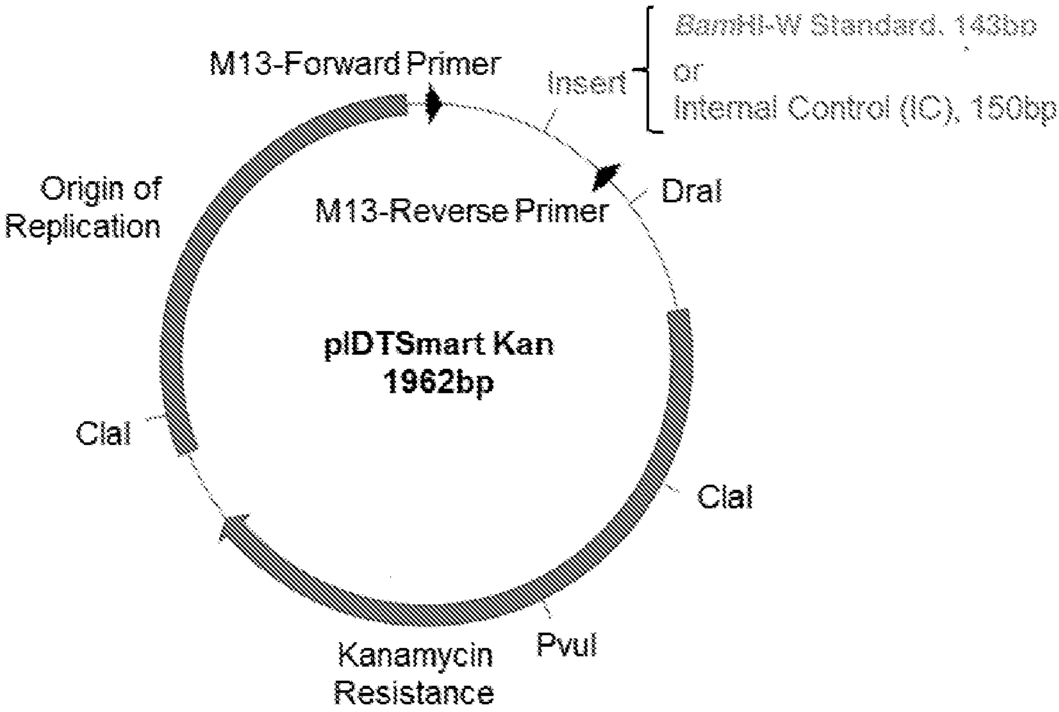
BamHI-W Standard, 143bp
or
Internal Control (IC), 150bp
Insert
M13-Forward Primer
M13-Reverse Primer
DraI
Origin of Replication
pIDTSmart Kan
1962bp
ClaI
ClaI
PvuI
Kanamycin Resistance

Fig. 7

**A**

| Score | Expect | Identities | Gaps |
|---|---|---|---|
| 265 bits(143) | 3e-75 | 143/143(100%) | 0/143(0%) |

```
Query  78   AGATCTAAGGCCGGGAGAGGCAGCCCCAAAGCGGGTGCAGTAACAGGTAATCTCTGGTAG
            ||||||||||||||||||||||||||||||||||||||||||||||||||||||||||||
Sbjct  1    AGATCTAAGGCCGGGAGAGGCAGCCCCAAAGCGGGTGCAGTAACAGGTAATCTCTGGTAG

Query  138  TGATTTGGACCCGAAATCTGACACTTTAGAGCTCTGGAGGACTTTAAAACTCTAAAAATC
            ||||||||||||||||||||||||||||||||||||||||||||||||||||||||||||
Sbjct  61   TGATTTGGACCCGAAATCTGACACTTTAGAGCTCTGGAGGACTTTAAAACTCTAAAAATC

Query  198  AAAACTTTAGAGGCGAATGGGCG  220
            |||||||||||||||||||||||
Sbjct  121  AAAACTTTAGAGGCGAATGGGCG  143
```

**B**

| Score | Expect | Identities | Gaps |
|---|---|---|---|
| 265 bits(143) | 2e-75 | 143/143(100%) | 0/143(0%) |

```
Query  46   CGCCCATTCGCCTCTAAAGTTTTGATTTTTAGAGTTTTAAAGTCCTCCAGAGCTCTAAAG
            ||||||||||||||||||||||||||||||||||||||||||||||||||||||||||||
Sbjct  1    CGCCCATTCGCCTCTAAAGTTTTGATTTTTAGAGTTTTAAAGTCCTCCAGAGCTCTAAAG

Query  106  TGTCAGATTTCGGGTCCAAATCACTACCAGAGATTACCTGTTACTGCACCCGCTTTGGGG
            ||||||||||||||||||||||||||||||||||||||||||||||||||||||||||||
Sbjct  61   TGTCAGATTTCGGGTCCAAATCACTACCAGAGATTACCTGTTACTGCACCCGCTTTGGGG

Query  166  CTGCCTCTCCCGGCCTTAGATCT  188
            |||||||||||||||||||||||
Sbjct  121  CTGCCTCTCCCGGCCTTAGATCT  143
```

**Fig. 8**

**C**

```
Score             Expect   Identities        Gaps
278 bits(150)     3e-79    150/150(100%)     0/150(0%)

Query  78   TTAGCAGCGACGAAGATCATGCGCTCACGCTCTCGGTGTCCTCATTCATCAGTTATTCAC
            ||||||||||||||||||||||||||||||||||||||||||||||||||||||||||||
Sbjct  1    TTAGCAGCGACGAAGATCATGCGCTCACGCTCTCGGTGTCCTCATTCATCAGTTATTCAC

Query  138  AACGCTATGCTGTAACTCGACCTGACAAGACTGTACCTATGAGAAGGCACTTGCTACCTT
            ||||||||||||||||||||||||||||||||||||||||||||||||||||||||||||
Sbjct  61   AACGCTATGCTGTAACTCGACCTGACAAGACTGTACCTATGAGAAGGCACTTGCTACCTT

Query  198  ATGCAAGCGTCAGCCCGCGGTATCGCTTGG  227
            ||||||||||||||||||||||||||||||
Sbjct  121  ATGCAAGCGTCAGCCCGCGGTATCGCTTGG  150
```

**D**

```
Score             Expect   Identities        Gaps
278 bits(150)     3e-79    150/150(100%)     0/150(0%)

Query  46   CCAAGCGATACCGCGGGCTGACGCTTGCATAAGGTAGCAAGTGCCTTCTCATAGGTACAG
            ||||||||||||||||||||||||||||||||||||||||||||||||||||||||||||
Sbjct  1    CCAAGCGATACCGCGGGCTGACGCTTGCATAAGGTAGCAAGTGCCTTCTCATAGGTACAG

Query  106  TCTTGTCAGGTCGAGTTACAGCATAGCGTTGTGAATAACTGATGAATGAGGACACCGAGA
            ||||||||||||||||||||||||||||||||||||||||||||||||||||||||||||
Sbjct  61   TCTTGTCAGGTCGAGTTACAGCATAGCGTTGTGAATAACTGATGAATGAGGACACCGAGA

Query  166  GCGTGAGCGCATGATCTTCGTCGCTGCTAA  195
            ||||||||||||||||||||||||||||||
Sbjct  121  GCGTGAGCGCATGATCTTCGTCGCTGCTAA  150
```

**Fig. 8 (Con't)**

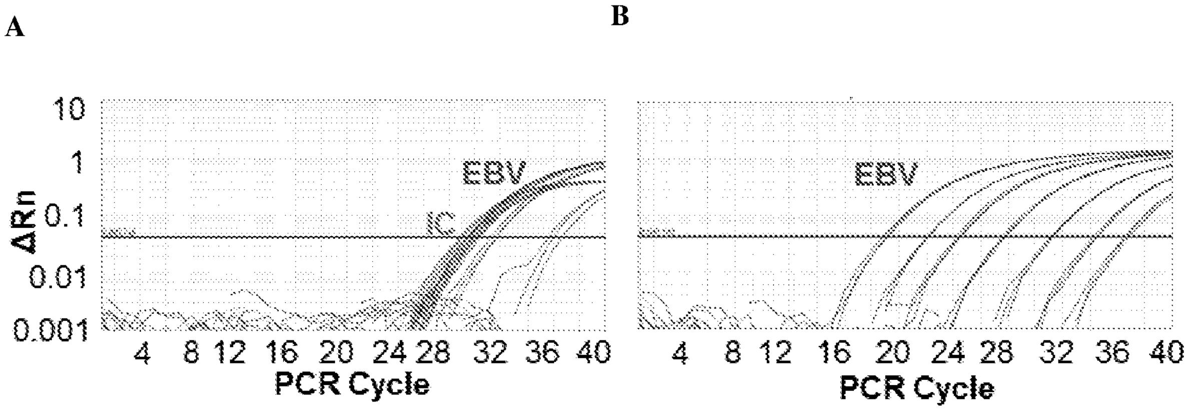
A
B
10
1
0.1
0.01
0.001
ΔRn
EBV
IC
4 8 12 16 20 24 28 32 36 40
PCR Cycle
EBV
4 8 12 16 20 24 28 32 36 40
PCR Cycle

**Fig. 9**

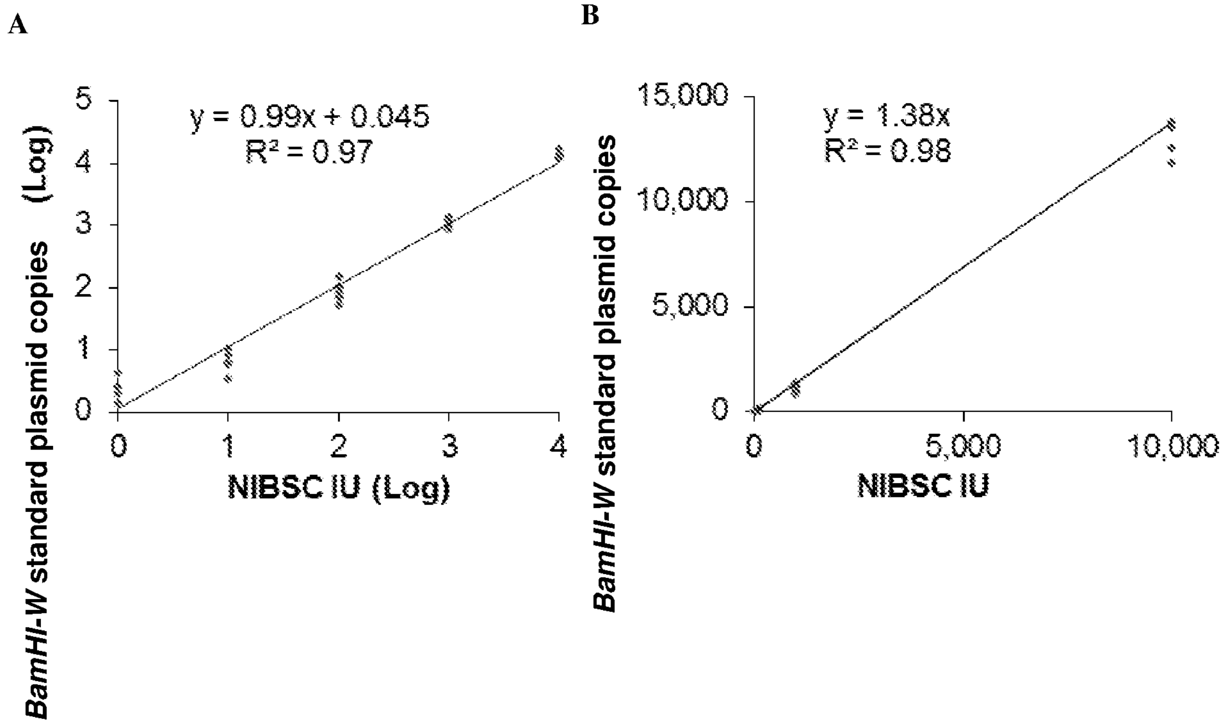
A
B
y = 0.99x + 0.045
R² = 0.97
BamHI-W standard plasmid copies (Log)
NIBSC IU (Log)
0 1 2 3 4 5
y = 1.38x
R² = 0.98
BamHI-W standard plasmid copies
15,000
10,000
5,000
0
0 5,000 10,000
NIBSC IU

**Fig. 10**

A

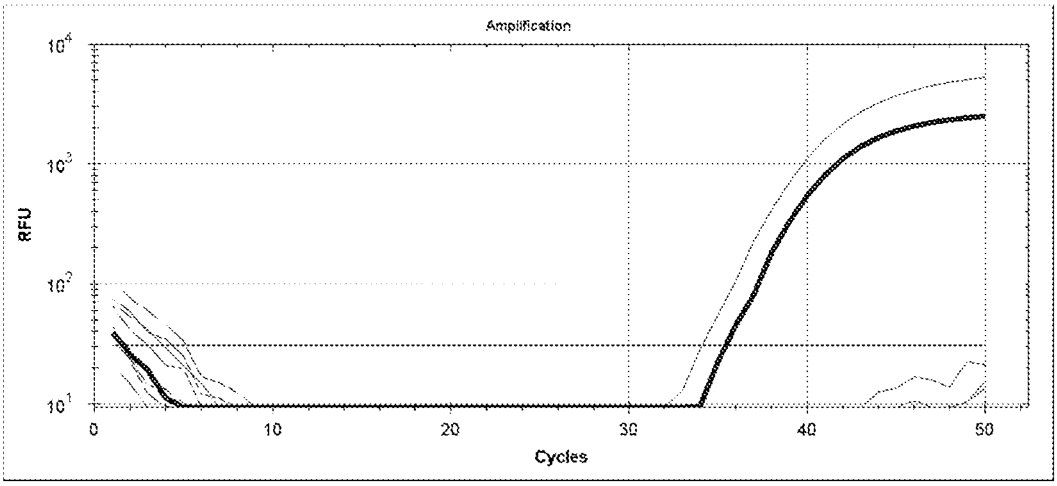
Amplification
$10^4$
$10^3$
$10^2$
$10^1$
RFU
0
10
20
30
40
50
Cycles

B

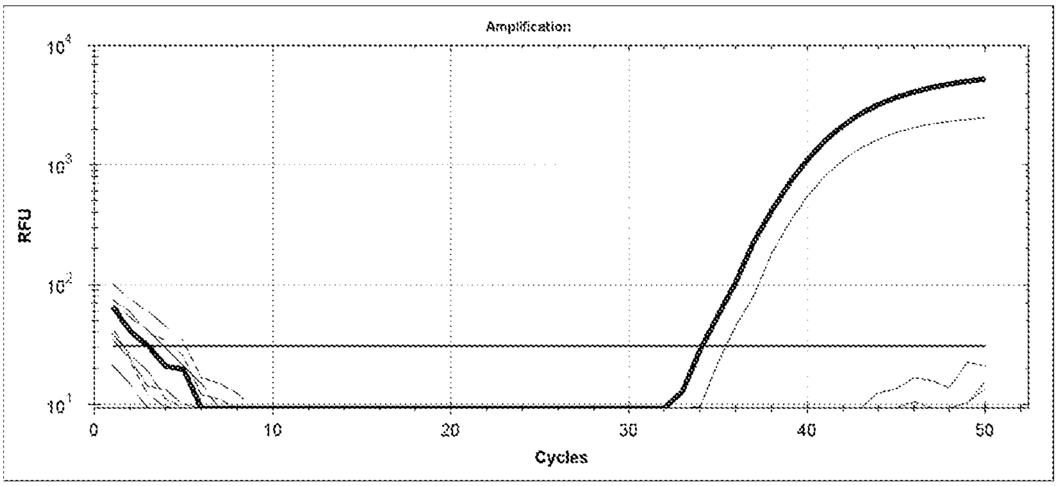
Amplification
$10^4$
$10^3$
$10^2$
$10^1$
RFU
0
10
20
30
40
50
Cycles

**Fig. 11**

C

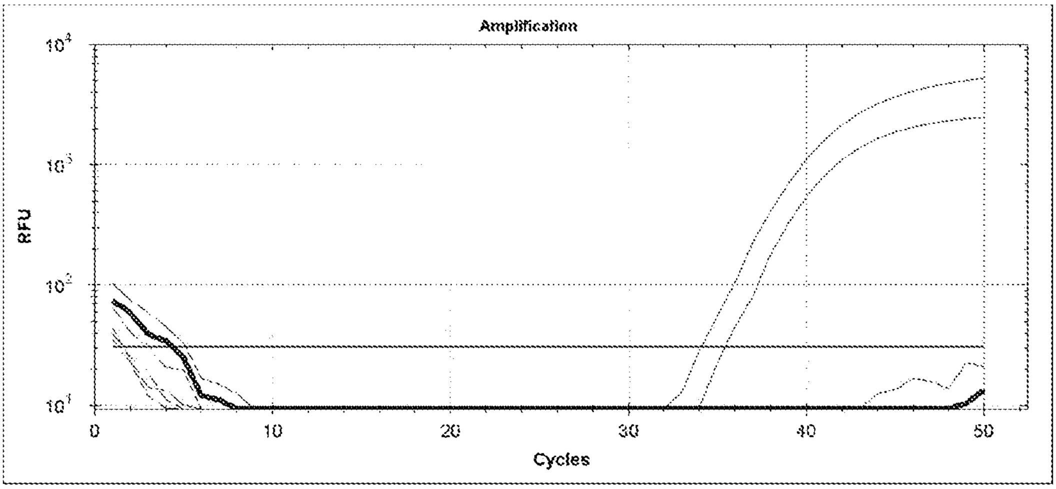


D

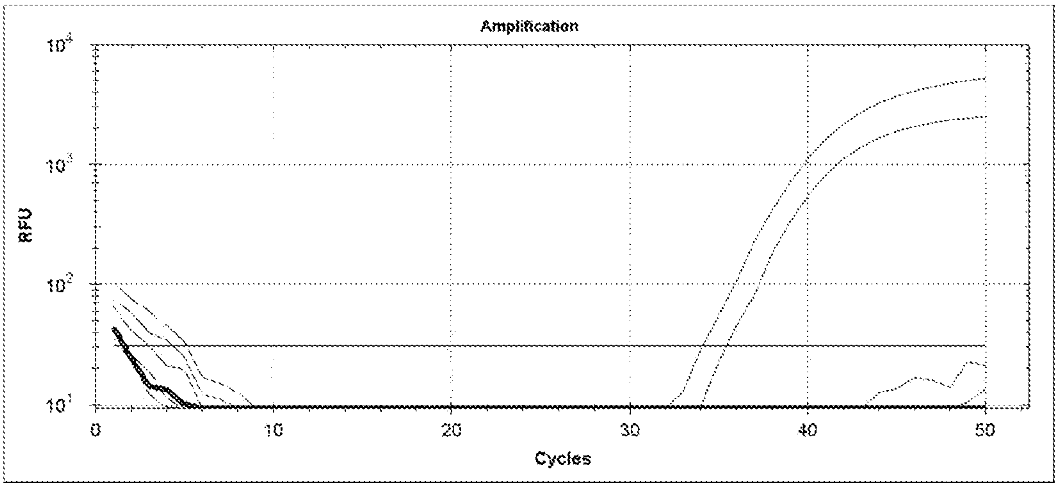


Fig. 11 (Con't)

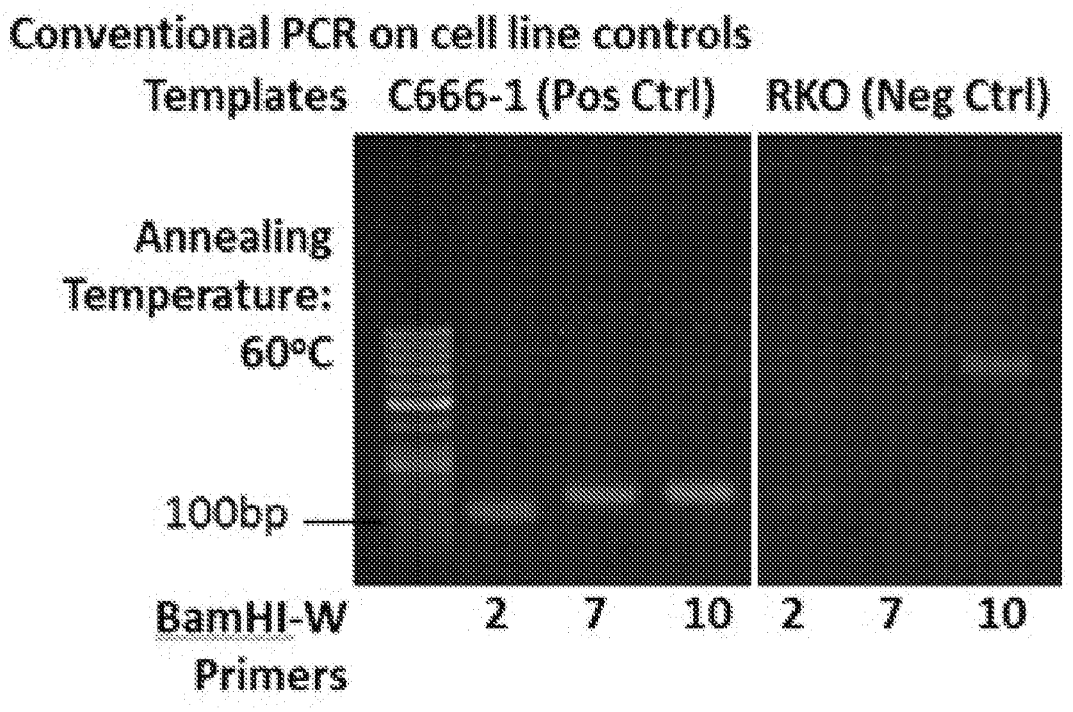
Conventional PCR on cell line controls
Templates
C666-1 (Pos Ctrl)
RKO (Neg Ctrl)
Annealing Temperature: 60°C
100bp
BamHI-W Primers
2
7
10
2
7
10

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **WAGNER et al.** Detection and quantification of latently infected B lymphocytes in Epstein-Barr virus-seropositive, healthy individuals by polymerase chain reaction. *J. Clin. Microbiol.,* November 1992, vol. 30 (11), 2826-2829
- **ARRIBAS et al.** Detection of Epstein-Barr virus DNA in cerebrospinal fluid for diagnosis of AIDS-related central nervous system lymphoma. *J. Clin. Microbiol.,* June 1995, vol. 33 (6), 1580-1583
- **MIYASHITA et al.** A Novel Form of Epstein-Barr Virus Latency in Normal B Cells In Vivo. *CELL,* 24 February 1995, vol. 80, 593-601

SEQUENCE LISTING

<110> Agency for Science, Technology and Research

<120> DETECTION AND QUANTIFICATION OF TARGET NUCLEIC ACID SEQUENCE OF A MICROORGANISM

<130> 9869SG4168

<160> 89

<170> PatentIn version 3.5

<210> 1
<211> 143
<212> DNA
<213> Human herpesvirus 4

<400> 1

```
agatctaagg ccgggagagg cagccccaaa gcgggtgcag taacaggtaa tctctggtag        60
tgatttggac ccgaaatctg acactttaga gctctggagg actttaaaac tctaaaaatc       120
aaaactttag aggcgaatgg gcg                                               143
```

<210> 2
<211> 20
<212> DNA
<213> Artificial Sequence

<220>
<223> Synthetic sequence

<400> 2
agatctaagg ccgggagagg 20

<210> 3
<211> 20

<212> DNA
<213> Artificial Sequence

<220>
<223> Synthetic sequence

<400> 3
cgcccattcg cctctaaagt 20

<210> 4
<211> 29
<212> DNA
<213> Artificial Sequence

<220>
<223> Synthetic sequence

<220>
<221> misc_feature
<222> (1)..(1)
<223> 6-carboxyfluorescein (6-FAM)

<220>
<221> misc_feature
<222> (29).. (29)
<223> 5-Carboxytetramethylrhodamine (TAMRA)

<400> 4
ctctggtagt gatttggacc cgaaatctg 29

<210> 5
<211> 3072
<212> DNA
<213> Human herpesvirus 4

<400> 5

```
ggatcccccc accggccctt ctctctgtcc ccctgctcct ctccaacctt cgctccaccc      60
tagaccccag cttctggcct ccccgggtcc accaggccag ccggagggac cccggcagcc     120
cgggcgagtc gccttccctc tcccctggcc tctccttccc gcctcccacc cgagccccct     180
cagcttgcct ccccaccggg tccatcaggc cggccggagg gaccccggcg gcccggtgtc     240
agtcccccct gcagccgccc agtctctgcc tccaggcaag ggcgccagct tttctccccc     300
cagcctgagg cccagtctcc tgtgcactgt ctgtaaagtc cagcctccca cgcccgtcca     360
cggctcccgg gcccagcctc gtccacccct ccccacggtg gacaggccct ctgtccaccc     420
gggccatccc cgcccccctg tgtccacccc agtcccgtcc aggggggact ttatgtgacc     480
cttgggcctg gctccccata gactcccatg taagcctgcc tcgagtaggt gcctccagag     540
ccccttttgc cccccctggcg gcccagcccg acccccgggc gcccccaaac tttgtccaga     600
tgtccagggg tccccgaggg tgaggcccag ccccctcccg cccctgtcca ctgccccggt     660
ccccccagaa gcccccaaaa gtagaggctc aggccatgcg cgccctgtca ccaggcctgc     720
caaagagcca gatctaaggc cgggagaggc agccccaaag cgggtgcagt aacaggtaat     780
ctctggtagt gatttggacc cgaaatctga cactttagag ctctggagga ctttaaaact     840
ctaaaaatca aaactttaga ggcgaatggg cgccattttg tccccacgcg cgcataatgg     900
cggacctagg cctaaaaccc ccaggaagcg ggtctatggt tggctgcgct gctgctatct     960
ttagagggga aaagaggaat aagcccccag acaggggagt gggcttgttt gtgacttcac    1020
caaaggtcag ggcccaaggg ggttcgcgtt gctaggccac cttctcagtc cagcgcgttt    1080
acgtaagcca gacagcagcc aattgtcagt tctagggagg gggaccactg cccctggtat    1140
aaagtggtcc tgcagctatt tctggtcgca tcagagcgcc aggagtccac acaaatgtaa    1200
gagggggtct tctacctctc cctagccctc cgccccctcc aaggactcgg gcccagtttc    1260
taacttttcc ccttccctcc ctcgtcttgc cctgcgcccg gggccacctt catcaccgtc    1320
gctgactccg ccatccaagc ctaggggaga ccgaagtgaa ggccctggac caacccggcc    1380
cgggcccccc ggtatcgggc cagaggtaag tggactttaa ttttttctgc taagcccaac    1440
```

actccaccac acccaggcac acactacaca cacccacccg tctcagggtc ccctcggaca 1500

gctcctaaga aggcaccggt cgcccagtcc taccagaggg ggccaagaac ccagacgagt 1560

ccgtagaagg gtcctcgtcc agcaagaaga ggaggtggta agcggttcac cttcaggggt 1620

aagtaacctg acctctccag ggctcacata aagggaggct tagtatacat gcttcttgct 1680

tttcacagga acctgggggc tagtctgggt gggattaggc tgcctcaagt tgcatcagcc 1740

agggcttcat gccctcctca gttccctagt ccccgggctt caggccccct ccgtccccgt 1800

cctccagaga cccgggcttc aggccctgcc tctcctgtta cccttttaga accacagcct 1860

ggacacatgt gccagacgcc ttggcctcta aggccctcgg gtcccctgg accccggcct 1920

cagcaaccct gctgctcccc tcctgccacc ccagcctccc cccctccccg tccccttcg 1980

ctcctgatcc tcccccggtc cccagtaggg ccgcctgccc ccctgcaccc agtacctgcc 2040

cctcttggcc acgcaccccg ggccaggcca ccttagaccc ggccaagccc catccctgaa 2100

gacccagcgg ccattctctc tggtaacgag cagagaagaa gtagaggccc gcggccattg 2160

ggcccagatt gagagaccag tccaggggcc cgaggttgga gccagcgggc acccgaggtc 2220

ccagcacccg gtccctccgg ggggcagaga caggcagggc cccccggcag ctggccccga 2280

ggaggcgccc ggagtggggc cggtcggctg ggctggccga gcccgggtct gggaggtctg 2340

gggtggcgag cctgctgtct caggaggggc ctggctccgc cgggtggccc tggggtaagt 2400

ctgggaggca gagggtcggc ctaggcccgg ggaagtggag gggggatcgcc cgggtctctg 2460

ttggcagagt ccgggcgatc ctctgagacc ctccgggccc ggacggtcgc cctcagcccc 2520

ccagacagac cccagggtct ccaggcaggg tccggcatct tcaggggcag caggctcacc 2580

accacaggcc ccccagaccc gggtctcggc cagccgagcc gaccggcccc gcgcctggcg 2640

cctcctcggg gccagccgcc ggggttggtt ctgcccctct ctctgtcctt cagaggaacc 2700

agggacctcg ggcaccccag agcccctcgg gcccgcctcc aggcgccctc ctggtctccg 2760

ctcccctctg agccccgtta aacccaaaga atgtctgagg ggagccaccc tcggggccca 2820

ggccccagag tccagaggtc aggggcacct cagggtgcct ccccgggtcc caggccagcc 2880

ggagggaccc cggcagcccg ggcggcccca gaggccggtt cctcgcccct tccccgggct 2940

tcagagccca ggatgtcccc cagaagggac cctaggcgtc ccctctcctc ccctccaggc 3000

ccgagcctct ccctcgcgga gaggggcctc tttgggccct caagtccagc cccaccgaga 3060

cccgagtggc cc 3072

<210> 6
<211> 206
<212> DNA
<213> Human herpesvirus 4

<400> 6

```
ctcctctcca accttcgctc caccctagac cccagcttct ggcctccccg ggtccaccag      60
gccagccgga gggaccccgg cagcccgggc gagtcgcctt ccctctcccc tggcctctcc     120
ttcccgcctc ccacccgagc cccctcagct tgcctcccca ccgggtccat caggccggcc     180
ggagggaccc cggcggcccg gtgtca                                          206
```

<210> 7
<211> 398
<212> DNA
<213> Human herpesvirus 4

<400> 7

```
aggccatgcg cgccctgtca ccaggcctgc caaagagcca gatctaaggc cgggagaggc      60
agccccaaag cgggtgcagt aacaggtaat ctctggtagt gatttggacc cgaaatctga     120
cactttagag ctctggagga ctttaaaact ctaaaaatca aaactttaga ggcgaatggg     180
cgccattttg tccccacgcg cgcataatgg cggacctagg cctaaaaccc ccaggaagcg     240
ggtctatggt tggctgcgct gctgctatct ttagagggga aaagaggaat aagcccccag     300
acaggggagt gggcttgttt gtgacttcac caaaggtcag ggcccaaggg ggttcgcgtt     360
gctaggccac cttctcagtc cagcgcgttt acgtaagc                            398
```

<210> 8
<211> 365
<212> DNA
<213> Human herpesvirus 4

<400> 8

```
ctggtataaa gtggtcctgc agctatttct ggtcgcatca gagcgccagg agtccacaca      60
aatgtaagag gggtcttct acctctccct agccctccgc cccctccaag gactcgggcc     120
cagtttctaa cttttcccct tccctccctc gtcttgccct gcgcccgggg ccaccttcat     180
caccgtcgct gactccgcca tccaagccta ggggagaccg aagtgaaggc cctggaccaa     240
cccggcccgg gccccccggt atcgggccag aggtaagtgg actttaattt tttctgctaa     300
gcccaacact ccaccacacc caggcacaca ctacacacac ccacccgtct caggtcccc      360
tcgga                                                                 365
```

<210> 9
<211> 537
<212> DNA
<213> Human herpesvirus 4

<400> 9

cgaggaggcg cccggagtgg ggccggtcgg ctgggctggc cgagcccggg tctgggaggt 60
ctggggtggc gagcctgctg tctcaggagg ggcctggctc cgccgggtgg ccctggggta 120
agtctgggag gcagagggtc ggcctaggcc cggggaagtg gagggggatc gcccgggtct 180
ctgttggcag agtccgggcg atcctctgag accctccggg cccggacggt cgccctcagc 240
cccccagaca gaccccaggg tctccaggca gggtccggca tcttcagggg cagcaggctc 300
accaccacag gccccccaga cccgggtctc ggccagccga gccgaccggc cccgcgcctg 360
gcgcctcctc ggggccagcc gccggggttg gttctgcccc tctctctgtc cttcagagga 420
accagggacc tcgggcaccc cagagcccct cgggcccgcc tccaggcgcc ctcctggtct 480
ccgctcccct ctgagccccg ttaaacccaa agaatgtctg aggggagcca ccctcgg 537

<210> 10
<211> 111
<212> DNA
<213> Human herpesvirus 4

<400> 10

ggaataagcc cccagacagg ggagtgggct tgtttgtgac ttcaccaaag gtcagggccc 60
aaggggggttc gcgttgctag gccaccttct cagtccagcg cgtttacgta a 111

<210> 11
<211> 148
<212> DNA
<213> Human herpesvirus 4

<400> 11

aggaagcggg tctatggttg gctgcgctgc tgctatcttt agaggggaaa agaggaataa 60
gcccccagac aggggagtgg gcttgtttgt gacttcacca aaggtcaggg cccaaggggg 120
ttcgcgttgc taggccacct tctcagtc 148

<210> 12
<211> 20
<212> DNA
<213> Artificial Sequence

<220>
<223> Synthetic sequence

<400> 12
ggaataagcc cccagacagg 20

<210> 13
<211> 20
<212> DNA
<213> Artificial Sequence

<220>
<223> Synthetic sequence

<400> 13
aggaagcggg tctatggttg 20

<210> 14
<211> 20
<212> DNA
<213> Artificial Sequence

<220>
<223> Synthetic sequence

<400> 14
ttacgtaaac gcgctggact 20

<210> 15
<211> 20
<212> DNA
<213> Artificial Sequence

<220>
<223> Synthetic sequence

<400> 15
gactgagaag gtggcctagc 20

<210> 16
<211> 29
<212> DNA
<213> Artificial Sequence

<220>
<223> Synthetic sequence

<400> 16
ctctggtagt gatttggacc cgaaatctg 29

<210> 17
<211> 24
<212> DNA
<213> Artificial Sequence

<220>
<223> Synthetic sequence

<400> 17
ccaccttctc agtccagcgc gttt 24

<210> 18
<211> 25
<212> DNA
<213> Artificial Sequence

<220>
<223> Synthetic sequence

<400> 18
gtgacttcac caaaggtcag ggccc 25

<210> 19
<211> 25
<212> DNA
<213> Artificial Sequence

<220>
<223> Synthetic sequence

<400> 19
ggtggtaagc ggttcacctt caggg 25

<210> 20
<211> 150
<212> DNA
<213> Mycobacterium tuberculosis

<400> 20

ggtcgccgcg ggcaggctca acccggagcg gatcaccgaa tccacgatcg cccgccacct 60

gcagcgaccc gacattcccg acgttgacct cttcctgcgg acctcgggtg agcagcgctc 120

cagcaacttc atgctgtggc aggcggccta 150

<210> 21
<211> 150
<212> DNA
<213> Mycobacterium tuberculosis

<400> 21

cctcgcaggc cgcccacaag tcgcggcggt catagtcggg ccagagcttg tcctggaata 60

tgtattcagc gtaggccgcc tgccacagca tgaagttgct ggagcgctgc tcacccgagg 120

tccgcaggaa gaggtcaacg tcgggaatgt 150

<210> 22
<211> 150
<212> DNA
<213> Mycobacterium tuberculosis

<400> 22

ggtcgccgcg ggcaggctca acccggagcg gatcaccgaa tccacgatcg cccgccacct 60

gcagcgaccc gacattcccg acgttgacct cttcctgcgg acctcgggtg agcagcgctc 120

cagcaacttc atgctgtggc aggcggccta 150

<210> 23
<211> 150
<212> DNA
<213> Mycobacterium tuberculosis

<400> 23

cctcgcaggc cgcccacaag tcgcggcggt catagtcggg ccagagcttg tcctggaata 60

tgtattcagc gtaggccgcc tgccacagca tgaagttgct ggagcgctgc tcacccgagg 120

tccgcaggaa gaggtcaacg tcgggaatgt 150

<210> 24
<211> 108
<212> DNA
<213> Mycobacterium tuberculosis

<400> 24

tggtggtcgg ctcgcggccg gtgtcgagat caccgaaccg caaagaagtg tggtcgtctt 60

cgacaccgca cccaccgccc tgctgcgggt ttaccgcgac aagcttcc 108

<210> 25
<211> 108
<212> DNA
<213> Mycobacterium tuberculosis

<400> 25

ggaagcttgt cgcggtaaac ccgcagcagg gcggtgggtg cggtgtcgaa gacgaccaca 60

cttctttgcg gttcggtgat ctcgacaccg gccgcgagcc gaccacca 108

<210> 26
<211> 150
<212> DNA
<213> Mycobacterium tuberculosis

<400> 26

gatgtccggg ctcaccccgg cgtggtcggc ggcgaacaac gcgccggtgc ggccgaagcc 60

ggtggcgatc tcatcgaaga tcagcagcac ctcgtaacgg cggcagatgt cccgcaggtc 120

gtgcagatag cgcgggtcgt gaaaacgcat 150

<210> 27
<211> 150
<212> DNA
<213> Mycobacterium tuberculosis

<400> 27

gcacgccggc gagctggccg cggtggtcgt ggagccggtc gtgcagggtg cgggcggtat 60

gcgttttcac gacccgcgct atctgcacga cctgcgggac atctgccgcc gttacgaggt 120

gctgctgatc ttcgatgaga tcgccaccgg 150

<210> 28
<211> 150
<212> DNA
<213> Mycobacterium tuberculosis

<400> 28

ggtatggtcg ccgccgcgac gccgctgccg gtgatcgggg tgcccgtacc gctgggcagg 60

ctggacgggc ttgactccct gctgtcgatc gtgcaaacgc cggccggggg tccggtggcc 120

acgctcccca tcgggggcgc cggtaaggcc 150

<210> 29
<211> 150
<212> DNA
<213> Mycobacterium tuberculosis

<400> 29

cctcgggcag ctcgaacaga tcgaacgacg ggtttccggc ccatccgacc atcttggagc 60

cctcctaatc tccgggctag tcgcgggtta acttacccgg cggccgctcc acttccgcat 120

ccttggccgc cacgccgtcg gcgagccggt 150

<210> 30
<211> 57
<212> DNA
<213> Mycobacterium tuberculosis

<400> 30
gtcatgctga tcgcggtgct cgctcggctg atgatgcgcg gctggcggcg ccgttcg 57

<210> 31
<211> 57
<212> DNA
<213> Mycobacterium tuberculosis

<400> 31
cgaacggcgc cgccagccgc gcatcatcag ccgagcgagc accgcgatca gcatgac 57

<210> 32
<211> 77
<212> DNA
<213> Mycobacterium tuberculosis

<400> 32

tgcatggctg tcgtcagctc gtgtcgtgag atgttgggtt aagtcccgca acgagcgcaa 60

cccttgtcat tagttgc 77

<210> 33
<211> 71
<212> **DNA**
<213> Mycobacterium tuberculosis

<400> 33

ccggctggcg cagccgatac acctcgcgcc acatcatgcc ggcctcgaag tcggctccgg 60

cgaacgactt t 71

<210> 34
<211> 71
<212> DNA
<213> Mycobacterium tuberculosis

<400> 34

aaagtcgttc gccggagccg acttcgaggc cggcatgatg tggcgcgagg tgtatcggct 60

gcgccagccg g 71

<210> 35
<211> 150
<212> DNA
<213> Mycobacterium tuberculosis

<400> 35

acccgcggat acgggtccgg taccgccaac cgcggcgccg acggccgccg gcgccgcggc 60

ggcagcttcg gcggcctcgg gaccgatcca tcccagcgcc cgccacgatg taataaggct 120

gttgccaata ccaatggcga aatatggcaa 150

<210> 36
<211> 150
<212> DNA
<213> Mycobacterium tuberculosis

<400> 36

ccggggtggc tggaatggtt catcaactgg tatctgccga tatcacagct gttttacaac 60

accgtgggtt tgccatattt cgccattggt attggcaaca gccttattac atcgtggcgg 120

gcgctgggat ggatcggtcc cgaggccgcc 150

<210> 37
<211> 111
<212> DNA
<213> Mycobacterium tuberculosis

<400> 37

caggtagccg gtcagccgga ttgggtctcg ttgcgccggc aggtgacggt cgcgcagcga 60

aaaagcgacc tgcgggccgc cgaggatccg atcgacgccg tcgtatgcgc c 111

<210> 38
<211> 111

<212> DNA
<213> Mycobacterium tuberculosis

<400> 38

ggcgcatacg acggcgtcga tcggatcctc ggcggcccgc aggtcgcttt ttcgctgcgc 60

gaccgtcacc tgccggcgca acgagaccca atccggctga ccggctacct g 111

<210> 39
<211> 150
<212> DNA
<213> Mycobacterium tuberculosis

<400> 39

tcacatccgg gtggcgggca gccgtcgtct gcaacgaggc cgacggtagg cgatgggtga 60

ggccatggag ggcaaatgtc cccatggtgt atcgattacg taccctctta cttcgtcggg 120

gtcgcatcgg cattcgcctt gcccgcctgt 150

<210> 40
<211> 150
<212> DNA
<213> Mycobacterium tuberculosis

<400> 40

cgggcgacac accgtcccga tacatgtcag caaccgggtc gatcgtggtg aatgcacagg 60

cgggcaaggc gaatgccgat gcgaccccga cgaagtaaga gggtacgtaa tcgatacacc 120

atggggacat ttgccctcca tggcctcacc 150

<210> 41
<211> 150
<212> DNA
<213> Mycobacterium tuberculosis

<400> 41

accggtgtct tctgccatca acaggcctcg accgcggggc agcgggccgc ccttcatctt 60

gccgcgaatg aagccctcgt cgggatcggc gtccatcacc agcagtggcg cattggcctg 120

atgcagggcc cgcaacatcg ggtcgctgcc 150

<210> 42
<211> 150
<212> DNA
<213> Mycobacterium tuberculosis

<400> 42

agccggcagc gacccgatgt tgcgggccct gcatcaggcc aatgcgccac tgctggtgat 60

ggacgccgat cccgacgagg gcttcattcg cggcaagatg aagggcggcc cgctgccccg 120

cggtcgaggc ctgttgatgg cagaagacac 150

<210> 43
<211> 69
<212> DNA
<213> Mycobacterium tuberculosis

<400> 43

agaacaccgt cgaatgcatg caagccggtg cggtgttcgg cttcgccggg ctggtagacg 60

ggttggtag 69

<210> 44
<211> 69
<212> DNA
<213> Mycobacterium tuberculosis

<400> 44

ctaccaaccc gtctaccagc ccggcgaagc cgaacaccgc accggcttgc atgcattcga 60

cggtgttct 69

<210> 45
<211> 112
<212> DNA
<213> Mycobacterium tuberculosis

<400> 45

cggggtcatg gtcgctctat gcctcggcgg cggcgttttc gggctgagcc tcggcaagca 60

cgtcacgcag agcggcttct acgacgacgg cagccaatcg gtgcaagcat cg 112

<210> 46
<211> 112
<212> DNA
<213> Mycobacterium tuberculosis

<400> 46

cgatgcttgc accgattggc tgccgtcgtc gtagaagccg ctctgcgtga cgtgcttgcc 60

gaggctcagc ccgaaaacgc cgccgccgag gcatagagcg accatgaccc cg 112

<210> 47
<211> 150
<212> DNA

<213> Mycobacterium tuberculosis

<400> 47

acgatctgcc gggcggcggc atgtgtgcgc ggcgcgatgc catcgacgag gcgcgccaac 60

cgcggcgccg gaaccgccag gatgacggcg tcggcctgcc agcggccgcc ggtttcgtcg 120

cgcagcaccc agccgcgttc gagctggacc 150

<210> 48
<211> 150
<212> DNA
<213> Mycobacterium tuberculosis

<400> 48

acgatctgcc gggcggcggc atgtgtgcgc ggcgcgatgc catcgactag gcgcgccaac 60

cgcggcgccg gaaccgccag gatgacggcg tcggcctgcc agcggccgcc ggtttcgtcg 120

cgcagcaccc agccgcgttc gagctggacc 150

<210> 49
<211> 150
<212> DNA
<213> Mycobacterium tuberculosis

<400> 49

tcacatccgg gtggcgggca gccgtcgtct gcaacgaggc cgacggtagg cgatgggtga 60

ggccatggag ggcaaatgtc cccatggtgt atcgattacg taccctctta cttcgtcggg 120

gtcgcatcgg cattcgcctt gcccgcctgt 150

<210> 50
<211> 150
<212> DNA
<213> Mycobacterium tuberculosis

<400> 50

cgggcgacac accgtcccga tacatgtcag caaccgggtc gatcgtggtg aatgcacagg 60

cgggcaaggc gaatgccgat gcgaccccga cgaagtaaga gggtacgtaa tcgatacacc 120

atggggacat ttgccctcca tggcctcacc 150

<210> 51
<211> 114
<212> DNA
<213> Mycobacterium tuberculosis

<400> 51

gcacgcaccc acctgcacat cgaaatcgtg cccggcctgg ccgccagcag cgcggtcccg 60

acctatgccg ggttgccgct gggttcgtcg cacaccgtcg ccgacgtgcg tatc 114

<210> 52
<211> 114
<212> DNA
<213> Mycobacterium tuberculosis

<400> 52

gatacgcacg tcggcgacgg tgtgcgacga acccagcggc aacccggcat aggtcgggac 60

cgcgctgctg gcggccaggc cgggcacgat ttcgatgtgc aggtgggtgc gtgc 114

<210> 53
<211> 114
<212> DNA
<213> Mycobacterium tuberculosis

<400> 53

gcacgcaccc acctgcacat cgaaatcgtg cccggcctgg ccgccagcag cgcggtcccg 60

acctatgccg ggttgccgct gggttcgtcg cacaccgtcg ccgacgtgcg tatc 114

<210> 54
<211> 114
<212> DNA
<213> Mycobacterium tuberculosis

<400> 54

gatacgcacg tcggcgacgg tgtgcgacga acccagcggc aacccggcat aggtcgggac 60

cgcgctgctg gcggccaggc cgggcacgat ttcgatgtgc aggtgggtgc gtgc 114

<210> 55
<211> 150
<212> DNA
<213> Mycobacterium tuberculosis

<400> 55

ccacgtggct acccatgacc gcgctctcag cgatcttgtc tttcttgacg tagtggaagt 60

atttcggtgt gtcgctgccg gtcccgtcgt cgacgcgttc gtcggcgtcg gtacgttcaa 120

tcgtctgggt ctgcatacct gacattgtgc 150

<210> 56
<211> 150
<212> DNA
<213> Mycobacterium tuberculosis

<400> 56

tgccaagggc acaatgtcag gtatgcagac ccagacgatt gaacgtaccg acgccgacga 60

acgcgtcgac gacgggaccg gcagcgacac accgaaatac ttccactacg tcaagaaaga 120

caagatcgct gagagcgcgg gcatgggtag 150

<210> 57
<211> 64
<212> DNA
<213> Mycobacterium tuberculosis

<400> 57

cttccgataa atagttagcc gaattatatc ctcaggcatc aatctcagct cgtccaatcg 60

agca 64

<210> 58
<211> 64
<212> DNA
<213> Mycobacterium tuberculosis

<400> 58

tgctcgattg gacgagctga gattgatgcc tgaggatata attcggctaa ctatttatcg 60

gaag 64

<210> 59
<211> 89
<212> DNA
<213> Mycobacterium tuberculosis

<400> 59

tgatggcgct ggtggaatac tccgccgacg aaatcagaga agtgttctcc gacttccccg 60

atctggaggt gtgtgtctac gccgcgccc 89

<210> 60
<211> 89
<212> DNA
<213> Mycobacterium tuberculosis

<400> 60

ggacgcggcg tagacacaca cctccagatc ggggaagtcg gagaacactt ctctgatttc 60

gtcggcggag tattccacca gcgccatca 89

<210> 61
<211> 93

<212> DNA
<213> Mycobacterium tuberculosis

<400> 61

ggtcgtgctg gcggccacgc cgtgatcgcc agctcgaaga caacgcgcca cgcgtgaagc 60

ggctcgatct ggtcgccggg cccaacggcg ccg 93

<210> 62
<211> 93
<212> DNA
<213> Mycobacterium tuberculosis

<400> 62

cggcgccgtt gggcccggcg accagatcga gccgcttcac gcgtggcgcg ttgtcttcga 60

gctggcgatc acggcgtggc cgccagcacg acc 93

<210> 63
<211> 150
<212> DNA
<213> Mycobacterium tuberculosis

<400> 63

caaaggatta ccagcgcgag gacctgaacc ctgagttctt cgcggcgtgt tctcggcatc 60

tgcatggacg tagcagactg tggttgttcc gctaccaggg cacgccaatt gccttctttt 120

tgaacgtttg gggtgcggat gagaactaca 150

<210> 64
<211> 150
<212> DNA
<213> Mycobacterium tuberculosis

<400> 64

cagtatgtag ttctcatccg caccccaaac gttcaaaaag aaggcaattg gcgtgccctg 60

gtagcggaac aaccacagtc tgctacgtcc atgcagatgc cgagaacacg ccgcgaagaa 120

ctcagggttc aggtcctcgc gctggtaatc 150

<210> 65
<211> 150
<212> DNA
<213> Mycobacterium tuberculosis

<400> 65

cgagggtgac gacccgtcga tgaccaaccc ctacatgtac gacgtcgtcg acaccaagcg 60

cggggcccgc aaaagctaca ccgaagccct gatcggacgt ggcgacatct cgatgaaaga 120

ggccgaggac gcgctgcgcg actaccaggg 150

<210> 66
<211> 104
<212> DNA
<213> Mycobacterium tuberculosis

<400> 66

ttagcccacc gtatcgggcg gcggggactt gccggaaacc gacgaacgcc tcggcatcca 60

tccaaatggc gacgaaagat cacggaattg tcgcgaaacg aacg 104

<210> 67
<211> 82
<212> DNA
<213> Mycobacterium tuberculosis

<400> 67

cctcgagcaa aaacgtcttc accggtgttg cccgcacccg tagccgccaa caacgcggca 60

tccagatcgc gctgttggct gg 82

<210> 68
<211> 82
<212> DNA
<213> Mycobacterium tuberculosis

<400> 68

ccagccaaca gcgcgatctg gatgccgcgt tgttggcggc tacgggtgcg ggcaacaccg 60

gtgaagacgt ttttgctcga gg 82

<210> 69
<211> 150
<212> DNA
<213> Mycobacterium tuberculosis

<400> 69

caaaggatta ccagcgcgag gacctgaacc ctgagttctt cgcggcgtgt tctcggcatc 60

tgcatggacg tagcagactg tggttgttcc gctaccaggg cacgccaatt gccgtctttt 120

tgaacggttg gggtgcggat gagacctaca 150

<210> 70
<211> 150
<212> DNA

<213> Mycobacterium tuberculosis

<400> 70

cagtatgtag ttctcatccg caccccaaac gttcaaaaag aaggcaattg gcgtgccctg 60

gtagcggaac aaccacagtc tgctacgtcc atgcagatgc cgagaacacg ccgcgaagaa 120

ctcaggggtc aggtcctcgc gctggtaatc 150

<210> 71
<211> 150
<212> DNA
<213> Mycobacterium tuberculosis

<400> 71

ggctaggcgc gaaaagcgcg tgttcgacat gttcggagat cgcatgaata aaaacgatgt 60

gagattcctg gattcgcccg gtgtcgcgtg acgggacgtt gatcaagaaa tctgcgaatt 120

ctgccagctg gccgccggag tcgcccgtca 150

<210> 72
<211> 83
<212> DNA
<213> Mycobacterium tuberculosis

<400> 72

ccacttcggg ttcaccctcg gcggcaacga gcgtgtcgag catccgttca cactgcgccg 60

cggtgatggc gtagccgtcg ggt 83

<210> 73
<211> 83
<212> DNA
<213> Mycobacterium tuberculosis

<400> 73

acccgacggc tacgccatca ccgcggcgca gtgtgaacgg atgctcgaca cgctcgttgc 60

cgccgagggt gaacccgaag tgg 83

<210> 74
<211> 18
<212> DNA
<213> Artificial Sequence

<220>
<223> Synthetic sequence

<400> 74
ggctgtgggt agcagacc 18

<210> 75
<211> 23
<212> DNA
<213> Artificial Sequence

<220>
<223> Synthetic sequence

<400> 75
acctgaaaga cgttatccac cat 23

<210> 76
<211> 18
<212> DNA
<213> Artificial Sequence

<220>
<223> Synthetic sequence

<400> 76
cgggtccaga tggcttgc 18

<210> 77
<211> 22
<212> DNA
<213> Artificial Sequence

<220>
<223> Synthetic sequence

<400> 77
cggctagtgc attgtcatag ga 22

<210> 78
<211> 21
<212> DNA
<213> Artificial Sequence

<220>
<223> Synthetic sequence

<400> 78
tgtcgacctg ggcagggttc g 21

<210> 79
<211> 22
<212> DNA
<213> Artificial Sequence

<220>
<223> Synthetic sequence

<400> 79
tccgaccgcg ctccgaccga cg 22

<210> 80
<211> 150
<212> DNA

<213> Artificial Sequence

<220>
<223> Synthetic sequence

<400> 80

ttagcagcga cgaagatcat gcgctcacgc tctcggtgtc ctcattcatc agttattcac 60

aacgctatgc tgtaactcga cctgacaaga ctgtacctat gagaaggcac ttgctacctt 120

atgcaagcgt cagcccgcgg tatcgcttgg 150

<210> 81
<211> 20
<212> DNA
<213> Artificial Sequence

<220>
<223> Synthetic sequence

<400> 81
cgctctcggt gtcctcattc 20

<210> 82
<211> 20
<212> DNA
<213> Artificial Sequence

<220>
<223> Synthetic sequence

<400> 82
ggctgacgct tgcataaggt 20

<210> 83
<211> 29
<212> DNA
<213> Artificial Sequence

<220>
<223> Synthetic sequence

<220>
<221> misc_feature
<222> (1)..(1)
<223> VIC fluorescent dye (VIC)

<220>
<221> misc_feature
<222> (29)..(29)
<223> Carboxytetramethylrhodamine (TAMRA)

<400> 83
cacaacgcta tgctgtaact cgacctgac 29

<210> 84
<211> 21

<212> DNA
<213> Artificial Sequence

<220>
<223> Synthetic sequence

<220>
<221> misc_feature
<222> (1)..(1)
<223> Carboxyfluorescein (FAM)

<220>
<221> misc_feature
<222> (21).. (21)
<223> Carboxytetramethylrhodamine (TAMRA)

<400> 84
tgtcgacctg ggcagggttc g 21

<210> 85
<211> 22
<212> DNA
<213> Artificial Sequence

<220>
<223> Synthetic sequence

<220>
<221> misc_feature
<222> (1)..(1)
<223> Fluorescein (FAM)

<220>
<221> misc_feature
<222> (22)..(22)
<223> Carboxytetramethylrhodamine (TAMRA)

<400> 85
tccgaccgcg ctccgaccga cg 22

<210> 86
<211> 20
<212> DNA
<213> Artificial Sequence

<220>
<223> Synthetic sequence

<400> 86
tcatcatcat ccgggtctcc 20

<210> 87
<211> 18
<212> DNA
<213> Artificial Sequence

<220>
<223> Synthetic sequence

<400> 87
gctcaccatc tgggccac 18

<210> 88
<211> 34
<212> DNA
<213> Artificial Sequence

<220>
<223> Synthetic sequence

<400> 88
cctccaggta gaaggccatt tttccaccct gtag 34

<210> 89
<211> 29
<212> DNA
<213> Artificial Sequence

<220>
<223> Synthetic sequence

<400> 89
cacaacgcta tgctgtaact cgacctgac 29